(19) Europäisches Patentamt / European Patent Office / Office européen des brevets

(11) EP 3 302 795 B1

(12) **EUROPÄISCHE PATENTSCHRIFT**

(45) Veröffentlichungstag und Bekanntmachung des Hinweises auf die Patenterteilung:
**31.07.2019 Patentblatt 2019/31**

(21) Anmeldenummer: **16727396.0**

(22) Anmeldetag: **27.05.2016**

(51) Int Cl.:
***B01J 31/24*** *(2006.01)* ***B01J 31/18*** *(2006.01)*
***C07C 209/26*** *(2006.01)* ***C07C 209/28*** *(2006.01)*
***B01J 31/20*** *(2006.01)*

(86) Internationale Anmeldenummer:
**PCT/EP2016/061999**

(87) Internationale Veröffentlichungsnummer:
**WO 2016/189129 (01.12.2016 Gazette 2016/48)**

(54) **VERFAHREN ZUR HOMOGEN KATALYSIERTEN REDUKTIVEN AMINIERUNG VON CARBONYL-VERBINDUNGEN**

METHOD FOR THE HOMOGENEOUS CATALYTIC REDUCTIVE AMINATION OF CARBONYL COMPOUNDS

PROCÉDÉ POUR L'AMINATION RÉDUCTIVE CATALYSÉE DE FAÇON HOMOGÈNE DE COMPOSÉS CARBONYLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.05.2015 EP 15169539**

(43) Veröffentlichungstag der Anmeldung:
**11.04.2018 Patentblatt 2018/15**

(73) Patentinhaber: **BASF SE**
**67056 Ludwigshafen (DE)**

(72) Erfinder:
- **SCHAUB, Thomas**
  **67433 Neustadt (DE)**
- **GALLARDO DONAIRE, Joan**
  **43007 Tarragona (ES)**
- **ERNST, Martin**
  **69121 Heidelberg (DE)**

(56) Entgegenhaltungen:
**WO-A1-2007/104359 US-A1- 2004 267 051**
**US-B1- 6 884 887**

- **GUNANATHAN CHIDAMBARAM ET AL: "Selective synthesis of primary amines directly from alcohols and ammonia", ANGEWANDTE CHEMIE INTERNATIONAL EDITION, WILEY - V C H VERLAG GMBH & CO. KGAA, DE, Bd. 47, Nr. 45, 1. Januar 2008 (2008-01-01), Seiten 8661-8664, XP002554983, ISSN: 1433-7851, DOI: 10.1002/ANIE.200803229 [gefunden am 2008-10-07]**
- **DENNIS PINGEN ET AL: "Mechanistic Study on the Ruthenium-Catalyzed Direct Amination of Alcohols", ORGANOMETALLICS, Bd. 33, Nr. 7, 14. April 2014 (2014-04-14) , Seiten 1623-1629, XP55288148, US ISSN: 0276-7333, DOI: 10.1021/om4011998**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 302 795 B1

Printed by Jouve, 75001 PARIS (FR)

## Beschreibung

Die vorliegende Erfindung betrifft ein Verfahren zur reduktiven Aminierung einer Carbonyl-Verbindung, umfassend eine oder mehrere reduktiv aminierbare Carbonylgruppen, unter Bildung des korrespondierenden primären Amins dadurch gekennzeichnet, dass man die Reaktion in Gegenwart eines homogen gelösten Komplexkatalysators K, enthaltend Ruthenium durchführt welcher einen zweizähnigen Phosphanliganden, einen Carbonylliganden, einen Neutralliganden und einen Hydridoliganden trägt, sowie einer Säure als Kokatalysators durchführt.

## Hintergrund der Erfindung:

Primäre Amine finden eine vielseitige Verwendung als Zwischenprodukte, beispielsweise zur Herstellung von Pharmazeutika oder Agrochemikalien (Amines, Alipahtic, Ullmann's Encylopedia of Industrial Chemistry, 2012, Vol. 2, S. 647-695). Besonders enantiomerenreine, chirale Amine sind hierbei wertvolle Bausteine.

Primäre Amine können auf verschiedene Weise hergestellt werden, wobei die reduktive Aminierung von Carbonylverbindungen mit Ammoniak und Wasserstoff eine besonders atomeffiziente Methode ist, da außer Wasser kein weiteres Koppelprodukt anfällt.

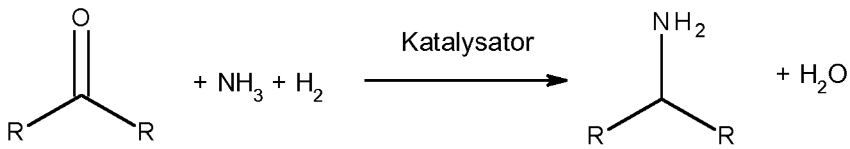


Im technischen Maßstab werden hierfür meist heterogene Katalysatoren eingesetzt, wobei sich mit diesen Systemen allerdings keine enatiomerenreinen chiralen Amine erhalten lassen. Kommerziell werden primäre, enantiomerenreine chirale Amine meist über mehrstufige enzymatische Verfahren aus den racemischen Gemischen der entsprechenden Amine hergestellt (D. Ghislieri, N.J. Turner, Top. Catal. 2014, 57, 284-300; Angew. Chem. Int. Ed. 2004, 43, 788-824).

Eine direkte Methode zur Herstellung von primären Aminen ist die homogen katalysierte reduktive Aminierung, welche sich auch asymmetrisch für die Synthese enantiomerenreiner chiraler Amine durchführen lässt (Thomas C. Nugent (Ed.), Chiral Amine Synthesis, 2010, S. 225-245, Wiley-VCH, Weinheim).

In US 6,884,887 wird die reduktive Aminierung von Carbonylverbindungen mit Aminen und Wasserstoff unter Verwendung von homogen gelösten Übergangsmetall-Phosphankomplexen offenbart. Bei der Verwendung von Ammoniak zur Herstellung von primären Aminen unter Verwendung der beschrieben Rhodium- und Iridiumkatalysatoren, welche zweizähnige Phosphanliganden tragen, werden allerdings unter diesen Bedingungen bei hohen Umsätzen unbefriedigende Selektivitäten durch die unerwünschte Hydrierung der Carbonylverbindung zum entsprechenden Alkohol erzielt. Die höchsten hier beschriebenen Selektivitäten Amin : Alkohol liegen bei Vollumsatz der Carbonylverbindung bei 4 : 1 d.h. es entstehen 20 % des unerwünschten Alkohols als Nebenprodukt bei dem beschriebenen System. Hierbei wurden für die Aminierung mit $NH_3$ keine Katalysatoren eingesetzt, die einen Carbonylliganden tragen bzw. keine Säure als Kokatalysator zugesetzt. Auch die asymmetrische Aminierung mit Ammoniak mit den beanspruchten Katalysatoren ist durch Beispiele nicht belegt. Nachteilig an diesem System ist Bildung relativ großer Mengen an Alkoholen als unerwünschte Nebenprodukte.

In US2004/0267051 wird die reduktive Aminierung von Carbonylverbindungen mit Aminen unter Verwendung von homogen gelösten Übergangsmetall-Phosphankomplexen offenbart, wobei nicht Wasserstoff, sondern Wasserstoffdonoren wie Ammoniumformiat in einer Transferhydrierung eingesetzt werden. Gemäß US2004/0267051 wird durch die Transferhydrierung unter Verwendung vergleichbarer Übergangsmetallkatalysatoren wie in US 6687887 beschrieben eine deutlich höhere Selektivität bezüglich des Amins erzielt (keine Bildung von Alkohol bei Umsätzen bis zu 96 % der Carbonylverbindung), als beim Einsatz von Wasserstoff als Reduktionsmittel. Unter Einsatz von chiralen Phosphanliganden können gemäß US2004/0267051 die Amine zudem auch enantioselektiv dargestellt werden. Nachteilig an diesem Verfahren ist, dass nicht der sehr günstige Wasserstoff als Reduktionsmittel eingesetzt werden kann, um hohe Selektivitäten zu erzielen sondern deutlich teurere Reduktionsmittel für eine Transferhydrierung verwendet werden müssen.

In Chem. Eur. J. 2014, 20, 245-252 wird ebenfalls vorgeschlagen, dass man die reduktive Aminierung von Ketonen zu primären Aminen mit Ammoniumformiat als Reduktionsmittel und $NH_3$-Quelle durchführt, um möglichst hohe Selektivitäten zum gewünschten Amin zu erzielen und die Bildung der Alkohole zu unterdrücken. Hierbei werden homogen gelöste Iridium-Halbsandwichkomplexe als Katalysatoren eingesetzt. Ebenfalls nachteilig ist, dass nicht der günstige Wasserstoff als Reduktionsmittel verwendet werden kann und sich zudem auch als Nebenprodukte die Ameisensäureamide der Produkte bilden, welche erst durch eine nachgeschaltete Hydrolyse zum gewünschten Amin umgesetzt werden.

Es bestand also die Aufgabe, ein Verfahren bereit zu stellen, durch das die vorstehend diskutierten Nachteile vermieden werden können. Ein derartiges Verfahren sollte einfach durchzuführen sein, mit Ammoniak als Aminquelle und Wasserstoff als Reduktionsmittel durchgeführt werden und möglichst wenig der unerwünschten Alkohole als Nebenprodukt liefern. Zudem soll es auch möglich sein mit diesem Verfahren die Amine enantioselektiv darzustellen.

## Kurzfassung der Erfindung:

Überraschenderweise konnte obige Aufgabe durch Verwendung von speziellen Rutheniumkatalysatoren zur homogen katalysierten reduktiven Aminierung für die Herstellung von primären Aminen gelöst werden.

## Detaillierte Beschreibung der Erfindung:

### a) Allgemeine Definitionen

Werden keine anderen Angaben gemacht so gelten erfindungsgemäß folgendeallgemeine Definitionen:
Unter "homogen-katalysiert" wird im Rahmen der vorliegenden Erfindung verstanden, dass der katalytisch-aktive Teil des Komplexkatalysators zumindest teilweise im flüssigen Reaktionsmedium gelöst vorliegt. In einer bevorzugten Ausführungsform liegen mindestens 90 % des im Verfahren eingesetzten Komplexkatalysators im flüssigen Reaktionsmedium gelöst vor, mehr bevorzugt mindestens 95 %, insbesondere bevorzugt mehr als 99 %, am meisten bevorzugt liegt der Komplexkatalysator vollständig gelöst im flüssigen Reaktionsmedium vor (100%), jeweils bezogen auf die Gesamtmenge im flüssigen Reaktionsmedium. Die Menge des Katalysators beträgt im Allgemeinen 0,1 bis 5.000 Gewichts-ppm, jeweils bezogen auf das gesamte flüssige Reaktionsmedium.

"Stereoisomere" sind Verbindungen gleicher Konstitution aber unterschiedlicher Atomanordnung im dreidimensionalen Raum.

"Enantiomere" sind Stereoisomere, die sich zueinander wie Bild zu Spiegelbild verhalten. Der bei einer asymmetrischen Synthese erzielte "Enantiomeren-Überschuss" (enantiomeric excess, ee) ergibt sich dabei nach folgender Formel:

$$ee[\%] = (R-S)/(R+S) \times 100.$$

R und S sind die Deskriptoren des CIP-Systems für die beiden Enantiomeren und geben die absolute Konfiguration am asymmetrischen Atom wieder. Die enantiomerenreine Verbindung (ee = 100 %) wird auch als "homochirale Verbindung" bezeichnet.

### b) Definitionen chemischer Reste

### b1) aliphatische nichtcyclische Reste

Wenn nicht anders definiert, so ist hierein der Begriff **"Alkyl"** grundsätzlich breit zu verstehen. Insbesondere umfasst "Alkyl" im weitesten Sinne aliphatische, geradkettige oder verzweigte, nichtcyclische Kohlenwasserstoffreste mit 1 bis 30 C-Atomen, welche ggf. zusätzlich ein oder mehrere, wie z.B. 1, 2 oder 3 Heteroatome, wie z.B. O, N, NH, S, in ihrer Kette enthalten können. Weiterhin können "Alkyl"-Reste im weitesten Sinn ein- oder mehrfach ungesättigt sein und ein oder mehrere nicht-kumulierte, wie z.B. 1 bis 5, wie 1, 2 oder 3 C-C Doppelbindungen oder C-C- Dreifachbindungen, insbesondere 1, 2 oder 3 Doppelbindungen, aufweisen. Sie können natürlichen oder synthetischen Ursprungs sein. Der Ausdruck "Alkyl" umfasst auch substituierte Alkylgruppen, welche im Allgemeinen 1, 2, 3, 4 oder 5, bevorzugt 1, 2 oder 3 und besonders bevorzugt 1 Substituenten, tragen können. Obige Reste können auch unter dem Oberbegriff **"Hydrocarbyl"**-Reste zusammengefasst werden.

Diese **"Substituenten"** sind dabei insbesondere ausgewählt unter F, Cl, Br, Hydroxy (OH), $C_1$-$C_{30}$-Alkoxy, $C_5$-$C_{30}$-Aryloxy, $C_5$-$C_{30}$-Alkylaryloxy, $C_5$-$C_{30}$-Heteroaryloxy enthaltend mindestens ein Heteroatom ausgewählt aus N, O, S, Oxo, $C_3$-$C_{30}$-Cycloalkyl, Phenyl, $C_5$-$C_{30}$-Heteroaryl enthaltend mindestens ein Heteroatom ausgewählt aus N, O, S, $C_5$-$C_{30}$-Heterocyclyl enthaltend mindestens ein Heteroatom ausgewählt aus N, O, S, Naphthyl, Amino, $C_1$-$C_{36}$-Alkylamino, $C_5$-$C_{30}$-Arylamino, $C_5$-$C_{30}$-Heteroarylamino enthaltend mindestens ein Heteroatom ausgewählt aus N, O, S, $C_1$-$C_{30}$-Dialkylamino, $C_5$-$C_{14}$-Diarylamino, $C_6$-$C_{20}$-Alkylarylamino, $C_1$-$C_{30}$-Acyl, $C_1$-$C_{30}$-Acyloxy, $NO_2$, $C_1$-$C_{30}$-Carboxy, Carbamoyl, Carboxamid, Cyane, Sulfonyl, Sulfonylamino, Sulfinyl, Sulfinylamino, Thiol, $C_1$-$C_{30}$-Alkylthiol, $C_5$-$C_{30}$-Arylthiol und $C_1$-$C_{30}$-Alkylsulfonyl **("Substituenten-Gruppe 1")**.

Als nichtlimitierende Beispiele obiger erfindungsgemäßer "Alkyl"("Hydrocarbyl")-Gruppen gemäß obiger allgemeiner Definition sind zu nennen:

**"Alkyl"** im engeren Sinn (sowie alle Alkyl-Gruppen in davon abgeleiteten Resten, wie z.B. Alkoxy, Alkylthio, Alkoxyalkyl, Alkoxyalkoxy, Hydroxyalkyl, Aminoalkyl, Alkylamino und Dialkylamino; Halogenalkyl, Aralkyl sowie Acyl oder Alcoxycarbonyl) steht speziell für gesättigte, geradkettige oder verzweigte Kohlenwasserstoffreste, die keine Heteroatome enthalten, mit 1 bis 4, 1 bis 5, 1 bis 6, oder 1 bis 7, 1 bis 10, 1 bis 20 oder 1 bis 30, Kohlenstoffatomen, wie z. B. Methyl, Ethyl, n-Propyl, 1-Methylethyl, n-Butyl, 1-Methyl-propyl, 2-Methylpropyl, 1,1 -Dimethylethyl, n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; sowie n-Heptyl, sowie die ein- oder mehrfach verzweigten Analoga davon. Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Heneicosyl, Docosyl, Tricosyl, Tetracosyl, Pentacosyl, Hexacosyl, Heptacosyl, Octacosyl, Nonacosyl, Squalyl, Konstitutionsisomere, insbesondere ein oder mehrfach verzweigte Isomere und höhere Homologe davon.

"**Heteroalkyl**", steht insbesondere für die Gruppe oben aufgelisteter spezieller Alkylreste, welche zusätzlich ein oder mehrere, wie z.B. 1, 2 oder 3 Heteroatome, wie z.B. O, N, NH, S, in ihrer Kette enthalten können.

**"Niedrigalkyl"** steht für obige Alkyl-Reste mit 1 bis 4, 1 bis 5, 1 bis 6, oder 1 bis 7 Kohlenstoffatomen.

**"Alkenyl"** umfasst ein- oder mehrfach, insbesondere einfach ungesättigte, Analoga obiger Alkylgruppen und wenigstens einer C-C- Doppelbindung. Insbesondere umfasst der Begriff ein- oder mehrfach, insbesondere einfach ungesättigte, geradkettige oder verzweigte Kohlenwasserstoffreste mit 2 bis 4, 2 bis 6, 2 bis 8, 2 bis 10, 2 bis 20 oder 2 bis 30 Kohlenstoffatomen und einer Doppelbindung in einer beliebigen Position, z. B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

**"Alkinyl"** umfasst ein- oder mehrfach, insbesondere einfach ungesättigte, Analoga obiger Alkylgruppen und wenigstens einer C-C-Dreifachbindung. Insbesondere umfasst der Begriff ein- oder mehrfach, insbesondere einfach ungesättigte, geradkettige oder verzweigte Kohlenwasserstoffreste mit 2 bis 4, 2 bis 6, oder 2 bis 8, Kohlenstoffatomen und einer Dreifachbindung in einer beliebigen Position, insbesondere die zu den oben explizit genannten Alkenylresten analogen Reste mit C-C-Dreifachbindung; z. B. $C_2$-$C_6$-Alkinyl, wie Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 3-Methyl-1-butinyl, 1-Methyl-2-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl und dessen isomere Formen.

**"Halo(gen)alkyl"** umfasst geradkettige oder verzweigte Alkylgruppen mit 1 bis 4, 1 bis 6, 1 bis 8, 1 bis 10 oder 1 bis 20 Kohlenstoffatomen (wie vorstehend genannt), wobei in diesen Gruppen teilweise oder vollständig die Wasserstoffatome durch Halogenatome wie vorstehend genannt ersetzt sein können, z. B. $C_1$-$C_2$-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 1,1,1-Trifluorprop-2-yl.

**"Hydroxyalkyl"** steht insbesondere für die ein- oder mehrfach, insbesondere einfach hydroxylierten Analoga obiger Alkylreste, wie z.B. die monohydroxylierten Analoga obiger geradkettiger oder verzweigter Alkylreste, wie z.B. die linearen Hydroxyalkylgruppen, wie z.B. solchen mit primärer (endständigen) Hydroxylgruppe, wie Hydroxymethyl, 2-Hydroxyethyl, 3-Hydroxypropyl, 4-Hydroxybutyl, oder solchen mit nichtendständigen Hydroxylgruppen, wie 1-Hydroxyethyl, 1- oder 2-Hydroxypropyl, 1- oder 2-Hydroxybutyl oder 1-, 2- oder 3-Hydroxybutyl.

**"Hydroxyalkenyl"** steht insbesondere für die ein- oder mehrfach, insbesondere einfach hydroxylierten Analoga obiger Alkenylreste

**"Aminoalkyl"** und **"Aminoalkenyl"** steht insbesondere für die ein- oder mehrfach, insbesondere einfach aminierten Analoga obiger Alkyl- bzw. Alkenylreste, oder Analoga obiger Hydroxyalkyl, wobei die OH-Gruppe durch eine Aminogruppe ersetzt ist.

**"Alkoxy"** steht insbesondere für die sauerstoffterminierten Analoga obiger Alkyl-Reste. Als nichtlimitierende Beispiele können genannt werden: $C_1$-$C_6$-Alkoxy-Reste, wie z.B. Methoxy, Ethoxy, n-Propoxy, 1-Methylethoxy, Butoxy, 1-Methylpropoxy, 2-Methylpropoxy oder 1,1-Dimethylethoxy; sowie z. B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy.

**"Alkoxyalkyl"** steht insbesondere für obige Alkylgruppen, welche wenigstens einen der oben genannten Alkoxyreste als Substituenten tragen.

**"Alkoxycarbonyl"** steht insbesondere für obige Alkoxygruppen, welche an eine Carbonylgruppe gebunden sind.

**"Alkylthio"** steht insbesondere für die S-terminierten Analoga obiger AlkoxyReste.

**"Halogenalkoxy"** steht für einen Alkoxyrest mit 1 bis 8, insbesondere 1 bis 6 und speziell 1 bis 4 C-Atomen wie vorstehend genannt, der partiell oder vollständig durch Fluor, Chlor, Brom und/oder Iod, vorzugsweise durch Fluor substituiert ist, also z. B. $OCH_2F$, $OCHF_2$, $OCF_3$, $OCH_2Cl$, $OCHCl_2$, $OCCl_3$, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, $OC_2F_5$, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, $OCH_2$-$C_2F_5$, $OCF_2$-$C_2F_5$, 1-($CH_2F$)-2-fluorethoxy, 1-($CH_2Cl$)-2-chlorethoxy, 1-($CH_2Br$)-2-bromethoxy, 4-Fluorbutoxy, 4-Chlorbutoxy, 4-Brombutoxy oder Nonafluorbutoxy.

**b2) Polymere Reste**

**"Polyalkylen"** Reste, umfassen insbesondere solche, die im Wesentlichen aus $C_{2-6}$-, insbesondere $C_{2-4}$-Monomerbausteinen, wie Ethylen, Propylen, n- oder isoButylen oder Mischungen davon aufgebaut sind und einen Polymerisationsgrad von 2 bis 100, oder 3 bis 50 oder 4 bis 25 oder 5 bis 10 aufweisen.

**"Polyalkylenoxid"** steht insbesondere für einen von gleichen oder verschiedenen $C_{2-4}$-Oxyalkylen-Monomerbausteinen abgeleiteten Rest mit einem Polymerisationsgrad von 2 bis 100, oder 3 bis 50 oder 4 bis 25 oder 5 bis 10.

**"Polyalkylenimin"** steht für die strukturanalogen Reste zu obigen Polyalkylenoxidresten, wobei das Sauerstoffatom durch Imingruppen ersetzt ist.

**b3) nichtzyklische Brückengruppen**

**"Alkylen"** steht für geradkettige oder ein- oder mehrfach verzweigte Alkandiyl-Gruppen, d.h. Kohlenwasserstoff-Brückengruppen mit 1 bis 10 Kohlenstoffatomen, wie z.B. $C_1$-$C_7$-Alkylengruppen ausgewählt unter $-CH_2-$, $-(CH_2)_2-$, $-(CH_2)_3-$, $-(CH_2)_4-$, $-(CH_2)_2-CH(CH_3)-$, $-CH_2-CH(CH_3)-CH_2-$, $(CH_2)_4-$, $-(CH_2)_5-$, $-(CH_2)_6$, $-(CH_2)_7-$, $-CH(CH_3)-CH_2-CH_2-CH(CH_3)-$ oder $-CH(CH_3)-CH_2-CH_2-CH_2-CH(CH_3)-$ oder $C_1$-$C_4$-Alkylengruppen ausgewählt unter $-CH_2-$, $-(CH_2)_2-$, $-(CH_2)_3-$, $-(CH_2)_4-$, $-(CH_2)_2-CH(CH_3)-$, $-CH_2-CH(CH_3)-CH_2-$
oder für $C_2$-$C_6$-Alkylengruppen, wie z.B. $-CH_2-CH(CH_3)-$, $-CH(CH_3)-CH_2-$, $-CH(CH_3)-CH(CH_3)-$, $-C(CH_3)_2-CH_2-$, $-CH_2-C(CH_3)_2-$, $-C(CH_3)_2-CH(CH_3)-$, $-CH(CH_3)-C(CH_3)_2-$, $-CH_2-CH(Et)-$, $-CH(CH_2CH_3)-CH_2-$, $-CH(CH_2CH_3)-CH(CH_2CH_3)-$, $-C(CH_2CH_3)_2-CH_2-$, $-CH_2-C(CH_2CH_3)_2-$, $-CH_2-CH(n\text{-}Propyl)-$, $-CH(n\text{-}Propyl)-CH_2-$, $-CH(n\text{-}Propyl)-CH(CH_3)-$, $-CH_2-CH(n\text{-}Butyl)-$, $-CH(n\text{-}Butyl)-CH_2-$, $-CH(CH_3)-CH(CH_2CH_3)-$, $-CH(CH_3)-CH(n\text{-}Propyl)-$, $-CH(CH_2CH_3)-CH(CH_3)-$, $-CH(CH_3)-CH(CH_2CH_3)-$, oder für $C_2$-$C_4$-Alkylengruppen, wie z.B. ausgewählt unter $-(CH_2)_2-$, $-CH_2-CH(CH_3)-$, $-CH(CH_3)-CH_2-$, $-CH(CH_3)-CH(CH_3)-$, $-C(CH_3)_2-CH_2-$, $-CH_2-C(CH_3)_2-$, $-CH_2-CH(CH_2CH_3)-$, $-CH(CH_2CH_3)-CH_2-$.

**"Oxyalkylen"-**Reste entsprechen der Definition obiger geradkettiger oder ein- oder mehrfach verzweigter Alkylenreste mit 2 bis 10 Kohlenstoffatomen, wobei die Kohlenkette durch ein Sauerstoff-Heteroatom 1- oder mehrfach, insbesondere 1-fach unterbrochen ist. Als nichtlimitierende Beispiele sind zu nennen: $-CH_2-O-CH_2-$, $-(CH_2)_2-O-(CH_2)_2-$, $-(CH_2)_3-O-(CH_2)_3-$, oder $-CH_2-O-(CH_2)_2-$, $-(CH_2)_2-O-(CH_2)_3-$, $-CH_2-O-(CH_2)_3$

**"Aminoalkylen"** entsprechen der Definition obiger geradkettiger oder ein- oder mehrfach verzweigter Alkylenreste mit 2 bis 10 Kohlenstoffatomen, wobei die Kohlenkette durch eine Stickstoffgruppe (insbesondere -NH-Gruppe) **1**- oder mehrfach, insbesondere 1-fach unterbrochen ist. Als nichtlimitierende Beispiele sind zu nennen: - $CH_2-NH-CH_2-$, $-(CH_2)_2-NH-(CH_2)_2-$, $-(CH_2)_3-NH-(CH_2)_3-$, oder $-CH_2-NH-(CH_2)_2-$, $-(CH_2)_2-NH-(CH_2)_3-$, $-CH_2-NH-(CH_2)_3$.

**"Alkenylen"** steht für die ein- oder mehrfach, insbesondere einfach ungesättigten Analoga obiger Alkylengruppen mit 2 bis 10 Kohlenstoffatomen, insbesondere für $C_2$-$C_7$-Alkenylene oder $C_2$-$C_4$-Alkenylen, wie $-CH=CH-$, $-CH=CH-CH_2-$, - $CH_2-CH=CH-$, $-CH=CH-CH_2-CH_2-$, $-CH_2-CH=CH-CH_2-$, $-CH_2-CH_2-CH=CH-$, $-CH(CH_3)-CH=CH-$, $-CH_2-C(CH_3)=CH-$. Im Falle einer Substitution können diese im Allgemeinen 1, 2, 3, 4 oder 5, bevorzugt 1, 2 oder 3 und

besonders bevorzugt 1 "Substituenten" gemäß obiger Definition, insbesondere ausgewählt unter Alkyl, Aryl, Alkoxy und Halogen tragen.

**b4) Cyclische Reste**

**"Cycloalkyl"** steht für carbocyclische, mono- oder polycyclisch, wie z.B. mono-, bi- oder tricyclische, Reste mit 3 bis 30 oder 3 bis 20 Kohlenstoffatomen, wie z.B. $C_3$-$C_{12}$-Cycloalkyl, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl, Cycloheptyl, sowie Cyclopropyl-methyl, Cyclopropyl-ethyl, Cyclobutyl-methyl, Cyclobutyl-ethyl, Cyclopentyl-methyl, Cyclopentyl-ethyl, Cyclohexyl-methyl oder $C_3$-$C_7$-Cycloalkyl, wie Cyclopropyl, Cyclobutyl, Cyclopentyl, Cyclohexyl, Cycloheptyl, Cyclopropyl-methyl, Cyclopropyl-ethyl, Cyclobutyl-methyl, Cyclopentyl-ethyl, Cyclohexyl-methyl, wobei die Anbindung an den Rest des Moleküls über jegliches geeignetes C-Atom erfolgen kann. Im Falle einer Substitution können diese im Allgemeinen 1, 2, 3, 4 oder 5, bevorzugt 1, 2 oder 3 und besonders bevorzugt 1 "Substituenten" gemäß obiger Definition, insbesondere ausgewählt unter Alkyl, Aryl, Alkoxy und Halogen tragen.

**"Cycloalkoxy"** steht insbesondere für die sauerstoffterminierten Analoga obiger Cycloalkyl-Reste.

**"Cycloalkenyl"** steht für oder ein- oder mehrfach ungesättigte Analoga obiger "Cycloalkyl" -Reste. Insbesondere steht es für monocyclische, ein oder mehrfach ungesättigte Kohlenwasserstoffgruppen mit 5 bis 8, vorzugsweise bis 6 Kohlenstoffringgliedern, wie z.B. die einfach ungesättigten Rests Cyclopenten-1-yl, Cyclopenten-3-yl, Cyclohexen-1-yl, Cyclohexen-3-yl und Cyclohexen-4-yl. Im Falle einer Substitution können diese im Allgemeinen 1, 2, 3, 4 oder 5, bevorzugt 1, 2 oder 3 und besonders bevorzugt 1 "Substituenten" gemäß obiger Definition, insbesondere ausgewählt unter Alkyl, Aryl, Alkoxy und Halogen tragen.

**"Cycloalkylen"** steht insbesondere für Brückengruppen abgeleitet von obigen Cycloalkylgruppen, wobei die Anbindung an den Rest des Moleküls über jegliches geeignetes C-Atom erfolgen kann. Im Falle einer Substitution können diese im Allgemeinen 1, 2, 3, 4 oder 5, bevorzugt 1, 2 oder 3 und besonders bevorzugt 1 "Substituenten" gemäß obiger Definition, insbesondere ausgewählt unter Alkyl, Aryl, Alkoxy und Halogen tragen.

**"Cycloalkenylen"** steht insbesondere für Brückengruppen abgeleitet von obigen Cycloalkenylgruppen, wobei die Anbindung an den Rest des Moleküls über jegliches geeignetes C-Atom erfolgen kann. Im Falle einer Substitution können diese im Allgemeinen 1, 2, 3, 4 oder 5, bevorzugt 1, 2 oder 3 und besonders bevorzugt 1 "Substituenten" gemäß obiger Definition, insbesondere ausgewählt unter Alkyl, Aryl, Alkoxy und Halogen tragen.

**"Aryl"** steht erfindungsgemäß für ein- oder mehrkernige, vorzugsweise ein-, zwei-, drei - oder vierkernige, gegebenenfalls substituierte aromatische Reste mit 5 bis 20, wie z.B. 5 bis 10, 5 bis 14 oder 5 bis 18 Ring-Kohlenstoffatomen, wie z.B. Phenyl, Tolyl, Xylyl, Mesityl, Biphenyl, Naphthyl, wie 1- oder 2-Naphthyl, Tetrahydronaphthyl, Fluorenyl, Indenyl Naphthacenyl, und Phenanthrenyl. Diese Arylreste können gegebenenfalls 1, 2, 3, 4, 5 oder 6 vorzugsweise 1, 2 oder 3 und besonders bevorzugt 1, gleiche oder verschiedene Substituenten tragen. Im Falle eines mehrkernigen Arylrestes weist wenigstens einer der Kerne aromatischen Charakter auf; es können aber auch mehrere oder alle Kerne aromatischen Charakter besitzen.

**"Arylalkyl" oder "Aralkyl"** steht für die Aryl-substituierten Analoga obiger Alkyl-reste, wobei Aryl ebenfalls die oben angegebenen Bedeutungen besitzt, wie z.B. Phenyl-$C_1$-$C_4$-Alkylreste ausgewählt unter Phenyl-methyl oder Phenyl-ethyl.

**"Aryloxy"** steht für die Sauerstoff-verknüpften Analoga obiger gegebenenfalls substituierter Arylreste.

"Substituenten" für hierin angegebene "Aryl"-haltigen Reste, sind insbesondere, wenn keine anderen Angaben gemacht werde, ausgewählt sind unter Keto-, Carboxyl-, Carboxylat-, -COO-Alkyl, -OH, -SH, -CN, Amino-, $-NO_2$, $-SO_3H$, Sulfonat-, $-NE^1E^2$, - Alkylen-$NE^1E^2$, Halogen, Alkoxy-, Trifluormethyl-, Alkyl-, oder Alkenyl-Gruppen **("Substituenten-Gruppe 2")**.

**"Heterocyclyl"** umfasst Gruppen mit 1 bis 30 oder 2 bis 20 oder 5 bis 12 Ringkohlenstoffatomen und wenigstens einen fünf- bis siebengliedriger gesättigter, partiell ungesättigter oder aromatischer Heterocyclen bzw. Heterocyclylreste, enthaltend ein, zwei , drei oder vier Heteroatome aus der Gruppe O, N oder S. Für den Fall, dass Stickstoff ein Ringatom ist, umfasst die vorliegende Erfindung auch N-Oxide der stickstoffenthaltenden Heterocyclen. Beispielsweise können folgende Untergruppen genannt werden

- 5- oder 6-gliedriges gesättigtes oder einfach ungesättigtes Heterocyclyl, enthaltend ein bis zwei Stickstoffatome und/oder ein Sauerstoff- oder Schwefelatom oder ein oder zwei Sauerstoff- und/oder Schwefelatome als Ringglieder, z. B. 2-Tetrahydrofuranyl, 3-Tetrahydrofuranyl, 2-Tetrahydrothienyl, 3-Tetrahydrothienyl, 1-Pyrrolidinyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 1-Piperidinyl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;

- 5-gliedriges aromatisches Heterocyclyl (= **Heteroaryl** bzw. **Hetaryl**), enthaltend neben Kohlenstoffatomen ein, zwei oder drei Stickstoffatome oder ein oder zwei Stickstoffatome und ein Schwefel- oder Sauerstoffatom als Ringglieder, z. B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;

- 5-gliedriges aromatisches Heterocyclyl (= **Heteroaryl** bzw. **Hetaryl**), das 1, 2, 3 oder 4 Stickstoffatome als Ringglieder aufweist, wie 1-, 2- oder 3-Pyrrolyl, 1-, 3- oder 4-Pyrazolyl, 1-, 2- oder 4-Imidazolyl, 1,2,3-[1H]-Triazol-1-yl, 1,2,3-[2H]-Triazol-2-yl, 1,2,3-[1H]-Triazol-4-yl, 1,2,3-[1H]-Triazol-5-yl, 1,2,3-[2H]-Triazol-4-yl, 1,2,4-[1H]-Triazol-1-yl, 1,2,4-[1H]-Triazol-3-yl, 1,2,4-[1H]-Triazol-5-yl, 1,2,4-[4H]-Triazol-4-yl, 1,2,4-[4H]-Triazol-3-yl, [1H]-Tetrazol-1-yl, [1H]-Tetrazol-5-yl, [2H]-Tetrazol-2-yl und [2H]-Tetrazol-5-yl;

- 5-gliedriges aromatisches Heterocyclyl (= **Heteroaryl** bzw. **Hetaryl**), das 1 unter Sauerstoff und Schwefel ausgewähltes Heteroatome und gegebenenfalls 1, 2 oder 3 Stickstoffatome als Ringglieder aufweist, beispielsweise 2-Furyl, 3-Furyl, 2-Thienyl, 3-Thienyl, 3- oder 4-Isoxazolyl, 3- oder 4- Isothiazolyl, 2-, 4- oder 5-Oxazolyl, 2-, 4 oder 5-Thiazolyl, 1,2,4-Thiadiazol-3-yl, 1,2,4-Thiadiazol-5-yl, 1,3,4-Thiadiazol-2-yl, 1,2,4-Oxadiazol-3-yl, 1,2,4-Oxadiazol-5-yl und 1,3,4-Oxadiazol-2-yl;

- 6-gliedriges Heterocyclyl (= **Heteroaryl** bzw. **Hetaryl**), enthaltend neben Kohlenstoffatomen ein oder zwei bzw. ein, zwei oder drei Stickstoffatome als Ringglieder, z. B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,2,4-Triazin-3-yl; 1,2,4-Triazin-5-yl, 1,2,4-Triazin-6-yl und 1,3,5-Triazin-2-yl.

**"Heterocyclyl"** umfasst insbesondere auch "mehrkernige", wie z.B. zwei- oder dreikernige, cyclische Ringsysteme, in denen einer der oben genannten einkernigen Heterocyclylreste mit wenigstens einem weiteren, gleichen oder verschiedenen Heterocyclus, wenigstens einem Arylring und/oder wenigstens einem Cycloalkyl- oder Cycloalkenyl-Ring jeweils gemäß obiger Definition kondensiert ist.

**"Heteroaryl"** umfasst insbesondere auch "mehrkernige", wie z.B. zwei- oder dreikernige, cyclische Ringsysteme, in denen einer der oben genannten einkernigen Heteroarylreste mit wenigstens einem weiteren gleichen oder verschiedenen Heteroarylring, wenigstens einem Arylring und/oder wenigstens einem Cycloalkyl oder Cycloalkenylring jeweils gemäß obiger Definition kondensiert ist.

Obige "Heterocyclyl" oder Heteroaryl-Gruppen können unsubstituiert oder substituiert sein, mit einem oder mehreren, wie z.B. 1, 2, 3, 4, oder 5 insbesondere 1, 2 oder 3 Substituenten der "Substituenten- Gruppe 1".

Eine bevorzugte Untergruppe von Hetreocyclyl- oder Heteroaryl-Resten umfasst: Thienyl, Benzothienyl, 1-Naphthothienyl, Thianthrenyl, Furyl, Benzofuryl, Pyrrolyl, Imidazolyl, Pyrazolyl, Pyridyl, Pyrazinyl, Pyrimidinyl, Pyridazinyl, Indolyl, Isoindolyl, Indazolyl, Purinyl, Isoquinolinyl, Quinolinyl, Acridinyl, Naphthyridinyl, Quinoxalinyl, Quinazolinyl, Cinnolinyl, Piperidinyl, Carbolinyl, Thiazolyl, Oxazolyl, Isothiazolyl, und Isoxazolyl.

**"Heterocyclyloxy"** bzw. **"Heteroaryloxy"** steht für die Sauerstoff-verknüpften Analoga obigerr Heterocyclyl- bzw. Heteroarylreste.

Eine spezielle Untergruppe obiger Heterocyclyreste sind **"Heterocycloalkyl"** Reste. Diese Gruppe umfasst gesättigte, cycloaliphatische Gruppen mit 4 bis 7, vorzugsweise 5 oder 6 Ringatomen, in denen 1, 2, 3 oder 4 der Ringkohlenstoffatome durch Heteroatome, vorzugsweise ausgewählt unter N, O und S ersetzt sind und die gegebenenfalls substituiert sein können. Im Falle einer Substitution weisen diese heterocycloaliphatischen Gruppen bevorzugt 1, 2 oder 3, besonders bevorzugt 1 oder 2, insbesondere 1 Substituenten auf. Diese sind vorzugsweise ausgewählt **unter obiger Substituenten Gruppe 1** Beispielhaft für solche heterocycloaliphatischen Reste seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

**"Heterocycloalkoxy"** steht insbesondere für obige Alkoxygruppen, welche wenigstens einen der oben genannten Heterocyclylreste als Substituenten tragen.

Erfindungsgemäße Brückengruppen **"Arylen", "Heteroarylen", "Heteroalkylen"** entsprechen den zweifach verknüpften Analoga obiger Aryl, Heteroaryl und Heteroalkylgruppen.

**"Kondensierte Ringsysteme"** können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört. Bevorzugt unter den kondensierten Ringsystemen sind orthokondensierte Ringsysteme.

**b5) Verschiedene**

**"Acyl"** steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

**"Metallocen"** steht erfindungsgemäß für eine Gruppe von metallorganischen Verbindungen, in denen ein zentrales Metallatom Me, insbesondere Fe, wie in einem Sandwich zwischen zwei Cyclopentadienyl-Liganden ($C_5H_5$, Abkürzung: Cp) angeordnet ist. **"Metallocenyl"** steht für eine von einer solchen Sandwich-Verbindung abgeleitet Seitengruppe, welchen über wenigstens ein Cp-Ringkohlenstoffatom an ein Molekül gebunden ist.

Die Gruppen $NE^1E^2$, $NE^4E^5$, $NE^9E^{10}$, stehen vorzugsweise für N,N-Dimethylamino, N,N-Diethylamino, N,N-Dipropylamino, N,N-Diisopropylamino, N,N-Di-n-butylamino, N,N-Di-t.-butylamino, N,N-Dicyclohexylamino oder N,N-Diphenylamino.

**"Halogen"** steht für Fluor, Chlor, Brom und Iod, bevorzugt für Fluor, Chlor und Brom.

**"$M^+$"** steht für ein Kationäquivalent, d. h. für ein einwertiges Kation oder den einer positiven Einfachladung entsprechenden Anteil eines mehrwertigen Kations. Das Kation $M^+$ dient lediglich als Gegenion zur Neutralisation negativ geladener Substituentengruppen, wie der COO- oder der Sulfonat-Gruppe und kann im Prinzip beliebig gewählt werden. Vorzugsweise werden deshalb Alkalimetall-, insbesondere $Na^+$, $K^+$-, $Li^+$-Ionen oder Onium-Ionen, wie Ammonium-, Mono-, Di-, Tri-, Tetraalkylammonium-, Phosphonium-, Tetraalkylphosphonium- oder Tetraarylphosphonium-Ionen verwendet.

Entsprechendes gilt für das Anionäquivalent **"$X^-$",** das lediglich als Gegenion positiv geladener Substituentengruppen, wie den Ammoniumgruppen, dient und beliebig gewählt werden kann unter einwertigen Anionen und den einer negativen Einfachladung entsprechenden Anteilen eines mehrwertigen Anions. Geeignete Anionen sind z. B. Halogenid-Ionen $X^-$, wie Chlorid und Bromid. Bevorzugte Anionen sind Sulfat und Sulfonat, z. B. $SO_4^{2-}$, Tosylat, Trifluormethansulfonat und Methylsulfonat.

Die in diesem Kapitel ausgeführten Definitionen gelten auch für spezielle Aspekte der Erfindung, sofern nicht anders ausgeführt.

**c) Besondere Ausführungsformen der Offenbarung:**

Die vorliegende Offenbarung betrifft insbesondere (nur Rutheniumkatalysatoren sind erfindungsgemäss):

1. Verfahren zur reduktiven Aminierung einer Carbonyl-Verbindung, umfassend eine oder mehrere, wie z.B. 1, 2, 3, 4 oder 5, insbesondere 1, 2 oder 3, bevorzugt 1, reduktiv aminierbare Carbonylgruppen, dadurch gekennzeichnet, dass man die Reaktion in Gegenwart eines homogen gelösten Katalysators K sowie einer Säure als Kokatalysator durchführt, wobei K wenigstens ein Metallatom aus der Gruppe 8, 9 oder 10 des Periodensystems (IUPAC), insbesondere der Gruppe 8 oder 9, vorzugsweise der Gruppe 8 (Fe, Ru, Os), einen zweizähnigen Phosphanliganden, einen Carbonylliganden (CO), einen Hydridoliganden (H) und gegebenenfalls einen Neutralliganden ($L_N$) umfasst.

2. Verfahren nach Ausführungsform 1, wobei der Katalysator K ein Rutheniumkatalysator ist.

3. Verfahren nach einer der vorhergehenden Ausführungsformen, wobei der zweizähnige Phosphanligand folgende allgemeine Formel III aufweist:

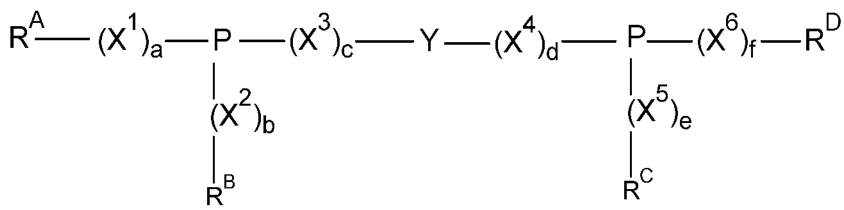

(III)

worin

a, b, c, d, e und f unabhängig voneinander für 0 oder 1 stehen,

$X^1$, $X^2$, $X^3$, $X^4$, $X^5$ und $X^6$ unabhängig voneinander für Alkylen, Arylen, -(C=O)-$NE^7$-Metallocenyl, Metallocenyl-

$NE^7$-(C=O)-, oder -(C=O)-$NE^7$-stehen, worin $E^7$ für H oder einen Alkyl-Rest steht; wobei dieser Rest und die Metallocenyl-, Alkylen- oder Arylen-Gruppe jeweils unabhängig voneinander gegebenenfalls ein- oder mehrfach substituiert sind, insbesondere 1, 2, 3, 4 oder 5 Substituenten, vorzugsweise 1 oder 2, tragen oder unsubstituiert sind, und wobei die Substituenten, unabhängig voneinander ausgewählt sind unter Alkyl, Cycloalkyl, Heterocyclyl, Aryl, Hetaryl, Alkoxy, Cycloalkoxy, Heterocycloalkoxy, Aryloxy, Hetaryloxy, Hydroxy, Mercapto, Polyalkylenoxid, Polyalkylenimin, COOH, Carboxylat, $SO_3H$, Sulfonat, Halogen, Nitro, Formyl, Acyl, Cyano, $NE^1E^2$, und $NE^1E^2E^{3+}X^-$, worin $E^1$, $E^2$ und $E^3$ jeweils gleiche oder verschiedene Reste, ausgewählt unter Wasserstoff, Alkyl, Cycloalkyl, oder Aryl bedeuten und $X^-$ für ein Anionäquivalent steht,

Y für eine zweiwertige kohlenstoffatomhaltige Brückengruppe steht, und

$R^A$, $R^B$, $R^C$ und $R^D$ unabhängig voneinander für Reste ausgewählt unter Alkyl, Cycloalkyl, Heterocycloalkyl, Aryl oder Hetaryl stehen, wobei diese Reste unabhängig voneinander gegebenenfalls ein- oder mehrfach substituiert sind, insbesondere 1, 2, 3, 4 oder 5 Substituenten, vorzugsweise 1 oder 2, tragen oder unsubstituiert sind, wobei die Substituenten unabhängig voneinander ausgewählt sind unter Alkyl, Cycloalkyl, Heterocyclyl, Aryl, Hetaryl, Alkoxy, Cycloalkoxy, Heterocycloalkoxy, Aryloxy, Hetaryloxy, Hydroxy, Mercapto, Polyalkylenoxid, Polyalkylenimin, COOH, Carboxylat, $SO_3H$, Sulfonat, Halogen, Nitro, Formyl, Acyl, Cyano, $NE^1E^2$, und $NE^1E^2E^{3+}X^-$, worin $E^1$, $E^2$ und $E^3$ jeweils gleiche oder verschiedene Reste, ausgewählt unter Wasserstoff, Alkyl, Cycloalkyl, oder Aryl bedeuten und $X^-$ für ein Anionäquivalent steht;
oder

$R^A$ und $R^B$ und/oder $R^C$ und $R^D$ zusammen mit dem Phosphoratom und, falls vorhanden, den Gruppen $X^1$, $X^2$, $X^5$ und $X^6$, an die sie gebunden sind, für einen 4- bis 8-gliedrigen Heterocyclus oder Heterobicyclus stehen, der gegebenenfalls zusätzlich insbesondere 1, 2, oder 3 Substituenten, vorzugsweise 1 oder 2 Substituenten, tragen oder unsubstituiert sind, mit Cycloalkyl, Heterocyclyl, Aryl oder Hetaryl anelliert ist, wobei der Heterocyclus oder Heterobicyclus und, falls vorhanden, die anellierten Gruppen unabhängig voneinander gegebenenfalls substituiert sind, insbesondere 1, 2, 3, 4 oder 5 Substituenten, vorzugsweise 1 oder 2 Substituenten, tragen oder unsubstituiert sind, wobei die Substituenten ausgewählt sind unter Alkyl, Cycloalkyl, Heterocyclyl, Aryl, Hetaryl, Hydroxy, Mercapto, Polyalkylenoxid, Polyalkylenimin, Alkoxy, Halogen, COOH, Carboxylat, $SO_3H$, Sulfonat, $NE^4E^5$, $NE^4E^5E^{6+}X^-$, Nitro, Alkoxycarbonyl, Formyl, Acyl und Cyano, worin $E^4$, $E^5$ und $E^6$ jeweils gleiche oder verschiedene Reste, ausgewählt unter Wasserstoff, Alkyl, Cycloalkyl und Aryl bedeuten und $X^-$ für ein Anionäquivalent steht;
oder

einer der Reste $R^A$ und $R^B$ und/oder einer der Reste $R^C$ und $R^D$ zusammen mit dem Phosphoratom und, falls vorhanden, den Gruppen $X^1$, $X^2$, $X^5$ und $X^6$, an die sie gebunden sind, **und zusammen mit einem Brückengruppenatom der Brückengruppe Y,** für einen 5- bis 8-gliedrigen Heterocyclus stehen, der gegebenenfalls zusätzlich insbesondere 1, 2, 3, 4 oder 5 Substituenten, vorzugsweise 1 oder 2 Substituenten, tragen oder unsubstituiert sind, mit Cycloalkyl, Heterocycloalkyl, Aryl oder Hetaryl anelliert ist, wobei der Heterocyclus und, falls vorhanden, die anellierten Gruppen unabhängig voneinander gegebenenfalls zusätzlich substituiert insbesondere 1, 2, 3, 4 oder 5 Substituenten, vorzugsweise 1 oder 2 Substituenten, tragen oder unsubstituiert sind, wobei die Substituenten ausgewählt sind unter Alkyl, Cycloalkyl, Heterocyclyl, Aryl, Hetaryl, Hydroxy, Mercapto, Polyalkylenoxid, Polyalkylenimin, Alkoxy, Halogen, COOH, Carboxylat, $SO_3H$, Sulfonat, $NE^4E^5$, $NE^4E^5E^{6+}X^-$, Nitro, Alkoxycarbonyl, Formyl, Acyl und Cyano, worin $E^4$, $E^5$ und $E^6$ jeweils gleiche oder verschiedene Reste, ausgewählt unter Wasserstoff, Alkyl, Cycloalkyl und Aryl bedeuten und $X^-$ für ein Anionäquivalent steht.

In obiger Formel III gelten insbesondere folgenden Bedeutungen:

Alkylen steht insbesondere für $C_1$-$C_{10}$-Alkylen;
Arylen steht insbesondere für $C_5$-$C_{20}$-Arylen;
Alkyl steht insbesondere für $C_1$-$C_{30}$-Alkyl;
Cycloalkyl steht insbesondere für $C_5$-$C_{30}$-Cycloalkyl;
Metallocenyl steht insbesondere für Ferrocenyl;
Heterocyclyl steht insbesondere für ein-, zwei-, oder dreikernige Gruppen mit 1 bis 30 Ring-Kohlenstoffatomen und 1 bis 4 Ringheteroatomen ausgewählt unter O, N und S; Heterocycloalkyl, steht insbesondere für ein-, zwei-, oder dreikernige Gruppen mit 1 bis 30 Ring-Kohlenstoffatomen und 1 bis 4 Ringheteroatomen ausgewählt

unter O, N und S, gebunden an einen $C_1$-$C_{30}$-Alkylrest;
Aryl steht insbesondere für ein-, zwei-, drei - oder vierkernige, gegebenenfalls substituierte aromatische Reste mit 5 bis 20 Ring-Kohlenstoffatomen;
Hetaryl steht insbesondere für eine Gruppe mit 1 bis 30 Ringkohlenstoffatomen und wenigstens einen fünf- bis siebengliedrigen aromatischen Ring;
Alkoxy steht insbesondere für die Sauerstoff-verknüpften Analoga obiger $C_1$-$C_{30}$-Alkyleste;
Cycloalkoxy steht insbesondere für die sauerstoffterminierten Analoga carbocyclischer, mono- oder polycyclischer, wie z.B. mono-, bi- oder tricyclischer, Reste mit 3 bis 30 Kohlenstoffatomen;
Heterocycloalkoxy steht insbesondere für obige $C_1$-$C_{30}$-Alkoxygruppen, welche wenigstens einen der oben genannten Heterocyclylreste als Substituenten tragen, der ein-, zwei-, oder dreikernigen Gruppen mit 1 bis 30 Ring-Kohlenstoffatomen und 1 bis 4 Ringheteroatomen, ausgewählt unter O, N und S aufweist;
Aryloxy steht insbesondere für die Sauerstoff-verknüpften Analoga obiger Arylreste mit 5 bis 20 Ringkohlenstoffatomen;
Hetaryloxy steht insbesondere für die Sauerstoff-verknüpften Analoga obiger Heteroarylreste mit 1 bis 30 Ringkohlenstoffatomen und wenigstens einen fünf- bis siebengliedrigen aromatischen Ring;
Polyalkylenoxid steht insbesondere für einen von gleichen oder verschiedenen $C_{2-4}$-Oxyalkylen-Monomerbausteinen abgeleiteten Rest mit einem Polymerisationsgrad von 2 bis 100;
Polyalkylenimin steht insbesondere für die strukturanalogen Imingruppen-haltigen Reste obiger Polyalkyennoxidreste;
Acyl steht insbesondere für Alkanoyl- oder Aroylgruppen mit 2 bis 11 Kohlenstoffatomen; und
Alkoxycarbonyl steht insbesondere für die Carbonyl-verknüpften Analoga obiger $C_1$-$C_{30}$-Alkoxyreste.

4. Verfahren nach Ausführungsform 3, wobei der zweizähnige Phosphanligand ausgewählt ist unter Verbindungen der allgemeinen Formel III,
worin

$X^1$, $X^2$, $X^3$, $X^4$, $X^5$ und $X^6$ unabhängig voneinander für gegebenenfalls substituiertes $C_1$-$C_6$-Alkylen, -(C=O)-$NE^7$-Ferrocenyl, Ferrocenyl-$NE^7$-(C=O)- oder -(C=O)-$NE^7$- stehen, worin $E^7$ die oben angegebenen Bedeutungen besitzt;

Y, a, b, c, d, e und f die oben angegebenen Bedeutungen besitzen;

$R^A$, $R^B$, $R^C$ und $R^D$ unabhängig voneinander für Reste, ausgewählt unter $C_1$-$C_{30}$-Alkyl, $C_4$-$C_{10}$-Cycloalkyl, $C_3$-$C_{10}$-Heterocyclyl, $C_5$-$C_{14}$-Aryl oder $C_5$-$C_{30}$-Hetaryl stehen, wobei diese Reste unabhängig voneinander gegebenenfalls ein- oder mehrfach substituiert sind, insbesondere 1, 2, 3, 4 oder 5, wie z.B. 1 oder 2 Substituenten tragen, wobei die Substituenten unabhängig voneinander ausgewählt sind unter $C_1$-$C_{10}$-Alkyl, $C_4$-$C_{10}$-Cycloalkyl, $C_3$-$C_{10}$-Heterocycloalkyl, $C_5$-$C_{14}$-Aryl, $C_5$-$C_{30}$-Hetaryl, $C_1$-$C_{10}$-Alkoxy, $C_3$-$C_{10}$-Cycloalkoxy, $C_3$-$C_{10}$-Heterocycloalkoxy, $C_5$-$C_{14}$-Aryloxy, $C_5$-$C_{30}$-Hetaryloxy, Hydroxy, Mercapto, Poly-$C_2$-$C_6$-alkylenoxid, Poly-$C_2$-$C_6$-alkylenimin, COOH, Carboxylat, $SO_3H$, Sulfonat, Halogen, Nitro, Formyl, $C_1$-$C_{10}$-Acyl, Cyano, $NE^1E^2$, und $NE^1E^2E^{3+}X^-$, worin $E^1$, $E^2$ und $E^3$ jeweils gleiche oder verschiedene Reste, ausgewählt unter Wasserstoff, $C_1$-$C_{10}$-Alkyl, $C_3$-$C_{10}$-Cycloalkyl, oder $C_5$-$C_{16}$-Aryl bedeuten und $X^-$ für ein Anionäquivalent steht;
oder

$R^A$ und $R^B$ und/oder $R^C$ und $R^D$ zusammen mit dem Phosphoratom und, falls vorhanden, den Gruppen $X^1$, $X^2$, $X^5$ und $X^6$, an die sie gebunden sind, für einen 4- bis 8-gliedrigen Heterocyclus oder Hetreobicyclus stehen, der gegebenenfalls zusätzlich, insbesondere 1- 2- oder 3-fach, mit $C_4$-$C_{10}$-Cycloalkyl, $C_3$-$C_{10}$-Heterocycloalkyl, $C_5$-$C_{14}$-Aryl oder $C_5$-$C_{30}$-Hetaryl anelliert ist, wobei der Heterocyclus oder Heterobicyclus und, falls vorhanden, die anellierten Gruppen unabhängig voneinander gegebenenfalls substituiert sind, insbesondere je einen, zwei, drei oder vier Substituenten tragen können, wobei die Substituenten ausgewählt sind unter $C_1$-$C_{10}$-Alkyl, $C_3$-$C_{10}$-Cycloalkyl, $C_3$-$C_{10}$-Heterocycloalkyl, $C_{5}$-$C_{14}$-Aryl, $C_{5}$-$C_{30}$-Hetaryl, Hydroxy, Mercapto, Poly-$C_2$-$C_6$-alkylenoxid, Poly-$C_2$-$C_6$-alkylenimin, $C_1$-$C_{10}$-Alkoxy, Halogen, COOH, Carboxylat, $SO_3H$, Sulfonat, $NE^4E^5$, $NE^4E^5E^{6+}X^-$, Nitro, $C_{1}$-$C_{10}$-Alkoxycarbonyl, Formyl, $C_{1}$-$C_{10}$-Acyl und Cyano, worin $E^4$, $E^5$ und $E^6$ jeweils gleiche oder verschiedene Reste, ausgewählt unter Wasserstoff, $C_1$-$C_{10}$-Alkyl, $C_3$-$C_{10}$-Cycloalkyl und $C_{5}$-$C1_4$-Aryl bedeuten und $X^-$ für ein Anionäquivalent steht;
oder

einer der Reste $R^A$ und $R^B$ und/oder einer der Reste $R^C$ und $R^D$ zusammen mit dem Phosphoratom und, falls vorhanden, den Gruppen $X^1$, $X^2$, $X^5$ und $X^6$, an die sie gebunden sind, und zusammen mit einem Brückengruppenatom der Brückengruppe Y, für einen 5- bis 8-gliedrigen Heterocyclus stehen, der gegebenenfalls zusätzlich, insbesondere 1- 2- oder 3-fach, mit $C_3$-$C_{10}$-Cycloalkyl, $C_3$-$C_{16}$-Heterocycloalkyl, $C_5$-$C_{14}$-Aryl oder $C_5$-$C_{30}$-Hetaryl anelliert ist, wobei der Heterocyclus und, falls vorhanden, die anellierten Gruppen unabhängig voneinander gegebenenfalls zusätzlich substituiert, insbesondere 1-, 2-, 3- oder 4-fach, sind, wobei die Substituenten ausgewählt sind unter $C_1$-$C_{10}$-Alkyl, $C_3$-$C_{16}$-Cycloalkyl, $C_3$-$C_{10}$-Heterocycloalkyl, $C_5$-$C_{14}$-Aryl, $C_5$-$C_{30}$-Hetaryl, Hydroxy, Mercapto, Poly-$C_2$-$C_6$-alkylenoxid, Poly-$C_2$-$C_6$-alkylenimin, $C_1$-$C_{10}$-Alkoxy, Halogen, COOH, Carboxylat, $SO_3H$, Sulfonat, $NE^4E^5$, $NE^4E^5E^{6+}X^-$, Nitro, $C_1$-$C_{10}$-Alkoxycarbonyl, Formyl, $C_1$-$C_{10}$-Acyl und Cyano, worin $E^4$, $E^5$ und $E^6$ jeweils gleiche oder verschiedene Reste, ausgewählt unter Wasserstoff, $C_1$-$C_{10}$-Alkyl, $C_3$-$C_{10}$-Cycloalkyl und $C_5$-$C_{14}$-Aryl bedeuten und $X^-$ für ein Anionäquivalent steht.

5. Verfahren nach einer der vorhergehenden Ausführungsformen, wobei in dem zweizähnigen Phosphanliganden der allgemeinen Formel III die Gruppe Y für eine Brückengruppe steht, ausgewählt unter

- geradkettiges oder verzweigtes Alkylen, insbesondere $C_1$-$C_6$-Alkylen, vorzugsweise $-CH_2-CH(CH_3)-$, $-CH(CH_3)-CH_2-$, $-CH(CH_3)-CH(CH_3)-$;
- geradkettiges oder verzweigtes Alkenylen, insbesondere $C_2$-$C_6$-Alkenylen,
- Cycloalkylen, insbesondere $C_3$-$C_{30}$-Cylcoalkylen, vorzugsweise $C_3$-$C_{12}$-Cylcoalkylen, gegebenenfalls enthaltend mindestens ein Ring-Heteroatom, insbesondere 1 oder 2 gleiche oder verschiedene, vorzugsweise gleiche, Ringheteroatome, ausgewählt aus N, O, S,
- Cycloalkenylen, insbesondere $C_3$-$C_{30}$-Cylcoalkyenlen, vorzugsweise $C_3$-$C_{12}$-Cylcoalkyenlen, gegebenenfalls enthaltend mindestens ein Ring-Heteroatom, insbesondere 1 oder 2 gleiche oder verschiedene, vorzugsweise gleiche, Ringheteroatome, ausgewählt aus N, O, S,
- bi-, tri- und tetracyclischen Brückengruppen;
- Arylen, insbesondere 1-, 2- oder 3-kerniges $C_5$-$C_{14}$-Arylen, vorzugsweise o-Phenylen ,
- Heteroarylen, insbesondere $C_5$-$C_6$-Heteroarylen, enthaltend mindestens ein Ring-Heteroatom, insbesondere 1 oder 2 gleiche oder verschiedene, vorzugsweise gleiche, Ringheteroatome, ausgewählt aus N, O und S,
- Heteroalkylen, insbesondere $C_2$-$C_6$-Heteroalkylen, enthaltend mindestens ein Ketten-Heteroatom, insbesondere 1 oder 2 gleiche oder verschiedene, vorzugsweise gleiche, Ringheteroatome, ausgewählt aus N, O und S,
- Heteroalkenylen, insbesondere $C_2$-$C_6$-Heteroalkenylen, enthaltend mindestens ein Ketten-Heteroatom, insbesondere 1 oder 2 gleiche oder verschiedene, vorzugsweise gleiche, Heteroatome, ausgewählt aus N, O und S,
- Mehrkernige, insbesondere 2-, 3-, 4, 5, oder 6-kernige Brückengruppen, umfassend wenigsten zwei miteinander verbundene aromatische, heteroaromatische, carbozyklische oder heterozyklische, 4- bis 8- gliedrige Ringe; und
- Metallocenbrückengruppen enthaltend wenigstens eine Metallocengruppe der allgemeinen Formel

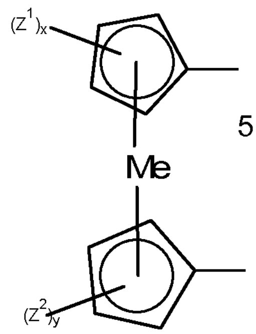

5

worin Me für ein Metallatom, insbesondere Fe, steht, wobei die P-Atome des Phosphanliganden am gleichen oder zwei verschiedenen Cp-Ringen des Metallocens, vorzugsweise an zwei verschiedenen, direkt oder über eine der Gruppen $X^3$ oder $X^4$, vorzugsweise direkt, gebunden sind, x und y unabhängig voneinander für 0, 1, 2, 3 oder 4 stehen und $Z^1$ und $Z^2$ unabhängig voneinander für H, gegebenenfalls ein- oder mehrfach substituiertes Alkyl, insbesondere $C_1$-$C_6$-Alkyl oder gegebenenfalls ein- oder mehrfach substituiertes Aryl, insbesondere $C_5$-$C_{12}$-Aryl stehen; und wobei die Substituenten unabhängig voneinander ausgewählt sind unter $C_1$-$C_{10}$-Alkyl, $C_4$-$C_{10}$-Cycloalkyl, $C_3$-$C_{16}$-Heterocycloalkyl, $C_5$-$C_{14}$-Aryl, $C_5$-$C_{30}$-Hetaryl, $C_1$-$C_{10}$-Alkoxy, $C_3$-$C_{16}$-Cycloalkoxy, $C_3$-$C_{10}$-Heterocycloalkoxy, $C_5$-$C_{14}$-Aryloxy, $C_5$-$C_{30}$-Hetaryloxy, Hydroxy, Mercapto, Poly-$C_2$-$C_6$-alkylenoxid, Poly-$C_2$-$C_6$-alkylenimin, COOH, Carboxylat, $SO_3H$, Sulfonat, Halogen, Nitro, Formyl, $C_1$-$C_{10}$-Acyl, Cyano, $NE^1E^2$, und $NE^1E^2E^{3+}X^-$, worin $E^1$, $E^2$ und $E^3$ jeweils gleiche oder verschiedene Reste, ausgewählt unter Wasserstoff, $C_1$-$C_{10}$-Alkyl, $C_3$-$C_{10}$-Cycloalkyl, oder $C_5$-$C_{10}$-Aryl bedeuten und $X^-$ für ein Anionäquivalent steht.

6. Verfahren nach einer der vorhergehenden Ausführungsformen, wobei in dem zweizähnigen Phosphanliganden der allgemeinen Formel III die Gruppe Y für eine mehrkernige Brückengruppe steht, ausgewählt unter Gruppen der Formeln III.a bis III.k

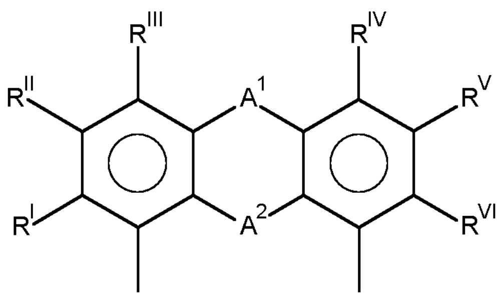

(III.a)

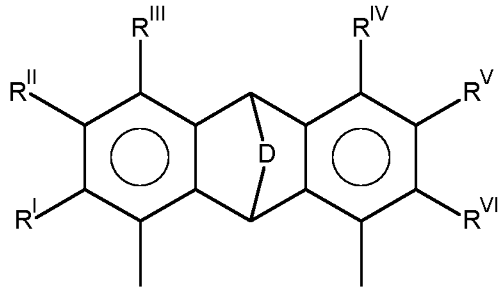

(III.b)

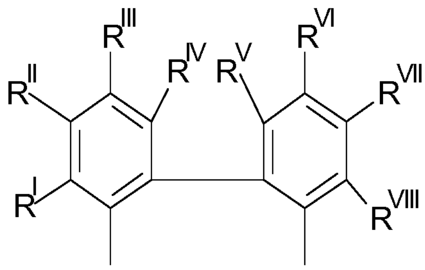

(III.c)

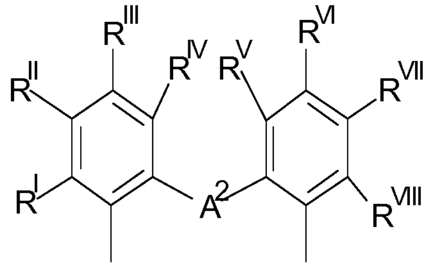

(III.d)

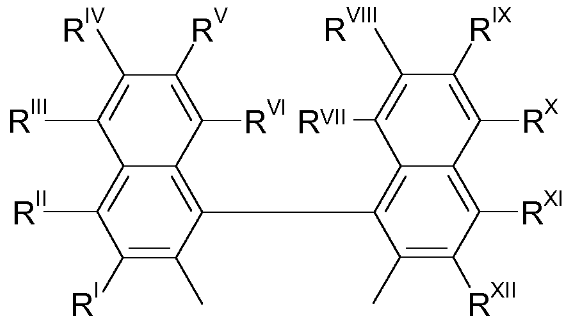

(III.e)

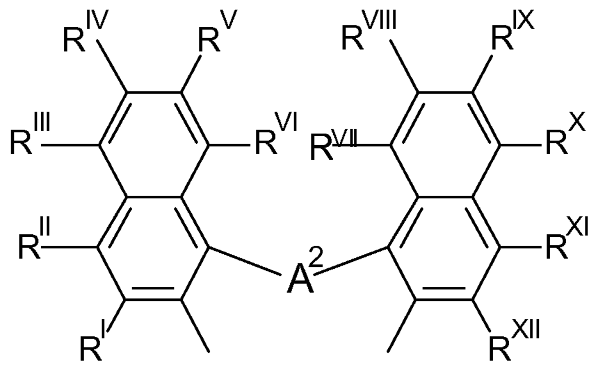

(III.f)

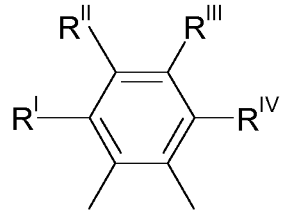

(III.g)

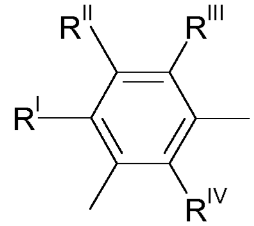

(III.h)

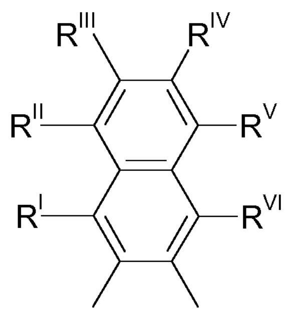

(III.i)

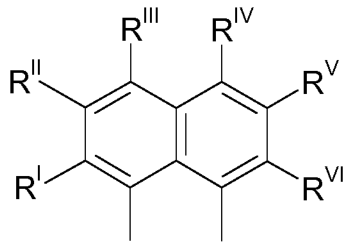

(III.k)

worin

$R^I$, $R^{II}$, $R^{III}$, $R^{IV}$, $R^V$, $R^{VI}$, $R^{VII}$, $R^{VIII}$, $R^{IX}$, $R^X$, $R^{XI}$ und $R^{XII}$ unabhängig voneinander für Wasserstoff, Alkyl, Cycloalkyl, Heterocycloalkyl, Aryl, Hetaryl, Hydroxy, Mercapto, Polyalkylenoxid, Polyalkylenimin, Alkoxy, Halogen, $SO_3H$, Sulfonat, $NE^9E^{10}$, Alkylen-$NE^9E^{10}$, Trifluormethyl, Nitro, Alkoxycarbonyl, Carboxyl, Formyl, Acyl oder Cyano stehen, worin $E^9$ und $E^{10}$ jeweils gleiche oder verschiedene Reste, ausgewählt unter Wasserstoff, Alkyl, Cycloalkyl und Aryl bedeuten,
oder

jeweils zwei benachbarte Reste $R^I$, $R^{II}$, $R^{III}$, $R^{IV}$, $R^V$, $R^{VI}$, $R^{VII}$, $R^{VIII}$, $R^{IX}$, $R^X$, $R^{XI}$ und $R^{XII}$ zusammen mit den Kohlenstoffatomen an die sie gebunden sind einen 4- bis 7-gliedrigen carbocyclischen oder heterozyclischen, aromatischen oder nichtaromatischen Ring bilden, der seinerseits gegebenenfalls mit einem ein- oder mehrkerigen carbocyclischen oder heterozyclischen, aromatischen oder nichtaromatischen Ring anelliert sein kann, wobei dieses Ringsystem gegebenenfalls, insbesondere 1-, 2- oder 3-fach substituiert ist, wobei die Substituenten ausgewählt sind unter Alkyl, Cycloalkyl, Heterocyclyl, Aryl, Hetaryl, Hydroxy, Mercapto, Polyalkylenoxid, Polyalkylenimin, Alkoxy, Halogen, COOH, Carboxylat, $SO_3H$, Sulfonat, $NE^4E^5$, $NE^4E^5E^{6+}X^-$, Nitro, Alkoxycarbonyl, Formyl, Acyl und Cyano, worin $E^4$, $E^5$ und $E^6$ jeweils gleiche oder verschiedene Reste, ausgewählt unter Wasserstoff, Alkyl, Cycloalkyl und Aryl bedeuten und $X^-$ für ein Anionäquivalent steht; wobei die zwei benachbarten Reste aus der Gruppe $R^I$, $R^{II}$, $R^{III}$, $R^{IV}$, $R^V$, $R^{VI}$, $R^{VII}$, $R^{VIII}$, $R^{IX}$, $R^X$, $R^{XI}$ und $R^{XII}$ an Kohlenstoffatome des gleichen aromatischen Rings oder an Kohlenstoffatome zweier verschiedener, vorzugsweise benachbarter aromatischer Ringe gebunden sein können, wie zum Beispiel $R^{IV}$ und $R^V$ in den Formeln (III.c) und (III.d) oder $R^{VI}$ und $R^{VII}$ oder $R^V$ und $R^{VIII}$ in den Formeln (III.e) und (III.f).

$A^1$ für eine Bindung, NH, $NR^{11}$, O, S, $CR^{11}$ oder $CR^{11}R^{12}$ steht, wobei $R^{11}$ und $R^{12}$ unabhängig voneinander ausgewählt sind unter Alkyl; Aryl und H;

$A^2$ für eine NH- Gruppe, N, O oder S steht,

D für eine zweibindige Brückengruppe der allgemeinen Formel

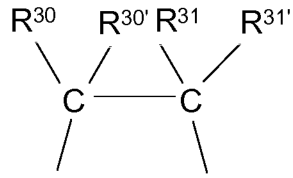

steht, in der

$R^{30}$, $R^{30'}$, $R^{31}$ und $R^{31'}$ unabhängig voneinander für Wasserstoff, Alkyl, Cycloalkyl, Aryl, Halogen, Trifluormethyl, Carboxyl, Carboxylat oder Cyano stehen,

wobei $R^{30'}$ auch gemeinsam mit $R^{31'}$ für den Bindungsanteil einer Doppelbindung zwischen den beiden Kohlenstoffatomen, an die $R^{30'}$ und $R^{31'}$ gebunden sind, stehen kann, und/oder $R^{30}$ und $R^{31}$ gemeinsam mit den Kohlenstoffatomen, an die sie gebunden sind, auch für einen 4- bis 8-gliedrigen Carbo- oder Heterocyclus stehen können, der gegebenenfalls zusätzlich ein-, zwei- oder dreifach mit Cycloalkyl, Heterocycloalkyl, Aryl oder Hetaryl anelliert ist, wobei der Carbo- oder Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, $COOR^e$, $COO^-M^+$, $SO_3R^e$, $SO_3^-M^+$, $NE^{11}E^{12}$, Alkylen-$NE^{11}E^{12}$, $NE^{11}E^{12}E^{13+}X^-$, Alkylen-$NE^{11}E^{12}E^{13+}X^-$, $OR^e$, $SR^e$, $(CHR^f\ CH_2O)_yR^e$, $(CH_2N(E^{11}))_yR^e$, $(CH_2CH_2N(E^{11}))_yR^e$, Halogen, Trifluormethyl, Nitro, Formyl, Acyl oder Cyano, stehen, worin

$R^e$, $E^{11}$, $E^{12}$ und $E^{13}$ jeweils gleiche oder verschiedene Reste, ausgewählt unter Wasserstoff, Alkyl, Cycloalkyl oder Aryl bedeuten,

$R^f$ für Wasserstoff, Methyl oder Ethyl steht,

$M^+$ für ein Kationäquivalent steht,

$X^-$ für ein Anionäquivalent steht, und

y für eine ganze Zahl von 1 bis 120 steht.

In obiger Formeln III.a bis III.k gelten insbesondere folgenden Bedeutungen:

Alkylen steht insbesondere für $C_1$-$C_{10}$-Alkylen;
Arylen steht insbesondere für $C_5$-$C_{20}$-Arylen;
Alkyl steht insbesondere für $C_1$-$C_{30}$-Alkyl;
Cycloalkyl steht insbesondere für $C_5$-$C_{30}$-Cycloalkyl;
Metallocenyl steht insbesondere für Ferrocenyl;
Heterocyclyl steht insbesondere für ein-, zwei-, oder dreikernige Gruppen mit 1 bis 30 Ring-Kohlenstoffatomen und 1 bis 4 Ringheteroatomen ausgewählt unter O, N und S; Heterocycloalkyl, steht insbesondere für ein-, zwei-, oder dreikernige Gruppen mit 1 bis 30 Ring-Kohlenstoffatomen und 1 bis 4 Ringheteroatomen ausgewählt unter O, N und S, gebunden an einen $C_1$-$C_{30}$-Alkylrest;

Aryl steht insbesondere für ein-, zwei-, drei - oder vierkernige, gegebenenfalls substituierte aromatische Reste mit 5 bis 20 Ring-Kohlenstoffatomen;
Hetaryl steht insbesondere für eine Gruppe mit 1 bis 30 Ringkohlenstoffatomen und wenigstens einen fünf- bis siebengliedrigen aromatischen Ring;
Alkoxy steht insbesondere für die Sauerstoff-verknüpften Analoga obiger $C_1$-$C_{30}$-Alkyleste;
Cycloalkoxy steht insbesondere für die sauerstoffterminierten Analoga carbocyclischer, mono- oder polycyclischer, wie z.B. mono-, bi- oder tricyclischer, Reste mit 3 bis 30 Kohlenstoffatomen;
Heterocycloalkoxy steht insbesondere für obige $C_1$-$C_{30}$-Alkoxygruppen, welche wenigstens einen der oben genannten Heterocyclylreste als Substituenten tragen, der ein-, zwei-, oder dreikernigen Gruppen mit 1 bis 30 Ring-Kohlenstoffatomen und 1 bis 4 Ringheteroatomen, ausgewählt unter O, N und S aufweist;
Aryloxy steht insbesondere für die Sauerstoff-verknüpften Analoga obiger Arylreste mit 5 bis 20 Ringkohlenstoffatomen;
Hetaryloxy steht insbesondere für die Sauerstoff-verknüpften Analoga obiger Heteroarylreste mit 1 bis 30 Ringkohlenstoffatomen und wenigstens einen fünf- bis siebengliedrigen aromatischen Ring;
Polyalkylenoxid steht insbesondere für einen von gleichen oder verschiedenen $C_{2-4}$-Oxyalkylen-Monomerbausteinen abgeleiteten Rest mit einem Polymerisationsgrad von 2 bis 100;
Polyalkylenimin steht insbesondere für die strukturanalogen Imingruppen-haltigen Reste obiger Polyalkyennoxidreste;
Acyl steht insbesondere für Alkanoyl- oder Aroylgruppen mit im 2 bis 11 Kohlenstoffatome; und
Alkoxycarbonyl steht insbesondere für die Carbonyl--verknüpften Analoga obiger $C_1$-$C_{30}$-Alkoxyreste.

Falls in Formel III.a $A^1$ für $CR^{11}$ (d.h. -C($R^{11}$)=) und $A^2$für N (d.h. -N=) steht, so erhält man eine Brückengruppe mit Acridinstruktur.

7. Verfahren nach einer der vorhergehenden Ausführungsformen, wobei in dem zweizähnigen Phosphanliganden der allgemeinen Formel III die Gruppe Y für eine Brückengruppe steht, ausgewählt unter polycyclischen Gruppen III.m bis III.o

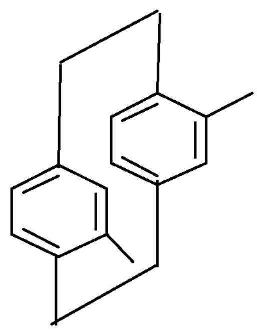

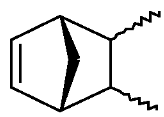

(III.n)

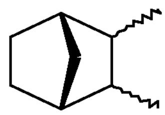

(III.o)

(III.m)

8. Verfahren nach einer der vorhergehenden Ausführungsformen, wobei in dem zweizähnigen Phosphanliganden der allgemeinen Formel III die Gruppe Y für eine Brückengruppe steht, ausgewählt unter den heterocyclischen Gruppen V.a bis V.g

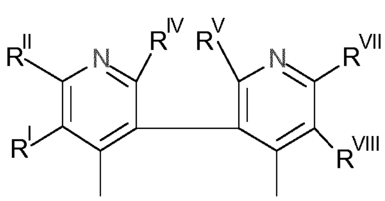

(V.a)

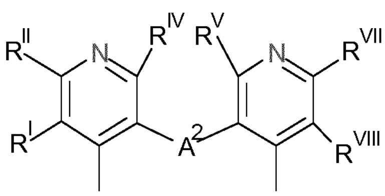

(V.b)

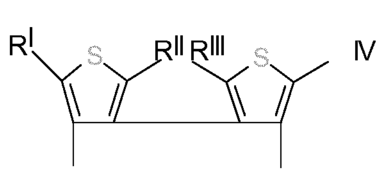

(V.c)

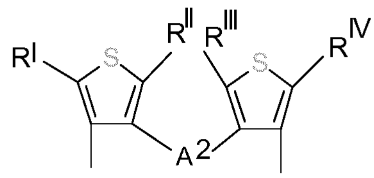

(V.d)

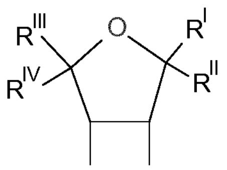

(V.e)

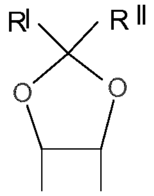

(V.f)

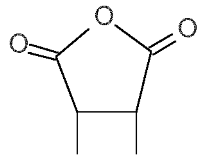

(V.g)

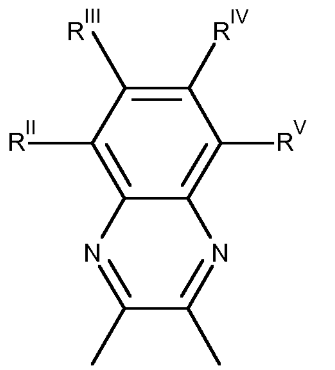

**(V.h)**

worin

die Reste $R^I$ bis $R^{VIII}$ unabhängig voneinander ausgewählt sind unter Wasserstoff, Alkyl, Cycloalkyl, Heterocycloalkyl, Aryl, Hetaryl, Hydroxy, Mercapto, Polyalkylenoxid, Polyalkylenimin, Alkoxy, Halogen, $SO_3H$, Sulfonat, $NE^9E^{10}$, Alkylen-$NE^9E^{10}$, Trifluormethyl, Nitro, Alkoxycarbonyl, Carboxyl, Formyl, Acyl oder Cyano stehen, worin $E^9$ und $E^{10}$ jeweils gleiche oder verschiedene Reste, ausgewählt unter Wasserstoff, Alkyl, Cycloalkyl und Aryl bedeuten; und
$A^2$ für NH, N, O oder S steht,

In obiger Formeln V.a bis V.h gelten insbesondere folgenden Bedeutungen:

Alkylen steht insbesondere für $C_1$-$C_{10}$-Alkylen;
Alkyl steht insbesondere für $C_1$-$C_{30}$-Alkyl;
Cycloalkyl steht insbesondere für $C_5$-$C_{30}$-Cycloalkyl;
Heterocycloalkyl, steht insbesondere für ein-, zwei-, oder dreikernige Gruppen mit 1 bis 30 Ring-Kohlenstoffatomen und 1 bis 4 Ringheteroatomen ausgewählt unter O, N und S, gebunden an einen $C_1$-$C_{30}$-Alkylrest;
Aryl steht insbesondere für ein-, zwei-, drei - oder vierkernige, gegebenenfalls substituierte aromatische Reste mit 5 bis 20 Ring-Kohlenstoffatomen;
Hetaryl steht insbesondere für eine Gruppe mit 1 bis 30 Ringkohlenstoffatomen und wenigstens einen fünf- bis siebengliedrigen aromatischen Ring;
Alkoxy steht insbesondere für die Sauerstoff-verknüpften Analoga obiger $C_1$-$C_{30}$-Alkyleste;

Polyalkylenoxid steht insbesondere für einen von gleichen oder verschiedenen $C_{2-4}$-Oxyalkylen-Monomerbausteinen abgeleiteten Rest mit einem Polymerisationsgrad von 2 bis 100;
Polyalkylenimin steht insbesondere für die strukturanalogen Imingruppen-haltigen Reste obiger Polyalkyennoxidreste; und
Acyl steht insbesondere für Alkanoyl- oder Aroylgruppen mit im 2 bis 11 Kohlenstoffatome; und
Alkoxycarbonyl steht insbesondere für die Carbonyl--verknüpften Analoga obiger $C_1$-$C_{30}$-Alkoxyreste.

9. Verfahren nach einer der vorhergehenden Ausführungsformen, wobei in dem zweizähnigen Phosphanliganden der allgemeinen Formel III die beiden Phosphanendgruppen unabhängig voneinander ausgewählt sind unter Gruppen der formeln VI.b, VI.d, VI.e, VI.f, VI.g, VI.h, VI.i und VI.k

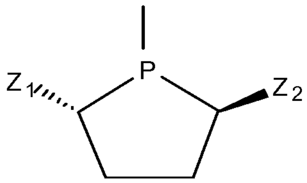

(VI.b)

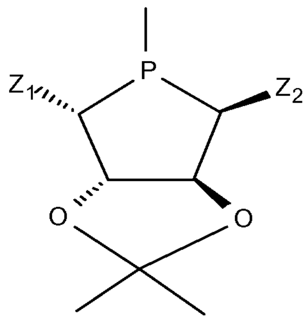

(VI.d)

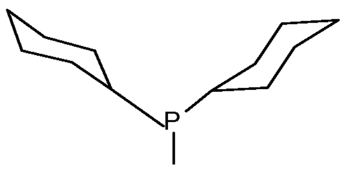

(VI.e)

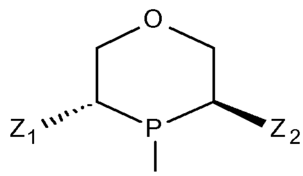

(VI.f)

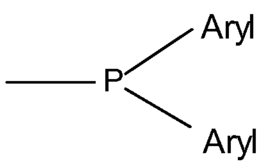

(VI.g)

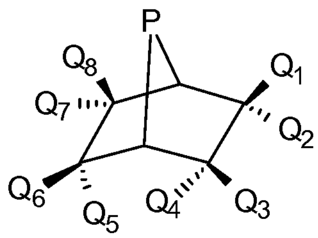

(VI.h)

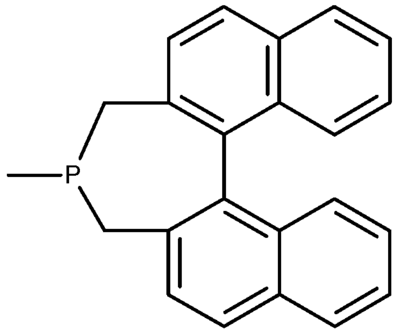

(VI.k)

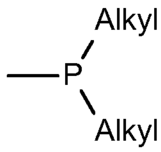

(VI.i)

worin

Aryl für gegebenenfalls ein oder mehrfach substituiertes Phenyl steht, wobei die Substituenten ausgewählt sind unter Alkyl oder Alkoxy
$Z_1$ und $Z_2$ unabhängig voneinander ausgewählt sind unter Alkyl, Alkoxy, Alkoxyalkyl, Aryl oder Hetaryl stehen und $Q_1$ bis $Q_8$ unabhängig voneinander für H oder Alkyl stehen.

In obigen Formeln VI.b bis VI.k gelten insbesondere folgenden Bedeutungen:

Alkyl steht insbesondere für $C_1$-$C_{30}$-Alkyl;
Alkoxy steht insbesondere für die Sauerstoff-verknüpften Analoga obiger $C_1$-$C_{30}$-Alkyleste;
Aryl steht insbesondere für ein-, zwei-, drei - oder vierkernige, gegebenenfalls substituierte aromatische Reste mit 5 bis 20 Ring-Kohlenstoffatomen;
Hetaryl steht insbesondere für eine Gruppe mit 1 bis 30 Ringkohlenstoffatomen und wenigstens einen fünf- bis siebengliedrigen aromatischen Ring;
Alkoxyalkyl steht insbesondere für obige $C_1$-$C_{30}$-Alkylgruppen, welche wenigstens einen der oben genannten $C_1$-$C_{30}$-Alkoxyreste als Substituenten tragen.

**Hinsichtlich obiger Ausführungsformen 3 bis 9** gelten allgemein folgende bevorzugte Bedeutungen in Verbindungen der Formel III:

(1) a, b, e und f stehen für den Wert 0 oder
(2) a, b, c, d, e und f stehen für den Wert 0
(3) $R^A$, $R^B$, $R^C$ und $R^D$ sind insbesondere gleich und stehen für gegebenenfalls 1- oder 2-fach substituiertes Aryl, insbesondere gegebenenfalls 1- oder 2-fach substituiertes Phenyl; Substituenten sind vorzugsweise $C_1$-$C_4$-Alkyleste
(4) Y steht für geradkettiges oder verzweigtes Alkylen mit 2 bis 5 Kohlenstoffatomen, insbesondere kann dabei die Brückengruppe 1 oder 2 Asymmetriezentren (Asymmetrische Kohlenstoffatome) aufweisen
(5) Y steht für eine Gruppe der Formel III.e worin $R^I$ bis $R^{XII}$ für H stehen
(6) Y steht für eine Gruppe der Formel III.d worin $R^I$ bis $R^{VIII}$ für H stehen und $A^2$ für O steht,
(7) Y steht für eine Gruppe der Formel III.g worin $R^I$ bis $RI^V$ für H stehen

Besonders bevorzugte Ausführungsformen für Liganden der Formel III umfassen Kombinationen der obigen Bedeutungen (1) oder (2) mit (3) und einer der Bedeutungen (4) bis (7)
**Hinsichtlich obiger Ausführungsform 8** gelten allgemein folgende bevorzugte Bedeutungen in Verbindungen der Formel V:

(1) $A^2$ steht für O oder S;
(2) $R^I$ bis $R^{VIII}$ stehen für H.

10. Verfahren nach einer der vorhergehenden Ausführungsformen, wobei der Säure-Kokatalysator eine Lewis-Säure ist.

11. Verfahren nach einer der vorhergehenden Ausführungsformen, wobei der Säure-Kokatalysator ausgewählt ist unter: $[NH_4]$TFA, $[NH_4]Cl$, $[NH_4]_2[SO_4]$, $[NH_4]Br$, $Al(OTf)_3$, $Sc(OTf)_3$, $AlCl_3$, $AlBr_3$, $BR_3$, $BCl_3$, $BBr_3$, $BF_3$ $NaPF_6$ und $Al(NO_3)_3$, insbesondere $[NH_4]$TFA, $Al(OTf)_3$, $Sc(OTf)_3$ und $AlCl_3$,.

12. Verfahren nach einer der vorhergehenden Ausführungsformen, wobei der Katalysator K ein Rutheniumkatalysator ist, der die folgende allgemeine Formel IV aufweist,

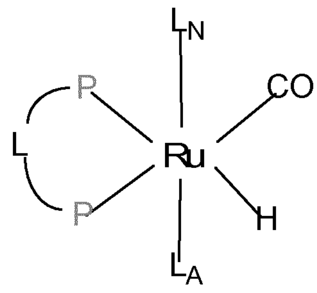

(IV)

worin

P-L-P der zweizähnige Phosphanligand die oben angegebenen Formel (III) ist;
$L_N$ für einen Neutralliganden, ausgewählt unter Aminen, Phosphanen, Phosphiten, Alkoholen, N-Heterocyclische Carbenen, Iminen, Carbonylverbindungen, Olefinen, Alkinen oder Nitrilen, insbesondere Phosphanen und Aminen, am meisten bevorzugt Triarylphosphanen und $NH_3$ steht, und
$L_A$ fehlt oder für einen anionischen Liganden, ausgewählt unter Chlorid, Fluorid, Bromid, Iodid, Hydrid, Cyanid, Alkoxy, Amido oder Hydroxy, insbesondere Chlorid oder Hydrid steht,

13. Verfahren nach einer der vorhergehenden Ausführungsformen, wobei man die Aminierung enantioselektiv durchführt, indem man einen Katalysator K einsetzt, der wenigstens einen chiralen zweizähnigen Phosphanliganden gemäß obiger Formel III trägt, insbesondere Liganden der Formeln (1) bis (86), und vor allem der Formeln (1), (9), (13), (20), (22) mit n = 3, (24) und (74).

14. Verfahren nach einer der vorhergehenden Ausführungsformen, wobei die zu aminierende Carbonylverbindung eine Verbindung der folgenden allgemeinen Formel II ist,

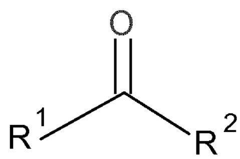

(II)

worin $R^1$ und $R^2$ gleich oder verschieden sind und unabhängig voneinander ausgewählt sind unter
H,
gegebenenfalls ein- oder mehrfach substituiertes Alkyl, insbesondere $C_1$-$C_{30}$-Alkyl, vorzugsweise $C_1$-$C_{10}$-Alkylreste, wobei die Kohlenstoffkette gegebenenfalls durch eine oder mehrere, wie z.B. 2, 3, 4 oder 5, insbesondere 1 oder 2, vorzugsweise 1, Carbonylgruppen unterbrochen ist; am meisten bevorzugt sind $C_1$-$C_{10}$-Alkylreste ohne weitere Carbonylgruppe;
gegebenenfalls ein- oder mehrfach substituiertes Cycloalkyl, insbesondere $C_3$-$C_{30}$-Cycloalkyl, vorzugsweise $C_3$-$C_{12}$-Cycloalkyl, wobei der carbocyclische Ring gegebenenfalls durch eine oder mehrere, wie z.B. 2, 3, 4

oder 5, insbesondere 1 oder 2, vorzugsweise 1, Carbonylgruppen unterbrochen ist; am meisten bevorzugt sind $C_3$-$C_{12}$-Cycloalkylreste ohne weitere Carbonylgruppe;
gegebenenfalls ein- oder mehrfach substituiertes Heterocyclyl, insbesondere $C_3$-$C_{30}$-Heterocyclyl, vorzugsweise $C_5$-$C_{12}$-Heterocyclyl, enthaltend mindestens ein Heteroatom ausgewählt aus N, O und S, wobei der heterocyclische Ring gegebenenfalls durch eine oder mehrere, wie z.B. 2, 3, 4 oder 5, insbesondere 1 oder 2, vorzugsweise 1, Carbonylgruppen unterbrochen ist; am meisten bevorzugt sind $C_3$-$C_{12}$-Heterocyclylreste ohne weitere Carbonylgruppe;
gegebenenfalls ein- oder mehrfach substituiertes 1-, 2- oder 3-kerniges Aryl, insbesondere $C_5$-$C_{14}$-Aryl; und
gegebenenfalls ein- oder mehrfach substituiertes 1-, 2- oder 3-kerniges Hetaryl, insbesondere $C_5$-$C_{14}$-Heteroaryl, enthaltend mindestens ein Heteroatom ausgewählt aus N, O und S;
wobei die gegebenenfalls vorhandenen Substituenten ausgewählt sind unter Halogen, insbesondere F, Cl, Br, -OH, -CN, -$NH_2$, $C_1$-$C_{30}$-Alkyl, $C_3$-$C_{30}$-Cycloalkyl, $C_3$-$C_{30}$-Heterocyclyl, enthaltend mindestens ein Heteroatom ausgewählt aus N, O und S, $C_5$-$C_{14}$-Aryl, $C_5$-$C_{14}$-Heteroaryl, enthaltend mindestens ein Heteroatom ausgewählt aus N, O und S, -$OR_7$, -$NHR_7$ oder -$N(R_7)_2$, -$C(O)R_7$,-$C(O)OR_7$, - $C(O)NHR_7$ oder -$C(O)N(R_7)_2$, wobei $R_7$ ausgewählt ist aus $C_1$-$C_{30}$-Alkyl und $C_5$-$C_{30}$-Aryl;
bevorzugt sind die Substituenten ausgewählt unter F, Cl, Br, -OH, -CN, -$NH_2$, $C_1$-$C_{10}$-Alkyl, $C_3$-$C_{12}$-Cycloalkyl, $C_5$-$C_{12}$-Heterocyclyl, enthaltend mindestens ein Heteroatom ausgewählt aus N, O und S, $C_5$-$C_{14}$-Aryl, $C_5$-$C_{14}$-Heteroaryl, enthaltend mindestens ein Heteroatom ausgewählt aus N, O und S, -$OR_7$, -$NHR_7$ oder -$N(R_7)_2$, -$C(O)R_7$,-$C(O)OR_7$, -$C(O)NHR_7$ oder -$C(O)N(R_7)_2$, wobei $R_7$ ausgewählt ist aus $C_1$-$C_{10}$-Alkyl und $C_5$-$C_{14}$-Aryl;
besonders bevorzugt sind die Substituenten ausgewählt unter F, Cl, Br, -OH, - CN, -$NH_2$, $C_1$-$C_4$-Alkyl, einkerniges Cycloalkyl, einkerniges Heterocyclyl, enthaltend mindestens ein Heteroatom ausgewählt aus N, O und S, einkerniges Aryl, einkörniges Heteroaryl, enthaltend mindestens ein Heteroatom ausgewählt aus N, O und S, -$OR_7$, -$NHR_7$ oder -$N(R_7)_2$, -$C(O)R_7$,-$C(O)OR_7$, -$C(O)NHR_7$ oder - $C(O)N(R_7)_2$, wobei $R_7$ ausgewählt ist aus $C_1$-$C_4$-Alkyl und einkerniges Aryl.

15. Verfahren nach einer der vorhergehenden Ausführungsformen, wobei der Katalysator K aus einer Katalysatorvorstufe, ausgewählt unter den folgenden Verbindungen gebildet wird:
$[Ru(PPh_3)_3(H)(Cl)(CO)]$, $[Ru(PPh_3)_3(H)_2(CO)]$, $[Ru(PPh_3)_3(Cl)_2]$, $[Ru(PPh_3)_3(Cl)_2(CO)]$, [Ru(p-cymene)$(Cl)_2$], [Ru(benzol)$Cl_2]_n$, $[Ru(CO)_2Cp]_n$ $[Ru(CO)_3(Cl)_2]$ [Ru(COD)(allyl)], [Ru(COD)(2-methylallyl)$_2$], $[RuCl_3{*}H_2O]$, [Ru(acteylacetonat)$_3$], [Ru(DMSO)$_4(Cl)_2$], $Ru_3CO_{12}$,[Ru(cyclopentadienyl)$(PPh_3)_2(Cl)$], Ru(cyclopentadienyl)$(CO)_2(Cl)$], [Ru(cyclopentadienyl)$(CO)_2H$], [Ru(cyclopentadienyl)$(CO)_2]_2$, [Ru(pentamethylcyclopentadienyl)$(CO)_2(Cl)$], [Ru(pentamethylcylcopentadienyl)$(CO)_2H$], [Ru(pentamethylcyclopentadienyl)$(CO)_2]_2$, [Ru(indenyl)$(CO)_2(Cl)$], [Ru(indenyl)$(CO)_2(H)$], [Ru(indenyl)$(CO)_2]_2$, Ruthenocen, [Ru(binap)$Cl_2$], [Ru(bipyridin)$_2Cl_2{*}2H_2O$], [Ru(COD)$Cl_2]_2$, [Ru(pentamethylcyclopentadienyl)(COD)(Cl)], $[Ru_3(CO)_{12}]$, [Ru(tetraphenylhydroxy-cyclopentadienyl)$(CO)_2(H)$], $[Ru(PPh_3)_4(H)_2]$.

16. Verfahren nach einer der vorhergehenden Ausführungsformen, wobei man den Katalysator K in einer Menge von 1 bis 5,000, 10 bis 2.000 oder 100 bis 1.000 ppm Gewichtsteilen, bezogen das Gesamtgewicht des eingesetzten flüssigen Reaktionsmediums einsetzt.

17. Verfahren nach einer der vorhergehenden Ausführungsformen, wobei man die Reaktion unter folgenden Reaktionsbedingungen durchführt:

a) Temperatur im Bereich von 20 bis 250, insbesondere 50 bis 150 °C und/oder
b) Gesamtdruck im Bereich von 0,1 bis 30, insbesondere 1 bis 10 oder MPa und/oder
c) Reaktionsdauer, 0,1 bis 100 h, insbesondere 5 bis 30 und/oder
d) Ammoniak: 1,5 bis 250-facher, insbesondere 10 bis 100-facher molarer Überschuss, bezogen auf Mol eingesetztem Edukt und/oder
e) Wasserstoff 1,5 bis 250-facher, insbesondere 10 bis 100-facher molarer Überschuss bezogen auf Mol eingesetztem Edukt.

**d) Edukte für die erfindungsgemäße reduktive Aminierungsreaktion**

Im erfindungsgemäßen Verfahren werden als Edukte Carbonyl-Verbindungen eingesetzt, die obige allgemeine Formel II aufweisen.

Dabei können die Substituenten $R^1$ und $R^2$ unabhängig voneinander wie oben definiert sein. Im erfindungsgemäßen Verfahren kann das Edukt auch mehrere, wie z.B. 2, 3, 4 oder 5, insbesondere 2, vorzugsweise jedoch nur eine

Carbonyleinheit als funktionelle Gruppen enthalten, welche aminiert werden. Falls $R^1$ und $R^2$ ungleich sind und keiner der beiden Substituenten Wasserstoff ist, dann ist es erfindungsgemäß auch möglich, diese Carbonylverbindung enantioselektiv zu aminieren.

$R^1$ und/oder $R^2$ können auch eine oder mehrere, wie z.B. 2, 3, 4 oder 5, insbesondere 2, vorzugsweise jedoch nur eine Carboxylgruppe oder deren Derivate, wie Amide, Ester und Nitrile enthalten, so dass durch reduktive Aminierung Aminosäuren-Gruppen erhalten werden können.

Als nicht-limitierende Beispiele für Edukte gemäß Formel II seien hier genannt: Acetophenon, Trifluoracetophenon, Trifluormethylacetophenon, Ketobutanol, Indanon, 2-Alkylindanonderivate, 2-(Benzyloxy)-cyclopentanon, (1-Naphtyl)-aceton, (2-Methoxyphenyl)-aceton, (2-Naphthyl)-aceton, (3,4-Dimethoxyphenyl)-aceton, (3-Bromphenyl)-aceton, (3-Chlorphenyl)-aceton, (3-Fluorphenyl)-aceton, (4-Methoxyphenyl)-aceton, (4-Methylphenyl)-aceton, (3-Pyridinyl)-aceton, (2-Pyridinyl)-aceton, (4-Pyridinyl)-aceton, Furanylaceton, Pyrrolyl-aceton, Pyrrolidinyl-aceton, Tetrahydrofuranyl-aceton, Cyclohexylaceton, Tetralinon, Cyclopropopylaceton, Cyclobutylaceton, Cyclopentylaceton, Phenyl-propyl-keton, Phenyl-butyl-keton, Phenyl-ethyl-keton, 2-Heptanon, 2-Hexanon, 2-Pentanon, 2-Butanon, 2-Octanon, 2-Nonanon, 2-Decanon, tertiär-Butyl-methyl-keton, Isopropyl-methylketon, Methoxyaceton, Ethoxyaceton, 2-Ketopropansäure(ester), 3-Ketobutansäure(ester).

**e) Komplexkatalysatoren**

Der erfindungsgemäße Komplexkatalysator K ist vorzugsweise ein Rutheniumkatalysator der die folgende allgemeine Formel IV aufweist, und wie oben definiert ist

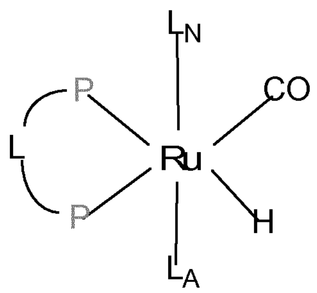

(IV)

Die hierin enthaltenen Liganden sind im Folgenden noch näher erläutert, wobei folgende Definitionen nicht nur für den konkreten Rutheniumkatalysator gelten e1) Phosphanliganden (P-L-P) (entsprechend obiger allgemeiner Formel III)

Als nichtlimitierende Beispiel erfindungsgemäß brauchbarere zweizähnige Phosphan-Liganden sind zu nennen:

Als erfindungsgemäße Phosphanliganden gemäß Formel III seien hier beispielhaft, aber nicht einschränkend, genannt: Diphenylphosphinomethan, 1,2-Bis(dimethylphosphino)ethan, 1,2-Bis(diisopropylphosphino)ethan, 1,2-Diphenylphosphinoethan, 1,3-Bis(diphenylphosphino)propan, (2,2-dimethyl-1,3-propanediyl)bis(diphenylphosphin), (2,2-dibutyl-1,3-propanediyl)bis(diphenylphosphin), Bis(diphenylphosphino)butan, Bis(diphenylphosphino)-9,9-dimethylxanthen, 2,3-Bis(dicyclohexylphosphino)ethan, ,1,1'-Bis(diphenylphosphino)ferrocen, 1,1'Bis(2-diphenylphosphinoethyl)phenylphosphin, Bis[2-(diphenylphosphanyl)phenyl] ether, Bis(2-diphenylphosphinoethyl)phenylphosphin, Bis[(2-diphenylphosphino-ethyl)ethyl]amin, 1,3-Bis(bis-o-methoxyphenyl-phosphino)-propan, 1,3-Bis(di-t-butylphosphinomethyl)benzene und auch noch Bis(dicyclohexylphosphino-methyl)-acridin.

Für die erfindungsgemäße Ausführungsform der enantioselektiven Aminierung der Carbonylverbindung werden chirale Phosphanliganden eingesetzt, welche Formel III entsprechen. Als erfindungsgemäße chirale Phosphanliganden gemäß Formel III seien hier beispielhaft, aber nicht einschränkend, genannt: Chiraphos (1), BINAP (13), f-Binaphan (74), sowie alle weiteren im Folgenden abgebildeten Phosphanliganden (1) - (86) sowie deren Enantiomere.

| | | | | | |
|---|---|---|---|---|---|
| 1 | 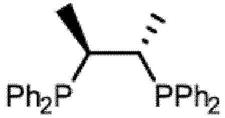 | 2 | 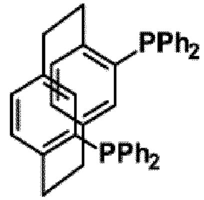 | 3 | 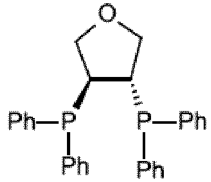 |
| 4 | 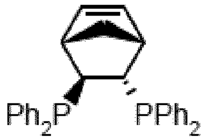 and enantiomer | 5 | 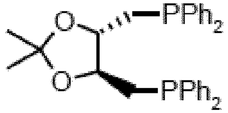 and enantiomer | 6 | 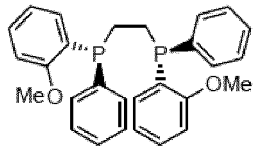 |
| 7 | 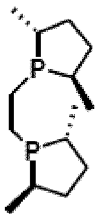 | 8 | 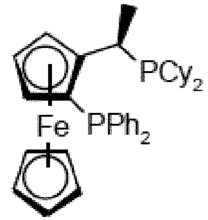 | 9 | 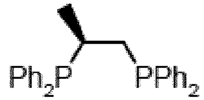 |
| 10 | 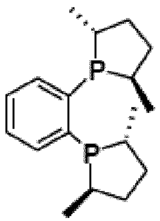 | | | 12 | 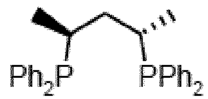 |
| 13 | 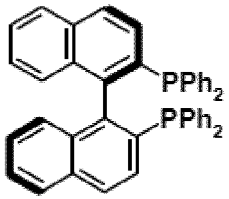 | 14 | 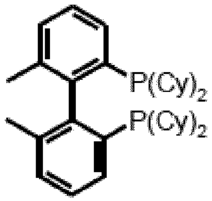 | 15 | 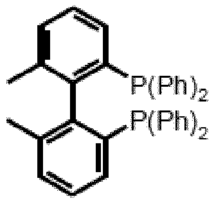 |

(fortgesetzt)

| | | | | | |
|---|---|---|---|---|---|
| 16 | 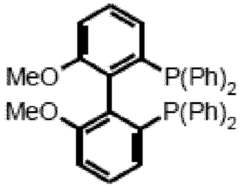 | 17 | 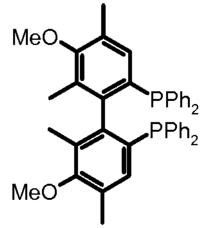 | 18 | 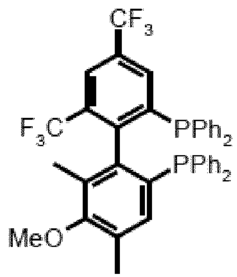 |
| 19 | 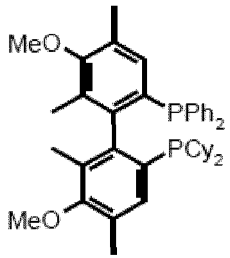 | 20 | 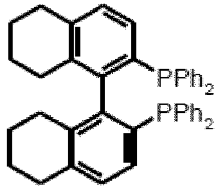 | 21 | 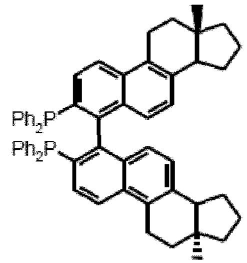 |
| 22 | 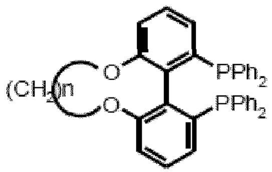 n = 1-6 | 23 | 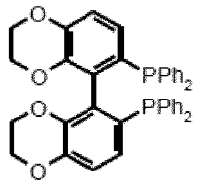 | 24 | 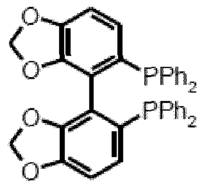 |
| 25 | 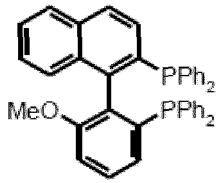 | 26 | 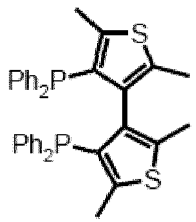 | 27 | 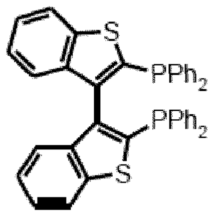 |

(fortgesetzt)

| | | | | | |
|---|---|---|---|---|---|
| 28 | 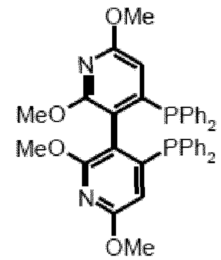 | 29 | 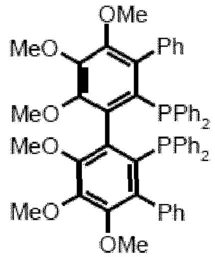 | 30 | 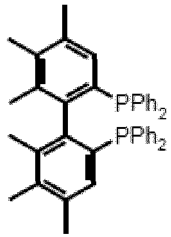 |
| 31 | 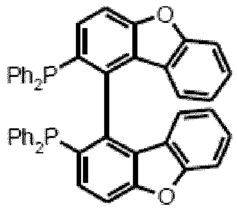 | 32 | 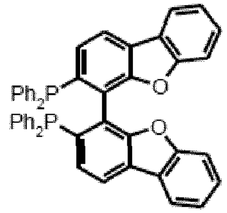 | 33 | 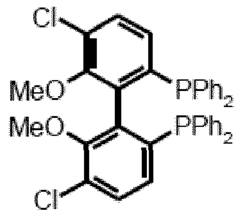 |
| 34 | 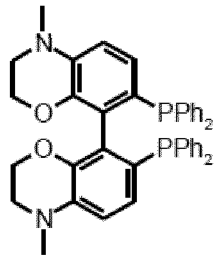 | 35 | 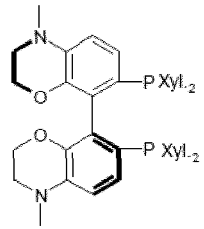 | 36 | 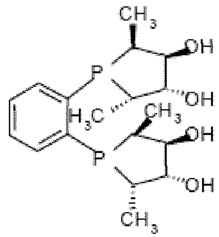 |
| 37 | 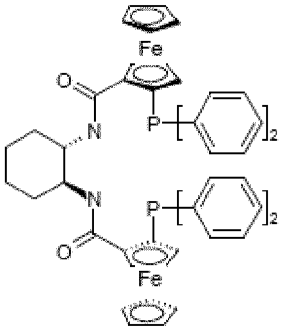 | 38 | 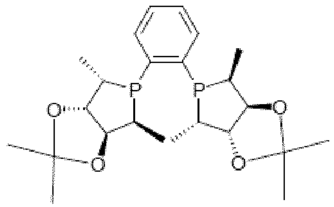 | 39 | 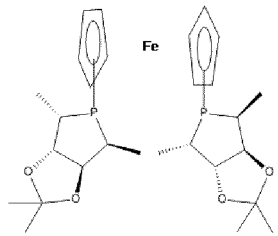 |

(fortgesetzt)

| | | | | | |
|---|---|---|---|---|---|
| 40 | 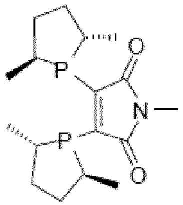 | 41 | 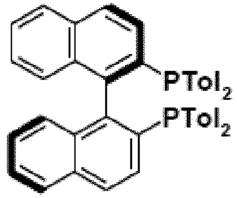 | 42 | 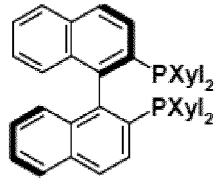 |
| 43 | 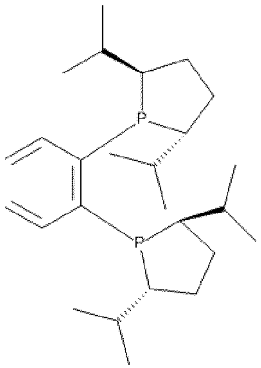 | 44 | 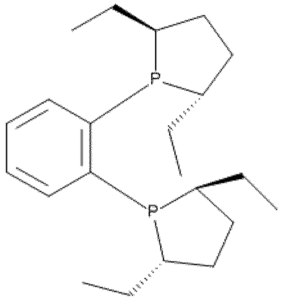 | 45 | 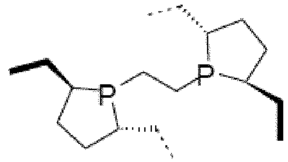 |
| 46 | 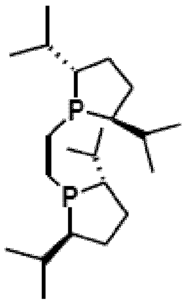 | 47 | 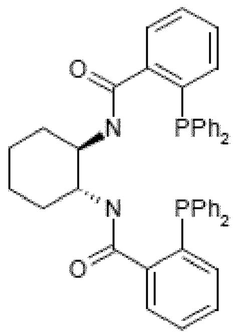 | 48 | 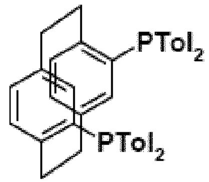 |

(fortgesetzt)

| | | | | | |
|---|---|---|---|---|---|
| 49 | 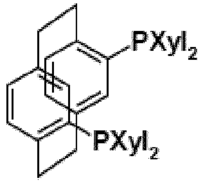 | 50 | 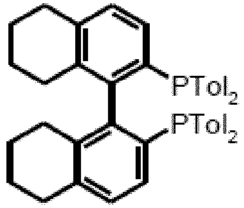 | 51 | 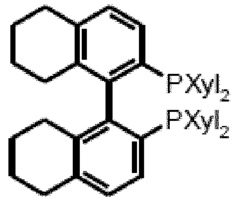 |
| 52 | 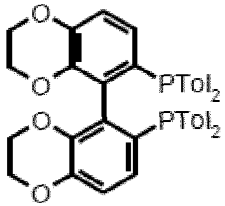 | 53 | 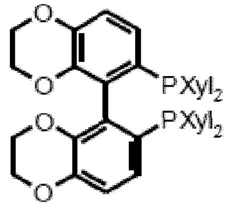 | 54 | 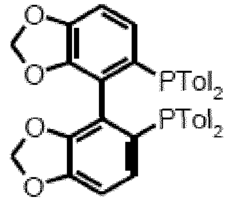 |
| 55 | 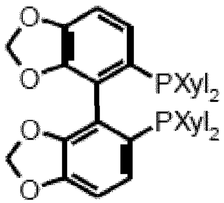 | 56 | 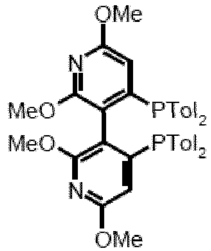 | 57 | 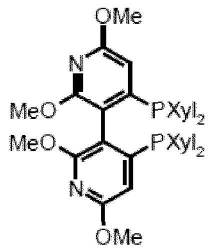 |
| 58 | 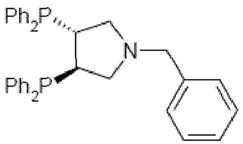 | 59 | 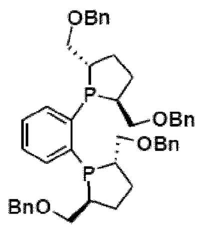 | 60 | 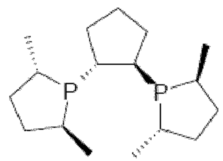 |

(fortgesetzt)

| | | | | | |
|---|---|---|---|---|---|
| 61 | 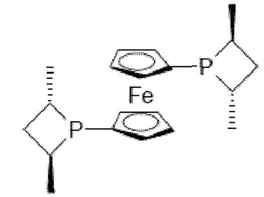 | 62 | 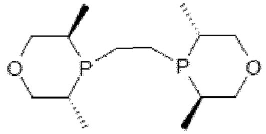 | 63 | 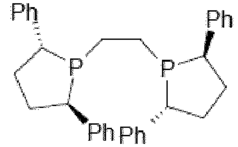 |
| 64 | 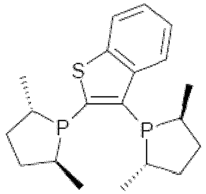 | 65 | 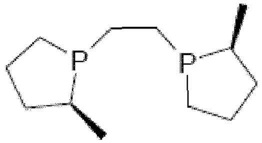 | 66 | 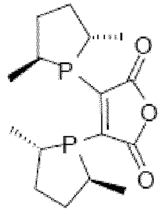 |
| 67 | 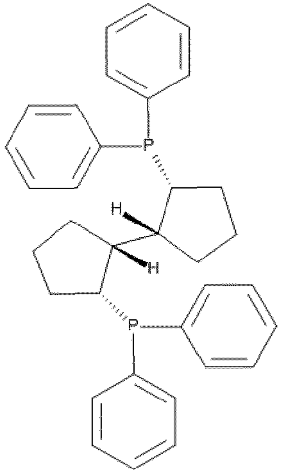 | 68 | 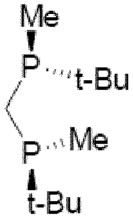 | 69 | 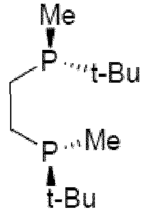 |
| 70 | 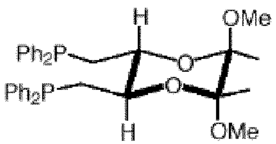 | 71 | 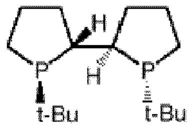 | 72 | 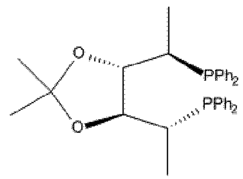 |

(fortgesetzt)

| | | | |
|---|---|---|---|
| 73 | 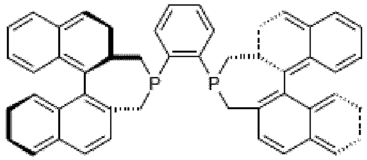 | 74 | 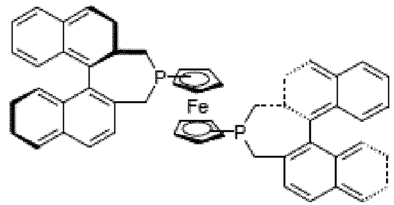 |
| 77 | 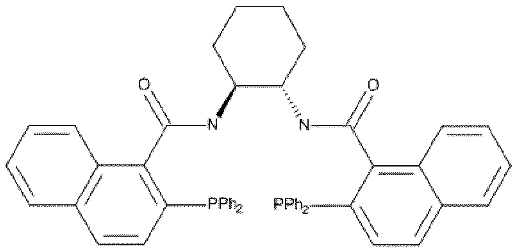 | 78 | 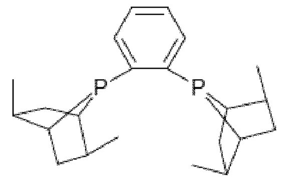 |
| 79 | 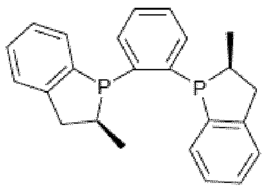 | 80 | 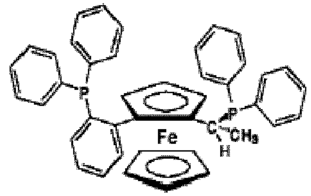 |

(fortgesetzt)

| | | | |
|---|---|---|---|
| | | 82 | 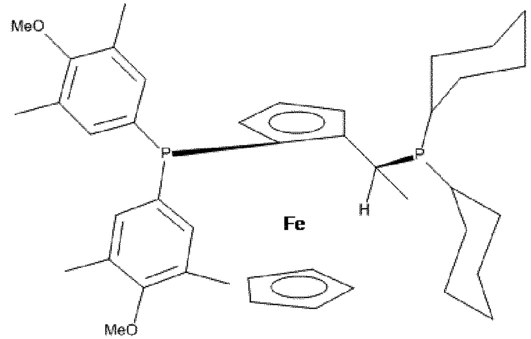 |
| | | 84 | 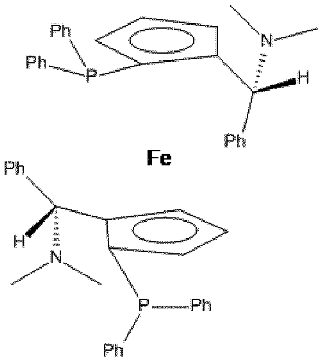 |
| 85 | 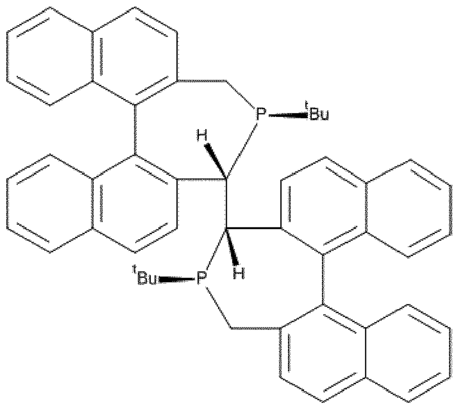 | 86 | 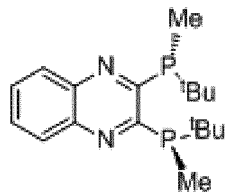 |

Cy steht für Cyclohexyl

e2) Neutralligand $L_N$

Der erfindungsgemäße Komplexkatalysator nach Formel IV kann wahlweise noch weitere Liganden tragen. Der Komplexkatalysator kann noch Neutralliganden $L_N$ tragen, wobei dieser nicht zwingend für das erfindungsgemäße Verfahren ist. Falls der Komplexkatalysator nach Formel IV einen Neutralliganden $L_N$ trägt, so wird dieser vorzugsweise, aber nicht einschränkend, ausgewählt unter Aminen, wie z.B. $C_1$-$C_{30}$- oder insbesondere $C_1$-$C_6$-Mono- oder Dialkylaminen, Phosphinen (i.e. Phosphanen), wie Trialkyl- oder Triaryl-Phosphanen, wie Tri-$C_1$-$C_{30}$-alkyl- oder Tri-$C_5$-$C_{14}$--aryl-Phosphanen, Phosphiten, Alkoholen, wie z.B. aliphatischen $C_1$-$C_{30}$- oder insbesondere $C_1$-$C_{6-}$-Mono-Alkoholen, N-heterocyclischen Carbenen, Iminen, Carbonylverbindungen, Olefinen, wie z.B. $C_2$-$C_{30}$- oder insbesondere $C_2$-$C_6$-Mono-Olefinen, Alkinen, wie z.B. $C_2$-$C_{30}$- oder insbesondere $C_2$-$C_6$-Monoalkinen, oder Nitrilen, wie z.B. $C_2$-$C_{30}$-oder insbesondere $C_2$-$C_6$-Monoalkylnitrilen. Insbesondere sind diese unter den genannten Aminen, Iminen, $NH_3$, Triaryl- oder Trialkylphosphanen ausgewählt.

3) Anionischer Ligand $L_A$

Der Komplexkatalysator kann noch einen anionischen Liganden $L_A$ tragen, wobei dieser nicht zwingend für das erfindungsgemäße Verfahren ist. Falls der Komplexkatalysator nach Formel IV einen Neutralliganden $L_N$ trägt, so wird dieser vorzugsweise, aber nicht einschränkend, ausgewählt aus Chlorid, Fluorid, Bromid, Iodid, Hydrid, Cyanid, Alkoxy, Amido oder Hydroxy, insbesondere Chlorid und Hydrid.

In einer bevorzugten Ausführungsform trägt der erfindungsgemäße Komplexkatalysator nach Formel IV einen Neutralliganden $L_N$ sowie einen anionische Liganden Y.

In einer besonders bevorzugten Ausführungsform handelt es sich bei $L_N$ um ein Triarylphosphan, wie Triphenylphosphan, oder $NH_3$ und bei $L_A$ um Chlorid oder Hydrid.

Der homogene Komplexkatalysator nach Formel IV kann sowohl direkt in seiner aktiven Form als auch ausgehend von üblichen Vorstufen unter Zugabe der entsprechenden Liganden erst unter den Reaktionsbedingungen (in situ) erzeugt werden. Nichtlimitierende Beispiele üblicher Vorstufen von Ruthenium-Komplexkatalysatoren sind: $[Ru(PPh_3)_3(H)(Cl)(CO)]$, $[Ru(PPh_3)_3(H)_2(CO)]$, $[Ru(PPh_3)_3(Cl)_2]$, $[Ru(PPh_3)_3(Cl)_2(CO)]$, [Ru(p-cymene)$(Cl)_2$], [Ru(benzol)$Cl_2]_n$, $[Ru(CO)_2Cp]_n$, $[Ru(CO)_3(Cl)_2]$[Ru(COD)(allyl)], [Ru(COD)(2-methylallyl)$_2$], $[RuCl_3{*}H_2O]$, [Ru(acteylacetonat)$_3$], [Ru(DMSO)$_4(Cl)_2$], $Ru_3CO_{12}$,[Ru(cyclopentadienyl)$(PPh_3)_2$(Cl)]Ru(cyclopentadienyl)$(CO)_2$(Cl)], [Ru(cyclopentadienyl)$(CO)_2$H], [Ru(cyclopentadienyl)$(CO)_2]_2$, [Ru(pentamethylcyclopentadienyl)$(CO)_2$(Cl)], [Ru(pentamethylcylcopentadienyl)$(CO)_2$H], [Ru(pentamethylcyclopentadienyl)$(CO)_2]_2$, [Ru(indenyl)$(CO)_2$(Cl)], [Ru(indenyl)$(CO)_2$(H)], [Ru(indenyl)$(CO)_2]_2$, Ruthenocen, [Ru(binap)$Cl_2$], [Ru(bipyridin)$_2Cl_2{*}2H_2O$], [Ru(COD)$Cl_2]_2$, [Ru(pentamethylcyclopentadienyl)(COD)(Cl)], $[Ru_3(CO)_{12}]$, [Ru(tetraphenylhydroxy-cyclopentadienyl)$(CO)_2$(H)], $[Ru(PPh_3)_4(H)_2]$.

**f) Kokatalysator**

Erfindungsgemäß wird die Reaktion insbesondere in der Anwesenheit einer Säure als Kokatalysator durchführt. Bei der Säure kann es sich hierbei um eine Bronsted- oder Lewis-Säure handeln. Der saure Kokatalysator kann in gelöster, in teilweise gelöster oder in ungelöster Form im Reaktionsgemisch vorliegen. Als Kokatalysator eignen sich hierbei verschiedenste saure Verbindungen, wie HCl, $H_2SO_4$, $H_3PO_4$, $HSO_3R$, HBr, HI, HF, $[NH_4]$TFA, $[NH_4]$Cl, $[NH_4]_2[SO_4]$, $[NH_4]$Br, $Al(OTf)_3$, $Sc(OTf)_3$, $AlCl_3$, $AlBr_3$, $Al(OR)_3$, $BR_3$, $BCl_3$, $BBr_3$, $BF_3$, $Al(NO_3)_3$, $NaPF_6$, $NaBF_4$, saure Ionenaustauscher, $TiO_2$ (sauer), $Al_2O_3$ (sauer), $SiO_2$, $TiCl_4$, $TiBr_4$, $TiI_4$, $Ti(OR)_4$, $ZrCl_4$. $ZrBr_4$, $ZrI_4$, $ZR(OR)_4$, $H_2CO_3$, $CO_2$, HCOOH, AcOH, $SO_3$, $SO_2$, oder auch saure ionische Flüssigkeiten, wie z.B. 1,3-Alkylmethyl-imidazolium- oder N-Alkylpyridinium-Salze, wie z.B. 1,3-Butyl-Methyl-imidazolium (BMIM)-Salze, wobei das Gegenion je nach gewünschter Acidität z.B. ausgewählt ist unter $MeSO_3^-$, $AlCl_4^-$, $CuCl_2^-$,$SbF_4^-$, $SbF_6^-$, $BF_4$, $PF_6^-$, $Al_2Cl_7^-$, $Cu_2Cl_3^-$, $Al_3Cl_{10}^-$ oder $Cu_3Cl_4^-$

Die Menge des Kokatalysators beträgt im Allgemeinen 0,001 bis 10 Gewichts-% jeweils bezogen auf das gesamte flüssige Reaktionsmedium.

**g) Durchführung der Reaktion**

Die Reaktion erfolgt in der Flüssigphase im Allgemeinen bei einer Temperatur von 20 bis 250°C. Bevorzugt wird das erfindungsgemäße Verfahren bei Temperaturen im Bereich von 100 °C bis 200 °C, besonders bevorzugt im Bereich von 110 bis 160°C durchgeführt.

Die Reaktion kann im Allgemeinen bei einem Gesamtdruck von 0,1 bis 20 MPa absolut, der sowohl dem Eigendruck des Lösungsmittels bei der Reaktionstemperatur als auch dem Druck des Ammoniaks und Wasserstoffes entspricht, durchgeführt werden. Bevorzugt wird das erfindungsgemäße Verfahren bei einem Gesamtdruck im Bereich

von 0,1 bis 30 MPa absolut, insbesondere bevorzugt bei einem Gesamtdruck im Bereich von 1 bis 10 MPa absolut durchgeführt.

Die mittlere Reaktionszeit beträgt im Allgemeinen 15 Minuten bis 100 Stunden, wie z.B. 1 bis 50 oder 5 bis 20 Stunden.

Das Aminierungsmittel (Ammoniak) kann bezüglich der zu aminierenden Carbonylgruppen in stöchiometrischen, unterstöchiometrischen oder überstöchiometrischen Mengen eingesetzt werden. In einer bevorzugten Ausführungsform wird Ammoniak mit einem 1,5- bis 250-fachen, bevorzugt mit einem 2- bis 100-fachen, insbesondere mit einem 2- bis 10-fachen molaren Überschuss pro Mol im Edukt umzusetzender Carbonylgruppe eingesetzt. Es sind auch höhere Überschüsse an Ammoniak möglich.

Das Reduktionsmittel (Wasserstoff) kann bezüglich der zu aminierenden Carbonylgruppen in stöchiometrischen, unterstöchiometrischen oder überstöchiometrischen Mengen eingesetzt werden. In einer bevorzugten Ausführungsform wird Wasserstoff mit einem 1,5- bis 250-fachen, bevorzugt mit einem 2- bis 100-fachen, insbesondere mit einem 2- bis 10-fachen molaren Überschuss pro Mol im Edukt umzusetzender Carbonylgruppe eingesetzt. Es sind auch höhere Überschüsse an Wasserstoff möglich.

Das erfindungsgemäße Verfahren kann sowohl in einem Lösungsmittel als auch ohne Lösungsmittel durchgeführt werden. Als Lösungsmittel eignen sich polare und unpolare Lösungsmittel, die in Reinform oder in Mischungen eingesetzt werden können. Beispielsweise kann im erfindungsgemäßen Verfahren nur ein unpolares oder ein polares Lösungsmittel eingesetzt werden. Es ist auch möglich, Mischungen von zwei oder mehr polaren Lösungsmitteln oder Mischungen von zwei oder mehr unpolaren Lösungsmitteln oder Mischungen aus einem oder mehr polaren mit einem oder mehr unpolaren Lösungsmitteln einzusetzen.

Als unpolare Lösungsmittel eignen sich beispielsweise gesättigte und ungesättigte Kohlenwasserstoffe, wie Hexan, Heptan, Oktan, Cyclohexan, Benzol, Toluol, Xylol und Mesitylen, sowie lineare und zyklische Ether, wie THF, Diethylether, 1,4-Dioxan, MTBE (tert-Butylmethylether), Diglyme und 1,2-Dimethoxyethan. Bevorzugt werden Toluol, Xylole oder Mesitylen eingesetzt.

Als polare Lösungsmittel eignen sich beispielsweise Wasser, Dimethylformamid, Formamid, tert-Amylalkohol und Acetonitril. Bevorzugt wird Wasser eingesetzt. Das Wasser kann sowohl vor der Reaktion zugesetzt werden, bei der Reaktion als Reaktionswasser entstehen oder auch nach der Reaktion zusätzlich zum Reaktionswasser zugesetzt werden.

Je nach Polarität des Produkts und des Eduktes, können auch Produkt und/oder Edukt als Lösungsmittel (in Reinform oder im Gemisch mit wenigstens einem der oben genannten polaren oder apolaren Lösungsmittel) für die Reaktion genutzt werden.

Gegebenenfalls können auch Ionische Flüssigkeiten (IL), wie z.B. die oben genannten sauren IL's als Lösungsmittel eingesetzt werden.

Für die Umsetzung in der Flüssigphase leitet man Ammoniak, Wasserstoff, das mindestens eine funktionelle Gruppe der Formel (-C=O-) aufweisende Edukt, gegebenenfalls zusammen mit einem oder mehreren Lösungsmitteln, zusammen mit dem Komplexkatalysator und dem Kokatalysator in einen geeigneten Reaktor.

Die Zuleitung von Ammoniak, Wasserstoff, Edukt, gegebenenfalls Lösungsmittel und Komplexkatalysator kann dabei simultan oder getrennt voneinander erfolgen. Die Reaktion kann dabei kontinuierlich, in semibatch-Fahrweise, in batch-Fahrweise, rückvermischt in Produkt als Lösungsmittel oder im geraden Durchgang nicht rückvermischt durchgeführt werden. In einer weiteren Ausführungsform kann dem Reaktionsgemisch noch CO zugeführt, um den Katalysator in seiner aktiven Form zu halten.

Für das erfindungsgemäße Verfahren können prinzipiell alle Reaktoren eingesetzt werden, welche für gas/flüssig-Reaktionen unter der gegebenen Temperatur und dem gegebenen Druck grundsätzlich geeignet sind. Geeignete Standardreaktoren für gas/flüssig- und für flüssig/flüssig-Reaktionssysteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren.

Der bei der Umsetzung entstehende Reaktionsaustrag enthält im Allgemeinen die entsprechenden Reaktionsprodukte (d.h. Aminierungsprodukte und gegebenenfalls anfallende Nebenprodukte), gegebenenfalls das eine oder die mehreren Lösungsmittel, den Komplexkatalysator, den sauren Kokatalysator und gegebenenfalls nicht umgesetzte Edukte, Ammoniak und Wasserstoff sowie das entstandene Reaktionswasser. Aus dem Reaktionsaustrag werden der gegebenenfalls vorhandene überschüssige Ammoniak, das gegebenenfalls vorhandene Lösungsmittel, der Komplexkatalysator und das Reaktionswasser entfernt. Das erhaltene Reaktionsprodukt kann weiter aufgearbeitet werden. Der überschüssige Ammoniak, der Komplexkatalysator, der saure Kokatalysator, gegebenenfalls das oder die Lösungsmittel und gegebenenfalls nicht umgesetzte Edukte können in die Aminierungsreaktion zurückgeführt werden.

Wird die Aminierungsreaktion ohne Lösungsmittel durchgeführt, so ist der homogene Komplexkatalysator nach der Reaktion im Produkt gelöst. Dieser kann im Produkt verbleiben oder durch eine geeignete Methode aus diesem abgetrennt werden. Möglichkeiten zur Abtrennung des Katalysators sind zum Beispiel das Auswaschen mit einem nicht

mit dem Produkt mischbaren Lösungsmittel, in welchem sich der Katalysator durch geeignete Wahl der Liganden besser löst als im Produkt. Gegebenenfalls wird der Katalysator durch mehrstufige Extraktion aus dem Produkt abgereichert. Als Extraktionsmittel wird bevorzugt ein auch für die Zielreaktion geeignetes Lösungsmittel wie Toluol, Benzol, Xylole, Alkane, wie Hexane, Heptane und Oktane, und azyklische oder zyklische Ether, wie Diethylether und Tetrahydrofuran eingesetzt, welches nach dem Einengen zusammen mit dem extrahierten Katalysator wieder für die Umsetzung eingesetzt werden kann. Möglich ist auch die Entfernung des Katalysators mit einem geeigneten Absorbermaterial. Eine Abtrennung kann auch durch Zufügen von Wasser zu der Produktphase erfolgen, falls die Reaktion in einem mit Wasser nicht mischbaren Lösungsmittel durchgeführt wird. Löst sich der Katalysator dabei bevorzugt in dem Lösungsmittel, kann er mit dem Lösungsmittel von der wässrigen Produktphase abgetrennt und gegebenenfalls erneut eingesetzt werden. Dies kann durch Wahl geeigneter Liganden bewirkt werden. Es ist auch möglich, das Aminierungsprodukt durch Destillation vom Katalysator zu trennen.

Wird die Reaktion in einem Lösungsmittels durchgeführt, so kann dieses mit dem Aminierungsprodukt mischbar sein und nach der Reaktion durch Destillation abgetrennt werden. Es können auch Lösungsmittel eingesetzt werden, welche eine Mischungslücke mit den Aminierungsprodukten oder den Edukten aufweisen. Als geeignete Lösungsmittel hierfür seien beispielsweise Toluol, Benzol, Xylole, Alkane, wie Hexane, Heptane und Oktane, und azyklische oder zyklische Ether, wie Diethylether, Tetrahydrofuran und Dioxan genannt. Durch geeignete Wahl der Phosphanliganden löst sich der Katalysator bevorzugt in der Lösungsmittelphase, d.h. in der nicht-Produkt- enthaltenden Phase. Die Phosphanliganden können auch so gewählt werden, dass sich der Katalysator im Aminierungsprodukt löst. In diesem Fall lässt sich das Aminierungsprodukt durch Destillation vom Katalysator trennen.

Das Lösungsmittel kann auch unter den Reaktionsbedingungen mit den Edukten und dem Produkt mischbar sein und erst nach dem Abkühlen eine zweite flüssige Phase ausbilden, welche den Großteil des Katalysators enthält. Als Lösungsmittel, die diese Eigenschaft zeigen, seien beispielsweise Toluol, Benzol, Xylole, Alkane, wie Hexane, Heptane und Oktane, genannt. Der Katalysator kann dann zusammen mit dem Lösungsmittel abgetrennt und wieder verwendet werden. Die Produktphase kann auch in dieser Variante mit Wasser versetzt werden. Der in dem Produkt enthaltene Teil des Katalysators kann anschließend durch geeignete Absorbermaterialien wie beispielsweise Polyacrylsäure und deren Salze, sulfonierte Polystyrole und deren Salze, Aktivkohlen, Montmorillonite, Bentonite sowie Zeolithe abgetrennt werden oder aber in dem Produkt belassen werden.

Die Aminierungsreaktion kann auch zweiphasig durchgeführt werden. Bei der Ausführungsform der zweiphasigen Reaktionsführung eignen sich als unpolare Lösungsmittel besonders Toluol, Benzol, Xylole, Alkane, wie Hexane, Heptane und Oktane, in Kombination mit lipophilen Phosphanliganden am Übergangsmetallkatalysator, wodurch sich der Übergangsmetallkatalysator in der unpolaren Phase anreichert. Bei dieser Ausführungsform, in welcher das Produkt sowie das Reaktionswasser und ggf. nicht umgesetzte Edukte eine mit diesen Verbindungen angereicherte Zweitphase bilden, kann der Großteil des Katalysators durch einfache Phasentrennung von der Produktphase abgetrennt und wiederverwendet werden.

Falls flüchtige Nebenprodukte oder nicht umgesetzte Edukte oder auch das bei der Reaktion entstandene oder nach der Reaktion zur besseren Extraktion zugesetzte Wasser unerwünscht sind, können diese problemlos von dem Produkt durch Destillation abgetrennt werden.

Es kann auch vorteilhaft sein, das bei der Reaktion gebildete Wasser kontinuierlich aus dem Reaktionsgemisch zu entfernen. Das Reaktionswasser kann direkt durch Destillation aus dem Reaktionsgemisch oder als Azeotrop unter Zusatz eines geeigneten Lösungsmittels (Schleppers) und unter Verwendung eines Wasserabscheiders abgetrennt, oder durch Zusatz von Wasser entziehenden Hilfsstoffen entfernt werden.

Die Erfindung wird durch die nachfolgenden Beispiele verdeutlicht, ohne sie darauf zu beschränken:

## Experimenteller Teil:

### Allgemeine Angaben:

Die gaschromatographischen Bestimmungen werden wie folgt durchgeführt: Die Proben wurden nach der Reaktion nach Versetzung mir Dodekan als internem Standard auf einem Hewlett-Packard 6890 Gaschromatograph, ausgestattet mit einer DB-1 Säule (30 m x 0.25 mm x 1 μm) gemessen. Temperaturen: Initial 130°C (5 min), 2°C/min auf 160°C und drei Minuten halten, dann mit 50°C/min auf 300°C und zehn Minuten halten.

Die HPLC Bestimmungen werden wie folgt durchgeführt: HPLC-Gerät von Agilent Technologies Model 1200 Infinity ausgestattet mit einem UV/Vis-Detektor, Säule Chiralpack IA (0.46 cm Durchmesser x 25 cm), Durchfluss 1mL/min; Lösungsmittel bei Start 98 % n-Hexan und 2 % 2-Propanol, nach 2 Minuten 90 % n-Hexan und 10 % 2-Propanol und nach vier Minuten 85 % n-Hexan und 15 % 2-Propanol.

"Inertbedingungen": Die Arbeiten wurden unter Ausschluss von Luft bzw. Sauerstoff durchgeführt. Die Einwaage des Eduktes, Lösungsmittels und des Katalysators erfolgte in einer Handschuhbox, welche mit Reinstargon betrieben wird. Die Arbeiten außerhalb der Handschuhbox erfolgten unter Anwendung von Standard-Schlenk Techniken und

Argon als Inertgas.

**Materialen:**

Alle eingesetzten Phosphanlinganden und Katalysatorvorstufen sind kommerziell erhältlich und wurden über Sigma-Aldrich bzw. Strem bezogen.

**Herstellungsbeispiele:**

Die Reaktionen in den folgenden Beispielen wurden unter Inertbedingungen in einem 100 mL Parr Edelstahlautoklav durchgeführt. Alle Ausgangsverbindungen waren kommerziell erhältlich und wurden ohne weitere Reinigung eingesetzt.

**Beispiele 1 bis 5: Reduktive Aminierung von Acetophenon - Variation des zweizähnigen Phosphan-Liganden**

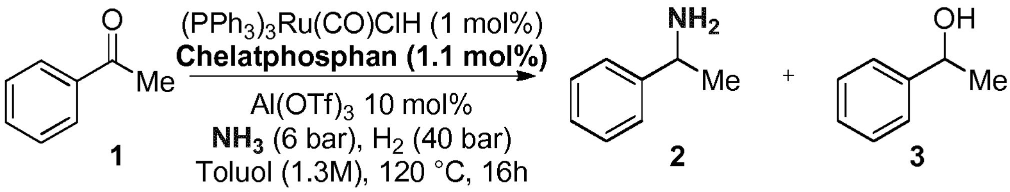


| Beispiele | Chelatphosphan | Umsatz (%) | 2 (%) | 3 (%) | Selelktivität 2:3 |
|---|---|---|---|---|---|
| 1 | I | 95 | 56 | 4 | 14 |
| 2 | II | 92 | 43 | 4 | 11 |
| 3 | III | 95 | 74 | 5 | 15 |
| 4[b] | III | 100 | 99 | 1 | 99 |
| 5 | IV | 95 | 68 | 5 | 14 |

[a] 2.97 mmol **1**, Ru-Katalysator (1 mol%), Ligand (1.1 mol%), $Al(OTf)_3$ (10 mol%), Toluol (20 mL), 120 °C, $p(NH_3)$ 6 bar, $p(H_2)$ 40 bar, 16h. Umsätz und Selektivität bestimmt durch Gaschromatographi mit Dodekan als internem Standard [b] 9 bar $NH_3$.

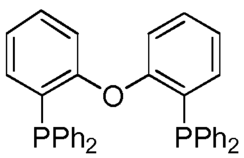

**I**

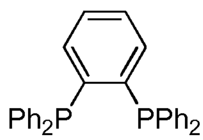

**II**

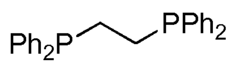

**III**

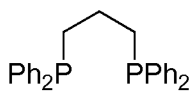

**IV**

Die Beispiele 1 bis 5 zeigen, dass sich unter Verwendung des erfindungsgemäßen Katalysators in Kombination mit einer Säure als Kokatalysator Selektivitäten zum primären Amin von bis zu 99% bei Vollumsatz der Ausgangsverbindung erhalten lassen.

**Beispiele 6 bis 8: Reduktive Aminierung von Acetophenon - Variation der Rutheniumquelle - CO als essentieller Co-Ligand**

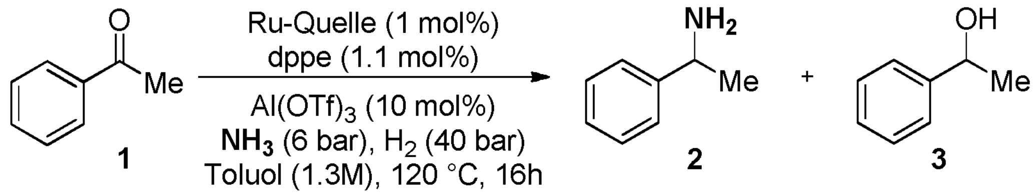


**1** **2** **3**

| Beispiel | Rutheniumquelle | Umsatz (%) | 2 (%) | 3 (%) | Selektivität 2:3 |
|---|---|---|---|---|---|
| 6 | $(PPh_3)_3Ru(CO)H_2$ | 88 | 53 | 2 | 27 |
| 7 | $(PPh_3)_3Ru(CO)HCl$ | 95 | 74 | 5 | 15 |
| 8 | $(PPh_3)_3RuHCl$ Toluol | 50 | 0 | 0 | - |

2.97 mmol **1,** R-Katalysatort (1 mol%), Ligand (1.1 mol%), $Al(OTf)_3$ (10 mol%), Toluol (20 mL), 120 °C, $p(NH_3)$ 6 bar, $p(H_2)$ 40 bar, 16h. Umsatz und Ausbeuten durch Gaschromatographie unter Verwendung von Dodekan als internem Standard bestimmt.

Beispiele 6 und 7 zeigen, dass CO als Ligand notwendig ist, um einen aktiven Katalysator zu erhalten, da bei Verwendung der CO-freien Rutheniumquelle in Beispiel 8 kein Produkt erhalten wird.

**Beispiele 9 bis 16: Reduktive Aminierung von Acetophenon - Variation des sauren Kokatalysators sowie Variation des zweizähnigen Phosphan-Liganden**

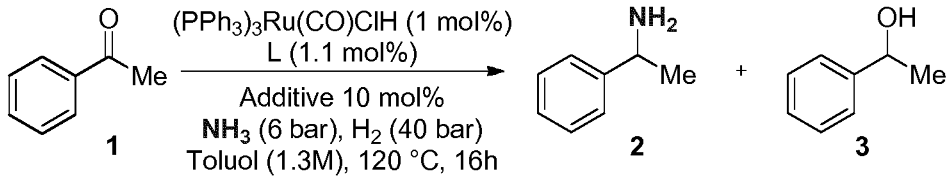


**1** **2** **3**

| Beispiel | Ligand | Säure | Umsatz (%) | 2 (%) | 3 (%) | Selektivität 2:3 |
|---|---|---|---|---|---|---|
| 9 | Xantphos | $Al(OTf)_3$ | 94 | 37 | 4 | 9.3 |
| 10 | dppp | $Sc(OTf)_3$ | 93 | 50 | 2 | 25 |
| 11 | dppp | $Al(OTf)_3$ | 95 | 70 | 5 | 14 |
| 12[b] | dppe | $NH_4TFA$ | 94 | 41 | 2 | 21 |
| 13[c] | dppe | $Al(OTf)_3$ | 100 | 99 | 1 | 99 |
| 14[c],[d] | dppe | $Al(OTf)_3$ | 96 | 92 | 5 | 18 |
| 15 | dppe | $AlCl_3$ | 100 | 76 | 4 | 19 |
| 16 | dppe | - | 70 | 21 | 32 | 0.7 |

[a] 2.97 mmol **1,** Ru-Katalysator (1 mol%), Ligand (1.1 mol%), $Al(OTf)_3$ (10 mol%), Toluol (20 mL), 120 °C, $p(NH_3)$ 6 bar, $p(H_2)$ 40 bar, 16h. Umsatz und Ausbetuen mittels Gaschromatographie unter Verwendung von Dodekan als internem Standard bestimmt [b] 1.0 eq $NH_4TFA$. [c] 9 bar $NH_3$. [d 5 mol%f $Al(OTf)_3$. [e] 0.5 mol% $(PPh_3)_3Ru(CO)HCl$ und 1 mol% $Al(OTf)_3$.

Die Beispiele 8-16 zeigen, dass im erfindungsgemäßen Verfahren der Zusatz von Säuren notwendig ist um hohe Selektivitäten zu erreichen, da im Vergleichsbeispiel 16 ohne Säurezusatz der Alkohol als Hauptprodukt gebildet wird.

**Beispiele 17 bis 29: Reduktive Aminierung von verschiedenen AcetophenonDerivaten**

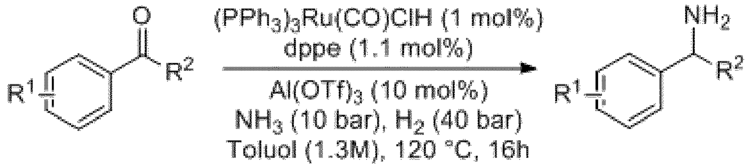


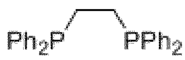

dppe

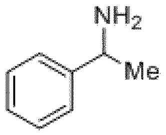

70%
4 bar $NH_3$
Beispiel 17

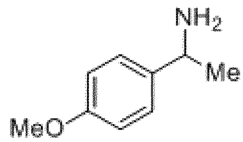

65%
4 bar $NH_3$
Beispiel 16

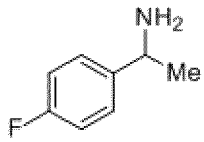

70%
4 bar $NH_3$
Beispiel 19

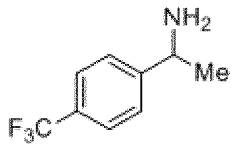

56%
4 bar $NH_3$
Beispiel 20

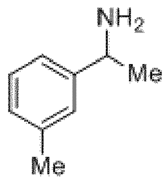

56%
4 bar $NH_3$
Beispiel 21

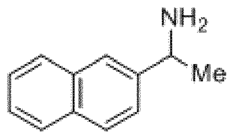

87%
9 bar $NH_3$
Beispiel 22

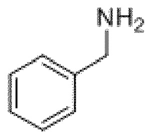

95%
9 bar $NH_3$
Beispiel 23

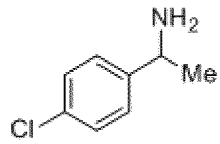

60%
9 bar $NH_3$
Beispiel 24

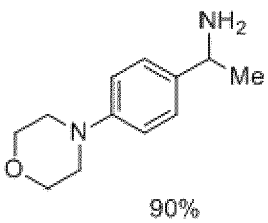

90%
6 bar $NH_3$
Beispiel 25

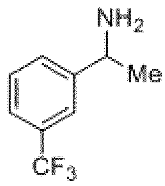

99% (140 °C)
6 bar $NH_3$
Beispiel 26

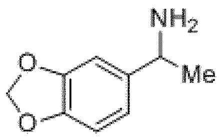

95% (140 °C)
6 bar $NH_3$
Beispiel 27

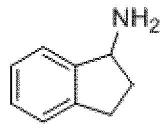

51% (140 °C)
6 bar $NH_3$
Beispiel 28

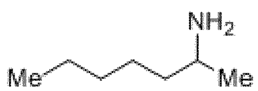

15%
4 bar $NH_3$
Beispiel 29

Isolierte Ausbeuten (durch Säulenchromatographie aus dem Reaktionsgemisch isoliert)

Die Beispiele 17-29 zeigen, dass sich mit dem erfindungsgemäßen Verfahren verschiedene Carbonylverbindungen in guten Ausbeuten zu den entsprechenden primären Aminen umsetzen lassen.

**Beispiel 30 bis 32: Enantioselektive reduktive Aminierung von Acetophenon mit verschiedenen chiralen Katalysatoren**

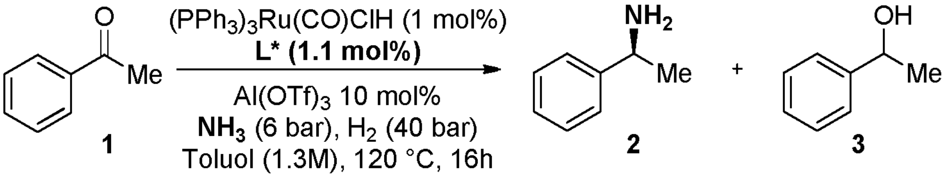

1 2 3

| Beispiel | Ligand | Umsatz (%) | 2 (%) | 3 (%) | Selektivität 2:3 | e.e. (%) |
|---|---|---|---|---|---|---|
| 30 | L5 | 73 | 19 | 0 | 100 | 26 |
| 31 | L6 | 73 | 10 | 10 | 1 | 53 |
| 32 | L7 | 85 | 65 | 13 | 5 | 26 |

[a] 2.97 mmol **1,** R-Katalysator (1 mol%), Ligand (1.1 mol%), $Al(OTf)_3$ (10 mol%), Toluol (20 mL), 120 °C, $p(NH_3)$ 6 bar, $p(H_2)$ 40 bar, 16h. Umsatz und Ausbeuten durch Gaschromatographie mittels Dodekan als internem Standard bestimm. Der Enatiomerenüberschuss (e.e) wurde mittels chiraler HPLC nach Benzylierung der Probe bestimmt.

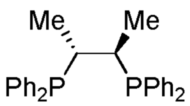
**L5**

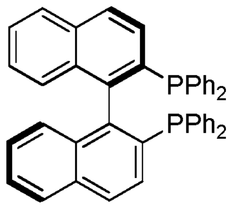
**L6**

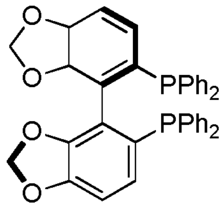
**L7**

Beispiele 30 bis 32 zeigen, dass sich mit dem erfindungsgemäßen Verfahren beim Einsatz chiraler Diphosphanliganden die reduktive Aminierung von unsymmetrischen Ketonen in der Weise durchführen lässt, dass sich bevorzugt ein Stereoisomer des Amines als Produkt bildet.

**Beispiel 33 bis 44: Enantioselektive reduktive Aminierung von Acetophenon mit verschiedenen chiralen Katalysatoren und Cokatalysatoren**

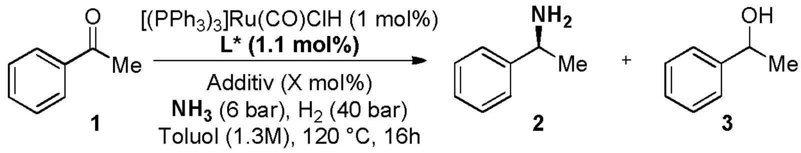

1 2 3

| Beispiel | Additiv (X mol%) | Ligand | Umsatz. (%) | 2 (%) | 3 (%) | e.e. (%) |
|---|---|---|---|---|---|---|
| 33 | $Al(OTf)_3$ (10 mol%) | L5 | 73 | 19 | 0 | 26 |
| 34 | $Al(OTf)_3$ (10 mol%) | L7 | 85 | 65 | 13 | 26 |
| 35 | $NaPF_6$ (3 mol%) | L8 | 72 | 49 | 0 | 5 |
| 36 | $NaPF_6$ (3 mol%) | L6 | 66 | 14 | 23 | 10 |
| 37 | $Al(OTf)_3$ (10 mol%) | L9 | 84 | 55 | 13 | 17 |
| 38 | $Al(OTf)_3$ (10 mol%) | L10 | 80 | 58 | 13 | 8 |
| 39 | $Al(OTf)_3$ (10 mol%) | L11 | 70 | 28 | 16 | 6 |
| 40 | $Al(OTf)_3$ (10 mol%) | L12 | 77 | 41 | 9 | 17 |
| 41 | $Al(OTf)_3$ (10 mol%) | L13 | 82 | 68 | 12 | 32 |
| 43[a] | $NaPF_6$ (4 mol%) | L15 | 76 | 26 | 6 | 23 |

(fortgesetzt)

| Beispiel | Additiv (X mol%) | Ligand | Umsatz. (%) | 2 (%) | 3 (%) | e.e. (%) |
|---|---|---|---|---|---|---|
| 44 | $NaPF_6$ (2 mol%) | L16 | 71 | 67 | 9 | 70 |

Reaktionsbedingungen: 2.97 mmol **1**, Ru-Katalysator (1 mol%), Ligand (1.1 mol%), Additiv (X mol%), Toluol (20 ml), 120 °C, p($NH_3$) 6 bar, p($H_2$) 40 bar, 16h. Umsatz und Ausbeuten durch Gaschromatographie mittels Dodekan als internem Standard bestimmt. Der Enantiomerenüberschuss (e.e.) wurde mittels chiraler HPLC nach Benzylierung der Probe bestimmt. [a] 2 mol% Ru-Komplex und 2.2 mol% Ligand.

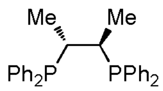
**L5**

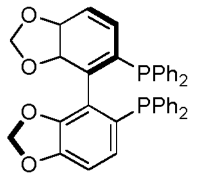
**L7**

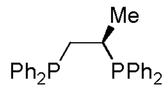
**L8**

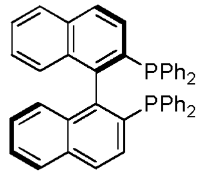
**L6**

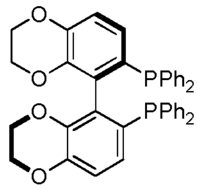
**L9**

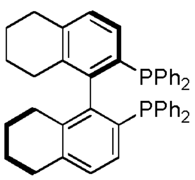
**L10**

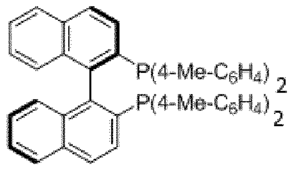
**L11**

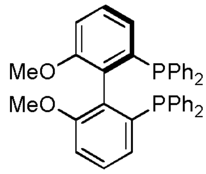
**L12**

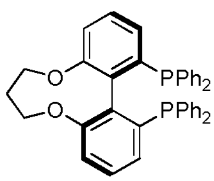
**L13**

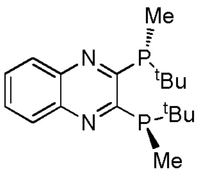
**L15**

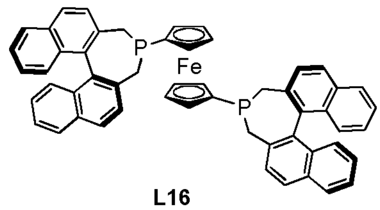
**L16**

**Beispiel 45 bis 48: Enantioselektive reduktive Aminierung von Acetophenon mit verschiedenen Cokatalysatoren**

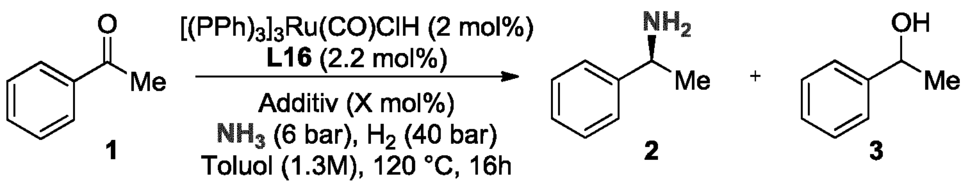

**1** **2** **3**

| Beispiel | Lösungsmittel | Additiv (X mol%) | Umsatz (%) | 2 (%) | 3 (%) | e.e. (%) |
|---|---|---|---|---|---|---|
| 45 | $PhCF_3$ | $NaPF_6$ (4 mol%) | 83 | 78 | 0 | 54 |
| 46 | PhOMe | $NaPF_6$ (4 mol%) | 62 | 51 | 3 | 50 |
| 47 | PhCl | $NaPF_6$ (4 mol%) | 98 | 93 | 1 | 63 |

(fortgesetzt)

| Beispiel | Lösungsmittel | Additiv (X mol%) | Umsatz (%) | 2 (%) | 3 (%) | e.e. (%) |
|---|---|---|---|---|---|---|
| 48 | PhCl | $Al(OTf)_3$ (10 mol%) | 85 | 77 | 0 | 66 |

Reaktionsbedingungen: 2.97 mmol **1**, Ru-Katalysator (2 mol%), Ligand (2.2 mol%), Additiv (X mol%), Toluol (20 ml), 120 °C, $p(NH_3)$ 6 bar, $p(H_2)$ 40 bar, 16h. Umsatz und Ausbeuten durch Gaschromatographie mittels Dodekan als internem Standard bestimmt. Der Enantiomerenüberschuss (e.e.) wurde mittels chiraler HPLC nach Benzylierung der Probe bestimmt

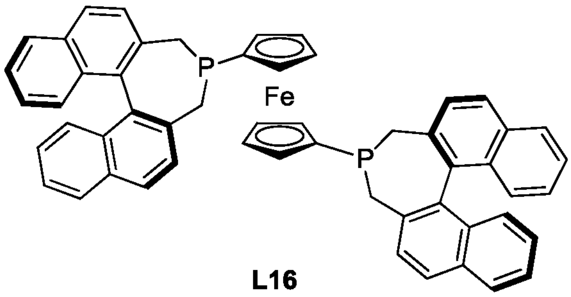

**L16**

Die Beispiele 33 bis 48 zeigen, dass sich mit dem erfindungsgemäßen Verfahren beim Einsatz chiraler Diphosphanliganden die reduktive Aminierung von unsymmetrischen Ketonen in der Weise durchführen lässt, dass sich bevorzugt ein Stereoisomer des Amins als Produkt bildet.

**Patentansprüche**

1. Verfahren zur reduktiven Aminierung einer Carbonyl-Verbindung, umfassend eine oder mehrere reduktiv aminierbare Carbonylgruppen, **dadurch gekennzeichnet, dass** man die Reaktion in Gegenwart eines homogen gelösten Katalysators K sowie einer Säure als Kokatalysator durchführt, wobei der Katalysator K ein Rutheniumkatalysator ist, welcher einen zweizähnigen Phosphanliganden, einen Carbonylliganden, einen Hydridoliganden und gegebenenfalls einen Neutralliganden umfasst.

2. Verfahren nach Anspruch 1, wobei der zweizähnige Phosphanligand folgende allgemeine Formel III aufweist:

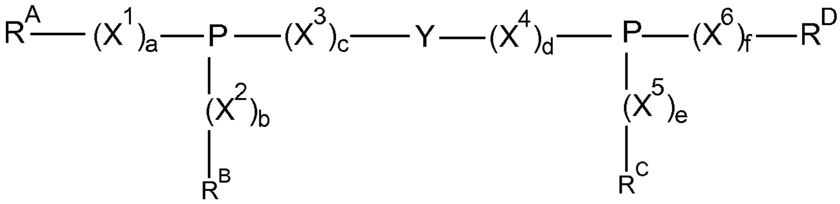

(III)

worin

a, b, c, d, e und f unabhängig voneinander für 0 oder 1 stehen.
$X^1$, $X^2$, $X^3$, $X^4$, $X^5$ und $X^6$ unabhängig voneinander für Alkylen, Arylen, -(C=O)-$NE^7$-Metallocenyl, Metallocenyl-$NE^7$-(C=O)-, oder -(C=O)-$NE^7$- stehen, worin $E^7$ für H oder einen Alkyl-Rest steht; wobei dieser Rest und die Metallocenyl-, Alkylen- oder Arylen-Gruppe jeweils unabhängig voneinander gegebenenfalls ein- oder mehrfach substituiert sind, und wobei die Substituenten, unabhängig voneinander ausgewählt sind unter Alkyl, Cycloalkyl, Heterocyclyl, Aryl, Hetaryl, Alkoxy, Cycloalkoxy, Heterocycloalkoxy, Aryloxy, Hetaryloxy, Hydroxy, Mercapto, Polyalkylenoxid, Polyalkylenimin, COOH, Carboxylat, $SO_3H$, Sulfonat, Halogen, Nitro, Formyl, Acyl, Cyano, $NE^1E^2$, und $NE^1E^2E^{3+}X^-$, worin $E^1$, $E^2$ und $E^3$ jeweils gleiche oder verschiedene Reste, ausgewählt unter Wasserstoff, Alkyl, Cycloalkyl, oder Aryl bedeuten und $X^-$ für ein Anionäquivalent steht,
Y für eine kohlenstoffatomhaltige Brückengruppe steht, und

$R^A$, $R^B$, $R^C$ und $R^D$ unabhängig voneinander für Reste ausgewählt unter Alkyl, Cycloalkyl, Heterocycloalkyl, Aryl oder Hetaryl stehen, wobei diese Reste unabhängig voneinander gegebenenfalls ein- oder mehrfach substituiert sind, wobei die Substituenten unabhängig voneinander ausgewählt sind unter Alkyl, Cycloalkyl, Heterocyclyl, Aryl, Hetaryl, Alkoxy, Cycloalkoxy, Heterocycloalkoxy, Aryloxy, Hetaryloxy, Hydroxy, Mercapto, Polyalkylenoxid, Polyalkylenimin, COOH, Carboxylat, $SO_3H$, Sulfonat, Halogen, Nitro, Formyl, Acyl, Cyano, $NE^1E^2$, und $NE^1E^2E^{3+}X^-$, worin $E^1$, $E^2$ und $E^3$ jeweils gleiche oder verschiedene Reste, ausgewählt unter Wasserstoff, Alkyl, Cycloalkyl, oder Aryl bedeuten und $X^-$ für ein Anionäquivalent steht;

oder

$R^A$ und $R^B$ und/oder $R^C$ und $R^D$ zusammen mit dem Phosphoratom und, falls vorhanden, den Gruppen $X^1$, $X^2$, $X^5$ und $X^6$, an die sie gebunden sind, für einen 4- bis 8-gliedrigen Heterocyclus oder Heterobicyclus stehen, der gegebenenfalls zusätzlich mit Cycloalkyl, Heterocyclyl, Aryl oder Hetaryl anelliert ist, wobei der Heterocyclus oder Heterobicyclus und, falls vorhanden, die anellierten Gruppen unabhängig voneinander gegebenenfalls substituiert sind, wobei die Substituenten ausgewählt sind unter Alkyl, Cycloalkyl, Heterocyclyl, Aryl, Hetaryl, Hydroxy, Mercapto, Polyalkylenoxid, Polyalkylenimin, Alkoxy, Halogen, COOH, Carboxylat, $SO_3H$, Sulfonat, $NE^4E^5$, $NE^4E^5E^{6+}X^-$, Nitro, Alkoxycarbonyl, Formyl, Acyl und Cyano, worin $E^4$, $E^5$ und $E^6$ jeweils gleiche oder verschiedene Reste, ausgewählt unter Wasserstoff, Alkyl, Cycloalkyl und Aryl bedeuten und $X^-$ für ein Anionäquivalent steht;

oder

einer der Reste $R^A$ und $R^B$ und/oder einer der Reste $R^C$ und $R^D$ zusammen mit dem Phosphoratom und, falls vorhanden, den Gruppen $X^1$, $X^2$, $X^5$ und $X^6$, an die sie gebunden sind, und zusammen mit einem Brückengruppenatom der Brückengruppe Y, für einen 5- bis 8-gliedrigen Heterocyclus stehen, der gegebenenfalls zusätzlich mit Cycloalkyl, Heterocycloalkyl, Aryl oder Hetaryl anelliert ist, wobei der Heterocyclus und, falls vorhanden, die anellierten Gruppen unabhängig voneinander gegebenenfalls zusätzlich substituiert sind, wobei die Substituenten ausgewählt sind unter Alkyl, Cycloalkyl, Heterocyclyl, Aryl, Hetaryl, Hydroxy, Mercapto, Polyalkylenoxid, Polyalkylenimin, Alkoxy, Halogen, COOH, Carboxylat, $SO_3H$, Sulfonat, $NE^4E^5$, $NE^4E^5E^{6+}X^-$, Nitro, Alkoxycarbonyl, Formyl, Acyl und Cyano, worin $E^4$, $E^5$ und $E^6$ jeweils gleiche oder verschiedene Reste, ausgewählt unter Wasserstoff, Alkyl, Cycloalkyl und Aryl bedeuten und $X^-$ für ein Anionäquivalent steht.

**3.** Verfahren nach Anspruch 2, wobei der zweizähnige Phosphanligand ausgewählt ist unter Verbindungen der allgemeinen Formel III, worin

$X^1$, $X^2$, $X^3$, $X^4$, $X^5$ und $X^6$ unabhängig voneinander für gegebenenfalls substituiertes $C_1$-$C_6$-Alkylen, -(C=O)-$NE^7$-Ferrocenyl, Ferrocenyl-$NE^7$-(C=O)- oder -(C=O)-$NE^7$- stehen, worin $E^7$ die oben angegebenen Bedeutungen besitzt;

Y, a, b, c, d, e und f die oben angegebenen Bedeutungen besitzen;

$R^A$, $R^B$, $R^C$ und $R^D$ unabhängig voneinander für Reste, ausgewählt unter $C_1$-$C_{30}$-Alkyl, $C_4$-$C_{10}$-Cycloalkyl, $C_3$-$C_{16}$-Heterocyclyl, $C_5$-$C_{14}$-Aryl oder $C_5$-$C_{30}$-Hetaryl stehen, wobei diese Reste unabhängig voneinander gegebenenfalls ein- oder mehrfach substituiert sind, wobei die Substituenten unabhängig voneinander ausgewählt sind unter $C_1$-$C_{10}$-Alkyl, $C_4$-$C_{10}$-Cycloalkyl, $C_3$-$C_{10}$-Heterocycloalkyl, $C_5$-$C_{14}$-Aryl, $C_5$-$C_{30}$-Hetaryl, $C_1$-$C_{10}$-Alkoxy, $C_3$-$C_{10}$-Cycloalkoxy, $C_3$-$C_{10}$-Heterocycloalkoxy, $C_5$-$C_{14}$-Aryloxy, $C_5$-$C_{30}$-Hetaryloxy, Hydroxy, Mercapto, Poly-$C_2$-$C_6$-alkylenoxid, Poly-$C_2$-$C_6$-alkylenimin, COOH, Carboxylat, $SO_3H$, Sulfonat, Halogen, Nitro, Formyl, $C_1$-$C_{10}$-Acyl, Cyano, $NE^1E^2$, und $NE^1E^2E^{3+}X^-$, worin $E^1$, $E^2$ und $E^3$ jeweils gleiche oder verschiedene Reste, ausgewählt unter Wasserstoff, $C_1$-$C_{10}$-Alkyl, $C_3$-$C_{10}$-Cycloalkyl, oder $C_5$-$C_{10}$-Aryl bedeuten und $X^-$ für ein Anionäquivalent steht;

oder

$R^A$ und $R^B$ und/oder $R^C$ und $R^D$ zusammen mit dem Phosphoratom und, falls vorhanden, den Gruppen $X^1$, $X^2$, $X^5$ und $X^6$, an die sie gebunden sind, für einen 4- bis 8-gliedrigen Heterocyclus oder Heterobicyclus stehen, der gegebenenfalls zusätzlich mit $C_4$-$C_{10}$-Cycloalkyl, $C_3$-$C_{10}$-Heterocycloalkyl, $C_5$-$C_{14}$-Aryl oder $C_5$-$C_{30}$-Hetaryl anelliert ist, wobei der Heterocyclus oder Heterobicyclus und, falls vorhanden, die anellierten Gruppen unabhängig voneinander gegebenenfalls substituiert sind, wobei die Substituenten ausgewählt sind unter $C_1$-$C_{10}$-Alkyl, $C_3$-$C_{10}$-Cycloalkyl, $C_3$-$C_{10}$-Heterocycloalkyl, $C_5$-$C_{14}$-Aryl, $C_5$-$C_{30}$-Hetaryl, Hydroxy, Mercapto, Poly-$C_2$-$C_6$-alkylenoxid, Poly-$C_2$-$C_6$-alkylenimin, $C_1$-$C_{10}$-Alkoxy, Halogen, COOH, Carboxylat, $SO_3H$, Sulfonat, $NE^4E^5$, $NE^4E^5E^{6+}X^-$, Nitro, $C_1$-$C_{10}$-Alkoxycarbonyl, Formyl, $C_1$-$C_{10}$-Acyl und Cyano, worin $E^4$, $E^5$ und $E^6$ jeweils gleiche oder verschiedene Reste, ausgewählt unter Wasserstoff, $C_1$-$C_{10}$-Alkyl, $C_3$-$C_{10}$-Cycloalkyl und $C_5$-$C_{14}$-Aryl bedeuten und $X^-$ für ein Anionäquivalent steht;

oder

einer der Reste $R^A$ und $R^B$ und/oder einer der Reste $R^C$ und $R^D$ zusammen mit dem Phosphoratom und, falls vorhanden, den Gruppen $X^1$, $X^2$, $X^5$ und $X^6$, an die sie gebunden sind, und zusammen mit einem Brückengruppenatom der Brückengruppe Y, für einen 5- bis 8-gliedrigen Heterocyclus stehen, der gegebenenfalls zusätzlich mit $C_3$-$C_{10}$-Cycloalkyl, $C_3$-$C_{10}$-Heterocycloalkyl, $C_5$-$C_{14}$-Aryl oder $C_5$-$C_{30}$-Hetaryl anelliert ist, wobei der Heterocyclus

und, falls vorhanden, die anellierten Gruppen unabhängig voneinander gegebenenfalls zusätzlich substituiert sind, wobei die Substituenten ausgewählt sind unter $C_1$-$C_{10}$-Alkyl, $C_3$-$C_{10}$-Cycloalkyl, $C_3$-$C_{10}$-Heterocycloalkyl, $C_5$-$C_{14}$-Aryl, $C_5$-$C_{30}$-Hetaryl, Hydroxy, Mercapto, Poly-$C_2$-$C_6$-alkylenoxid, Poly-$C_2$-$C_6$-alkylenimin, $C_1$-$C_{10}$-Alkoxy, Halogen, COOH, Carboxylat, $SO_3H$, Sulfonat, $NE^4E^5$, $NE^4E^5E^{6+}X^-$, Nitro, $C_1$-$C_{10}$-Alkoxycarbonyl, Formyl, $C_1$-$C_{10}$-Acyl und Cyano, worin $E^4$, $E^5$ und $E^6$ jeweils gleiche oder verschiedene Reste, ausgewählt unter Wasserstoff, $C_1$-$C_{10}$-Alkyl, $C_3$-$C_{10}$-Cycloalkyl und $C_5$-$C_{14}$-Aryl bedeuten und $X^-$ für ein Anionäquivalent steht.

**4.** Verfahren nach einem der vorhergehenden Ansprüche, wobei in dem zweizähnigen Phosphanliganden der allgemeinen Formel III die Gruppe Y für eine Brückengruppe steht, ausgewählt unter

- geradkettiges oder verzweigtes Alkylen,
- geradkettiges oder verzweigtes Alkenylen,
- Cycloalkylen gegebenenfalls enthaltend mindestens ein Ring-Heteroatom ausgewählt aus N, O, S,
- Cycloalkenylen, gegebenenfalls enthaltend mindestens ein Ring-Heteroatom ausgewählt aus N, O, S,
- Bi-, Tri- und Tetracyclischen Brückengruppen;
- Arylen,
- Heteroarylen enthaltend mindestens ein Ring-Heteroatom ausgewählt aus N, O und S,
- Heteroalkylen enthaltend mindestens ein Ketten-Heteroatom ausgewählt aus N, O und S,
- Heteroalkenylen enthaltend mindestens ein Ketten-Heteroatom ausgewählt aus N, O und S,
- Mehrkernige Brückengruppen, umfassend wenigsten zwei miteinander verbundene aromatische, heteroaromatische, carbozyklische oder heterozyklische, 4- bis 8- gliedrige Ringe,; und
- Metallocenbrückengruppen enthaltend wenigstens eine Metallocengruppe der allgemeinen Formel

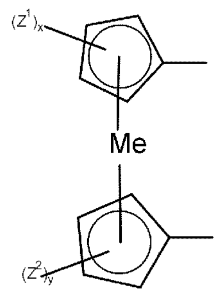

worin Me für ein Metallatom steht, wobei die P-Atome des Phosphanliganden am gleichen oder zwei verschiedenen Cp-Ringen des Metallocens direkt oder über eine der Gruppen $X^3$ oder $X^4$ gebunden sind, x und y unabhängig voneinander für 0, 1, 2, 3 oder 4 stehen und $Z^1$ und $Z^2$ unabhängig voneinander für H, gegebenenfalls ein- oder mehrfach substituiertes Alkyl oder gegebenenfalls ein- oder mehrfach substituiertes Aryl stehen.

**5.** Verfahren nach einem der vorhergehenden Ansprüche, wobei in dem zweizähnigen Phosphanliganden der allgemeinen Formel III die Gruppe Y für eine mehrkernige Brückengruppe steht, ausgewählt unter Gruppen der Formeln III.a bis III.k

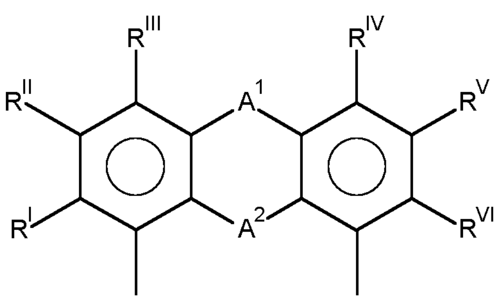

(III.a)

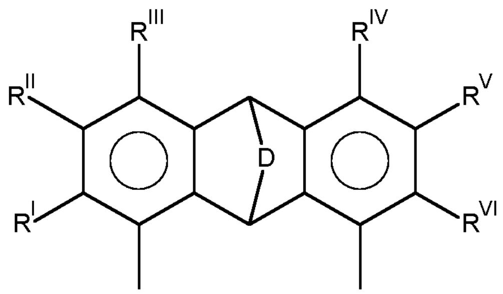

(III.b)

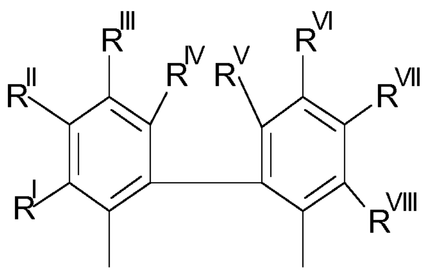

(III.c)

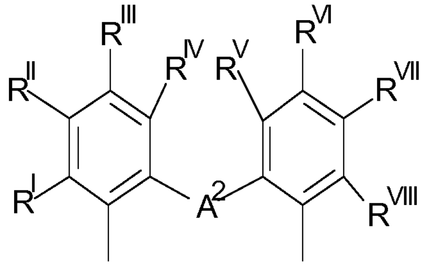

(III.d)

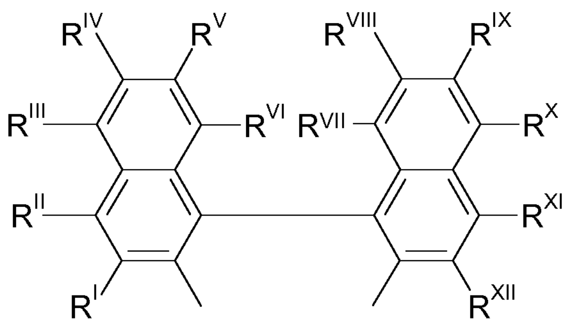

(III.e)

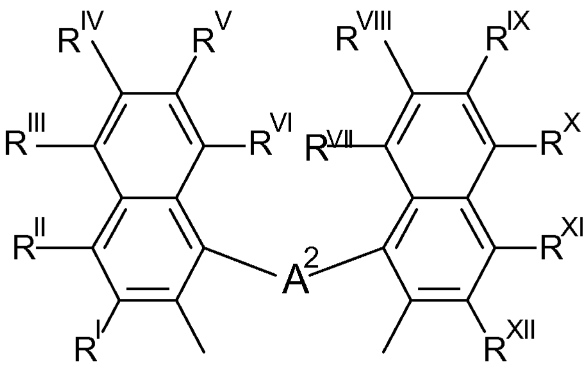

(III.f)

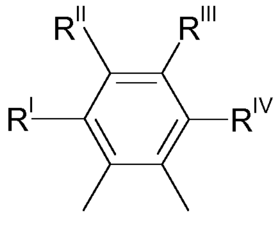

(III.g)

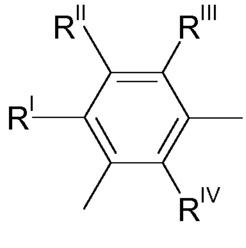

(III.h)

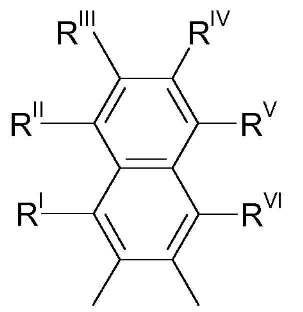

(III.i)

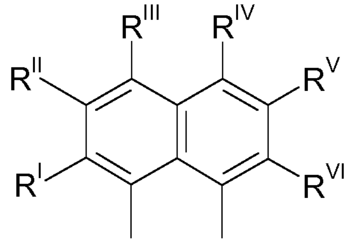

(III.k)

worin

$R^I$, $R^{II}$, $R^{III}$, $R^{IV}$, $R^V$, $R^{VI}$, $R^{VII}$, $R^{VIII}$, $R^{IX}$, $R^X$, $R^{XI}$ und $R^{XII}$ unabhängig voneinander für Wasserstoff, Alkyl, Cycloalkyl, Heterocycloalkyl, Aryl, Hetaryl, Hydroxy, Mercapto, Polyalkylenoxid, Polyalkylenimin, Alkoxy, Halogen, $SO_3H$, Sulfonat, $NE^9E^{10}$, Alkylen-$NE^9E^{10}$, Trifluormethyl, Nitro, Alkoxycarbonyl, Carboxyl, Formyl, Acyl oder Cyano stehen, worin $E^9$ und $E^{10}$ jeweils gleiche oder verschiedene Reste, ausgewählt unter Wasserstoff, Alkyl, Cycloalkyl und Aryl bedeuten,
oder
jeweils zwei benachbarte Reste $R^I$, $R^{II}$, $R^{III}$, $R^{IV}$, $R^V$, $R^{VI}$, $R^{VII}$, $R^{VIII}$, $R^{IX}$, $R^X$, $R^{XI}$ und $R^{XII}$ zusammen mit den Kohlenstoffatomen an die sie gebunden sind einen 4- bis 7-gliedrigen carbocyclischen oder heterozyclischen, aromatischen oder nichtaromatischen Ring bilden, der seinerseits gegebenenfalls mit einem ein- oder mehrkerigen carbocyclischen oder heterozyclischen, aromatischen oder nichtaromatischen Ring anelliert sein kann, wobei dieses Ringsystem gegebenenfalls substituiert ist, wobei die Substituenten ausgewählt sind unter Alkyl, Cycloalkyl, Heterocyclyl, Aryl, Hetaryl, Hydroxy, Mercapto, Polyalkylenoxid, Polyalkylenimin, Alkoxy, Halogen, COOH, Carboxylat, $SO_3H$, Sulfonat, $NE^4E^5$, $NE^4E^5E^{6+}X^-$, Nitro, Alkoxycarbonyl, Formyl, Acyl und Cyano, worin $E^4$, $E^5$ und $E^6$ jeweils gleiche oder verschiedene Reste, ausgewählt unter Wasserstoff, Alkyl, Cycloalkyl und Aryl bedeuten und $X^-$ für ein Anionäquivalent steht;
wobei die zwei benachbarten Reste aus $R^I$, $R^{II}$, $R^{III}$, $R^{IV}$, $R^V$, $R^{VI}$, $R^{VII}$, $R^{VIII}$, $R^{IX}$, $R^X$, $R^{XI}$ oder $R^{XII}$ an Kohlenstoffatome des gleichen aromatischen Rings oder an Kohlenstoffatome zweier voneinander verschiedener aromatischer Ringe gebunden sein können;
$A^1$ für eine Bindung, NH, $NR^{11}$, O, S, $CR^{11}$ oder $CR^{11}R^{12}$ steht, wobei $R^{11}$ und $R^{12}$ unabhängig voneinander ausgewählt sind unter H, Alkyl und Aryl;
$A^2$ für eine NH, N, O oder S steht,
D für eine zweibindige Brückengruppe der allgemeinen Formel

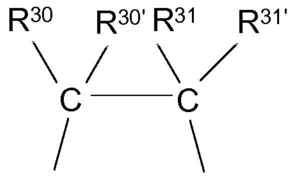

steht, in der
$R^{30}$, $R^{30'}$, $R^{31}$ und $R^{31'}$ unabhängig voneinander für Wasserstoff, Alkyl, Cycloalkyl, Aryl, Halogen, Trifluormethyl, Carboxyl, Carboxylat oder Cyano stehen,

wobei $R^{30'}$ auch gemeinsam mit $R^{31'}$ für den Bindungsanteil einer Doppelbindung zwischen den beiden Kohlenstoffatomen, an die $R^{30'}$ und $R^{31'}$ gebunden sind, stehen kann, und/oder $R^{30}$ und $R^{31}$ gemeinsam mit den Kohlenstoffatomen, an die sie gebunden sind, auch für einen 4- bis 8-gliedrigen Carbo- oder Heterocyclus stehen können, der gegebenenfalls zusätzlich ein-, zwei- oder dreifach mit Cycloalkyl, Heterocycloalkyl, Aryl oder Hetaryl anelliert ist, wobei der Carbo- oder Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, $COOR^e$, $COO^-M^+$, $SO_3R^e$, $SO_3^-M^+$, $NE^{11}E^{12}$, Alkylen-$NE^{11}E^{12}$, $NE^{11}E^{12}E^{13+}X^-$, Alkylen-$NE^{11}E^{12}E^{13+}X^-$, $OR^e$, $SR^e$, $(CHR^fCH_2O)_yR^e$, $(CH_2N(E^{11}))_yR^e$, $(CH_2CH_2N(E^{11}))_yR^e$, Halogen, Trifluormethyl, Nitro, Formyl, Acyl oder Cyano, stehen,

worin

$R^e$, $E^{11}$, $E^{12}$ und $E^{13}$ jeweils gleiche oder verschiedene Reste, ausgewählt unter Wasserstoff, Alkyl, Cycloalkyl oder Aryl bedeuten,

$R^f$ für Wasserstoff, Methyl oder Ethyl steht,

$M^+$ für ein Kationäquivalent steht,

$X^-$ für ein Anionäquivalent steht, und

y für eine ganze Zahl von 1 bis 120 steht.

**6.** Verfahren nach einem der vorhergehenden Ansprüche, wobei in dem zweizähnigen Phosphanliganden der allgemeinen Formel III die Gruppe Y für eine Brückengruppe steht, ausgewählt unter polycyclischen Gruppen III.m bis III.o

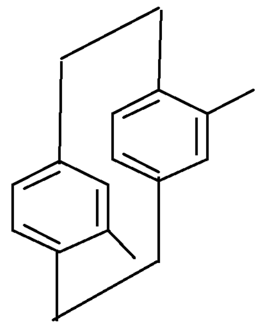

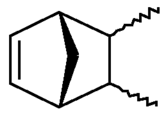

(III.n)

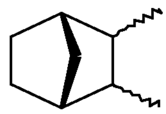

(III.o)

(III.m)

**7.** Verfahren nach einem der vorhergehenden Ansprüche, wobei in dem zweizähnigen Phosphanliganden der allgemeinen Formel III die Gruppe Y für eine Brückengruppe steht, ausgewählt unter den heterocyclischen Gruppen V.a bis V.h

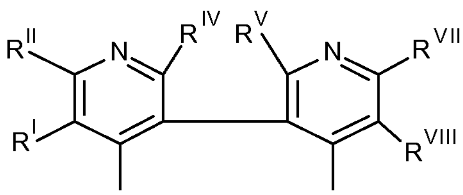

(V.a)

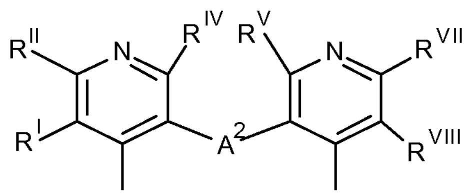

(V.b)

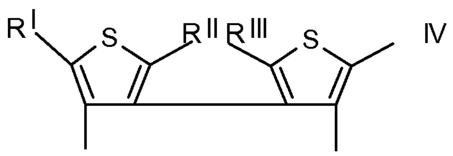

(V.c)

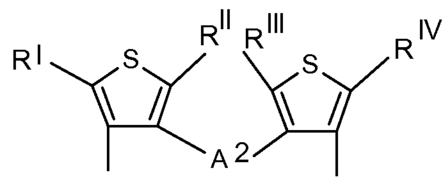

(V.d)

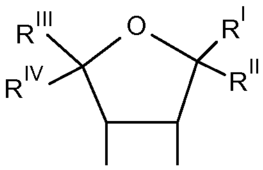

(V.e)

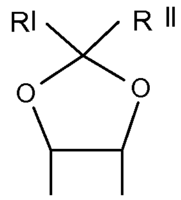

(V.f)

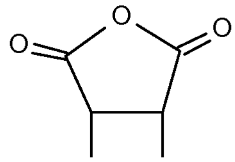

(V.g)

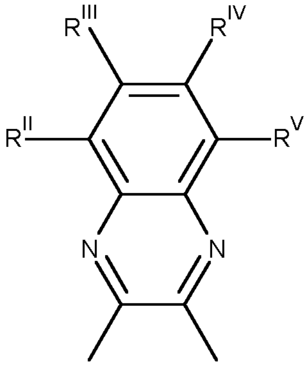

(V.h)

worin

die Reste $R^I$ bis $R^{VIII}$ unabhängig voneinander ausgewählt sind unter Wasserstoff, Alkyl, Cycloalkyl, Heterocycloalkyl, Aryl, Hetaryl, Hydroxy, Mercapto, Polyalkylenoxid, Polyalkylenimin, Alkoxy, Halogen, $SO_3H$, Sulfonat, $NE^9E^{10}$, Alkylen-$NE^9E^{10}$, Trifluormethyl, Nitro, Alkoxycarbonyl, Carboxyl, Formyl, Acyl oder Cyano, worin $E^9$ und $E^{10}$ jeweils gleiche oder verschiedene Reste, ausgewählt unter Wasserstoff, Alkyl, Cycloalkyl und Aryl bedeuten; und
$A^2$ für eine NH-Gruppe, N, O oder S steht.

**8.** Verfahren nach einem der vorhergehenden Ansprüche, wobei in dem zweizähnigen Phosphanliganden der allgemeinen Formel III die beiden Phosphanendgruppen unabhängig voneinander ausgewählt sind unter Gruppen der Formeln VI.b, VI.d, VI.e, VI.f, VI.g, VI.h, VI.i und VI.k

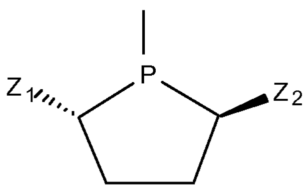

(VI.b)

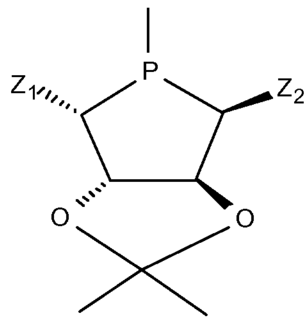

(VI.d)

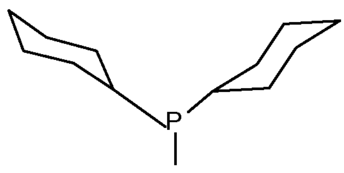

(V I.e)

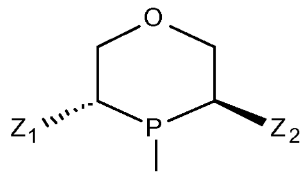

(V I.f)

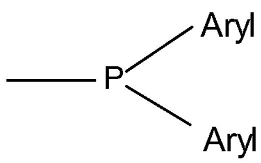

(VI.g)

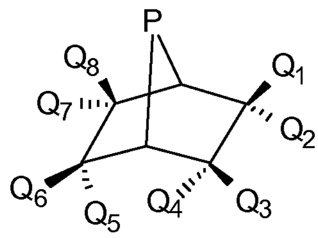

(VI.h)

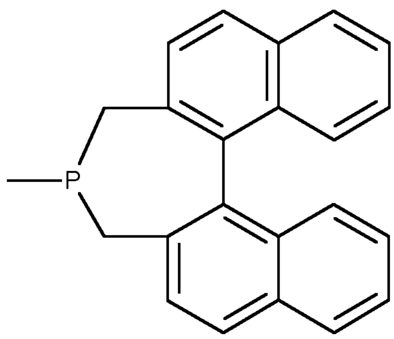

(VI.k)

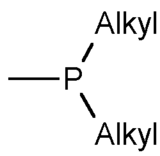

(VI.i)

worin

Aryl für gegebenenfalls ein oder mehrfach substituiertes Phenyl steht, wobei die Substituenten ausgewählt sind unter Alkyl oder Alkoxy
$Z_1$ und $Z_2$ unabhängig voneinander ausgewählt sind unter Alkyl, Alkoxy, Alkoxyalkyl, Aryl oder Hetaryl stehen und $Q_1$ bis $Q_8$ unabhängig voneinander für H oder Alky stehen.

**9.** Verfahren nach einem der vorhergehenden Ansprüche, wobei der Säure-Kokatalysator eine Lewis-Säure ist.

**10.** Verfahren nach einem der vorhergehenden Ansprüche, wobei der Säure-Kokatalysator ausgewählt ist unter: $[NH_4]TFA$, $[NH_4]Cl$, $[NH_4]_2[SO_4]$, $[NH_4]Br$, $Al(OTf)_3$, $Sc(OTf)_3$, $AlCl_3$, $AlBr_3$, $BR_3$, $BCl_3$, $BBr_3$, $BF_3$, $Al(NO_3)_3$.

**11.** Verfahren nach einem der vorhergehenden Ansprüche, wobei der Katalysator K ein Rutheniumkatalysator ist, der die folgende allgemeine Formel IV aufweist,

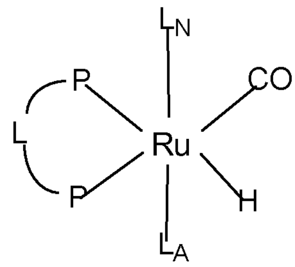

(IV)

worin

P-L-P der zweizähnige Phosphanligand die oben angegebenen Formel (III) ist;
$L_N$ für einen Neutralliganden, ausgewählt unter Aminen, Phosphinen, Phosphiten, Alkoholen, N-Heterocyclische Carbenen, Iminen, Carbonylverbindungen, Olefinen, Alkinen oder Nitrilen steht, und
$L_A$ fehlt oder für einen anionischen Liganden, ausgewählt unter Chlorid, Fluorid, Bromid, Iodid, Hydrid, Cyanid, Alkoxy, Amido oder Hydroxy steht,

**12.** Verfahren nach einem der vorhergehenden Ansprüche, wobei man die Aminierung enantioselektiv durchführt, indem man einen Katalysator K einsetzt, der wenigstens einen chiralen zweizähnigen Phosphanliganden gemäß obiger Formel III trägt.

**13.** Verfahren nach einem der vorhergehenden Ansprüche, wobei die zu aminierende Carbonylverbindung eine Verbindung der folgenden allgemeinen Formel II ist,

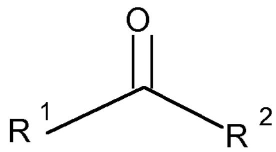

worin $R^1$ und $R^2$ gleich oder verschieden sind und unabhängig voneinander ausgewählt sind unter
H,
gegebenenfalls ein- oder mehrfach substituiertes Alkyl, wobei die Kohlenstoffkette gegebenenfalls durch eine oder mehrere Carbonylgruppen unterbrochen ist,
gegebenenfalls ein- oder mehrfach substituiertes Cycloalkyl, wobei der carbocyclische Ring gegebenenfalls durch eine oder mehrere Carbonylgruppen unterbrochen ist,
gegebenenfalls ein- oder mehrfach substituiertes Heterocyclyl, wobei der heterocyclische Ring gegebenenfalls durch eine oder mehrere Carbonylgruppen unterbrochen ist,
gegebenenfalls ein- oder mehrfach substituiertes Aryl und
gegebenenfalls ein- oder mehrfach substituiertes Hetaryl;
wobei die gegebenenfalls vorhandenen Substituenten ausgewählt sind unter Halogen (insbesondere F, Cl, Br) -OH, -CN, $-NH_2$, $C_1$-$C_{30}$-Alkyl, $C_3$-$C_{30}$-Cycloalkyl, $C_3$-$C_{30}$-Heterocyclyl, $C_5$-$C_{14}$-Aryl, $C_5$-$C_{14}$-Heteroaryl, $-OR_7$, $-NHR_7$ oder $-N(R_7)_2$, $-C(O)R_7$,$-C(O)OR_7$, $-C(O)NHR_7$ oder $-C(O)N(R_7)_2$,
wobei $R_7$ ausgewählt ist aus $C_1$-$C_{30}$-Alkyl und $C_5$-$C_{30}$-Aryl

**14.** Verfahren nach einem der vorhergehenden Ansprüche, wobei der Katalysator K aus einer Katalysatorvorstufe, ausgewählt unter den folgenden Verbindungen gebildet wird:
$[Ru(PPh_3)_3(H)(Cl)(CO)]$, $[Ru(PPh_3)_3(H)_2(CO)]$, $[Ru(PPh_3)_3(Cl)_2]$, $[Ru(PPh_3)_3(Cl)_2(CO)]$, [Ru(p-cymene)$(Cl)_2$], [Ru(benzol)$Cl_2]_n$, $[Ru(CO)_2Cp]_n$ $[Ru(CO)_3(Cl)_2]$ [Ru(COD)(allyl)], [Ru(COD)(2-methylallyl)$_2$], $[RuCl_3*H_2O]$, [Ru(acteylacetonat)$_3$], $[Ru(DMSO)_4(Cl)_2]$, $Ru_3CO_{12}$,[Ru(cyclopentadienyl)$(PPh_3)_2(Cl)$], Ru(cyclopentadienyl)$(CO)_2(Cl)$], [Ru(cyclopentadienyl)$(CO)_2H$], [Ru(cyclopentadienyl)$(CO)_2]_2$, [Ru(pentamethylcyclopentadienyl)$(CO)_2(Cl)$], [Ru(pentamethylcylcopentadienyl)$(CO)_2H$], [Ru(pentamethylcyclopentadienyl)$(CO)_2]_2$, [Ru(indenyl)$(CO)_2(Cl)$], [Ru(indenyl)$(CO)_2(H)$], [Ru(indenyl)$(CO)_2]_2$, Ruthenocen, [Ru(binap)$Cl_2$], [Ru(bipyridin)$_2Cl_2*2H_2O$], [Ru(COD)$Cl_2]_2$, [Ru(pentamethylcyclopentadienyl)(COD)(Cl)], $[Ru_3(CO)_{12}]$, [Ru(tetraphenylhydroxy-cyclopentadienyl)$(CO)_2(H)$],

$[Ru(PPh_3)_4(H)_2]$.

**15.** Verfahren nach einem der vorhergehenden Ansprüche, wobei man den Katalysator K in einer Menge von 1 bis 5000 ppm Gewichtsteilen, bezogen auf das Gesamtgewicht des eingesetzten flüssigen Reaktionsmediums einsetzt und/oder wobei man die Reaktion unter folgenden Reaktionsbedingungen durchführt:

a) Temperatur im Bereich von 20 bis 250 °C und/oder
b) Gesamtdruck im Bereich von 0,1 bis 30 MPa und/oder
c) Reaktionsdauer, 0,1 bis 100 h und/oder
d) Ammoniak: 1,5 bis 250-facher molarer Überschuss, bezogen auf Mol eingesetztem Edukt und/oder
e) Wasserstoff 1,5 bis 250-facher molarer Überschuss bezogen auf Mol eingesetztem Edukt.

**Claims**

**1.** A method for the reductive amination of a carbonyl compound, comprising one or more carbonyl groups amenable to reductive amination, wherein the reaction is carried out in the presence of a homogeneously dissolved catalyst K and an acid as co-catalyst, wherein the catalyst K is a ruthenium catalyst which comprises a bidentate phosphane ligand, a carbonyl ligand, a hydride ligand and optionally a neutral ligand.

**2.** The method according to claim 1, wherein the bidentate phosphane ligand has the following general formula III:

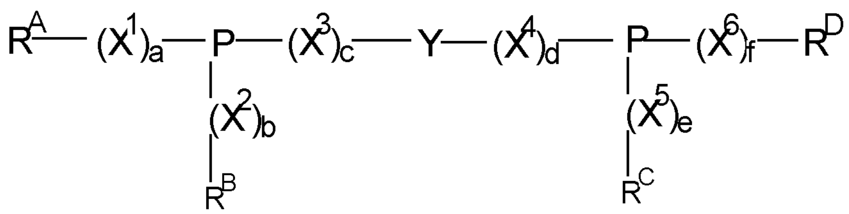

(III)

where

a, b, c, d, e and f are mutually independently 0 or 1,
$X^1$, $X^2$, $X^3$, $X^4$, $X^5$ and $X^6$ are mutually independently alkylene, arylene, -(C=O)-$NE^7$-metallocenyl, metallocenyl-$NE^7$-(C=O)- or -(C=O)-$NE^7$-, where $E^7$ is H or an alkyl residue; wherein said residues and the metallocenyl, alkylene or arylene groups are each independently optionally mono- or polysubstituted, and wherein the substituents are mutually independently selected from alkyl, cycloalkyl, heterocyclyl, aryl, hetaryl, alkoxy, cycloalkoxy, heterocycloalkoxy, aryloxy, hetaryloxy, hydroxyl, mercapto, polyalkylene oxide, polyalkylenimine, COOH, carboxylate, $SO_3H$, sulfonate, halogen, nitro, formyl, acyl, cyano, $NE^1E^2$, and $NE^1E^2E^{3+}X^-$, where $E^1$, $E^2$ and $E^3$ are each identical or different residues selected from hydrogen, alkyl, cycloalkyl, or aryl and $X^-$ is an anion equivalent,
Y is a carbon atom-containing bridging group, and
$R^A$, $R^B$, $R^C$ and $R^D$ are residues mutually independently selected from alkyl, cycloalkyl, heterocycloalkyl, aryl or hetaryl, wherein said residues are mutually independently optionally mono- or polysubstituted, wherein the substituents are mutually independently selected from alkyl, cycloalkyl, heterocyclyl, aryl, hetaryl, alkoxy, cycloalkoxy, heterocycloalkoxy, aryloxy, hetaryloxy, hydroxyl, mercapto, polyalkylene oxide, polyalkylenimine, COOH, carboxylate, $SO_3H$, sulfonate, halogen, nitro, formyl, acyl, cyano, $NE^1E^2$, and $NE^1E^2E^{3+}X^-$, where $E^1$, $E^2$ and $E^3$ are each identical or different residues selected from hydrogen, alkyl, cycloalkyl, or aryl and $X^-$ is an anion equivalent;
or
$R^A$ and $R^B$ and/or $R^C$ and $R^D$ together with the phosphorus atom and, if present, the groups $X^1$, $X^2$, $X^5$ and $X^6$ to which they are bound, are a 4- to 8-membered heterocycle or heterobicycle, which is optionally additionally fused to cycloalkyl, heterocyclyl, aryl or hetaryl, wherein the heterocycle or heterobicycle and, if present, the fused groups, are mutually independently optionally substituted, wherein the substituents are selected from alkyl, cycloalkyl, heterocyclyl, aryl, hetaryl, hydroxyl, mercapto, polyalkylene oxide, polyalkylenimine, alkoxy, halogen, COOH, carboxylate, $SO_3H$, sulfonate, $NE^4E^5$, $NE^4E^5E^{6+}X^-$, nitro, alkoxycarbonyl, formyl, acyl and

cyano, where $E^4$, $E^5$ and $E^6$ are each identical or different residues selected from hydrogen, alkyl, cycloalkyl and aryl and $X^-$ is an anion equivalent;
or
one of the residues $R^A$ and $R^B$ and/or one of the residues $R^C$ and $R^D$ together with the phosphorus atom and, if present, the groups $X^1$, $X^2$, $X^5$ and $X^6$ to which they are bound, and together with a bridging group atom of the bridging group Y, are a 5- to 8-membered heterocycle, which is optionally additionally fused to cycloalkyl, heterocycloalkyl, aryl or hetaryl, wherein the heterocycle and, if present, the fused groups, are in addition mutually independently optionally substituted, wherein the substituents are selected from alkyl, cycloalkyl, heterocyclyl, aryl, hetaryl, hydroxyl, mercapto, polyalkylene oxide, polyalkylenimine, alkoxy, halogen, COOH, carboxylate, $SO_3H$, sulfonate, $NE^4E^5$, $NE^4E^5E^{6+}X^-$ , nitro, alkoxycarbonyl, formyl, acyl and cyano, where $E^4$, $E^5$ and $E^6$ are each identical or different residues selected from hydrogen, alkyl, cycloalkyl and aryl and $X^-$ is an anion equivalent.

**3.** The method according to claim 2, wherein the bidentate phosphane ligand is selected from compounds of the general formula III, where

$X^1$, $X^2$, $X^3$, $X^4$, $X^5$ and $X^6$ are mutually independently optionally substituted $C_1$-$C_6$-alkylene, -(C=O)-$NE^7$-ferrocenyl, ferrocenyl-$NE^7$-(C=O)- or -(C=O)-$NE^7$-, where $E^7$ is as defined above;
Y, a, b, c, d, e and f are as defined above;
$R^A$, $R^B$, $R^C$ and $R^D$ are residues mutually independently selected from $C_1$-$C_{30}$-alkyl, $C_4$-$C_{10}$-cycloalkyl, $C_3$-$C_{10}$-heterocyclyl, $C_5$-$C_{14}$-aryl or $C_5$-$C_{30}$-hetaryl, wherein said residues are mutually independently optionally mono- or polysubstituted, wherein the substituents are mutually independently selected from $C_1$-$C_{10}$-alkyl, $C_4$-$C_{10}$-cycloalkyl, $C_3$-$C_{10}$-heterocycloalkyl, $C_5$-$C_{14}$-aryl, $C_5$-$C_{30}$-hetaryl, $C_1$-$C_{10}$-alkoxy, $C_3$-$C_{10}$-cycloalkoxy, $C_3$-$C_{10}$-heterocycloalkoxy, $C_5$-$C_{14}$-aryloxy, $C_5$-$C_{30}$-hetaryloxy, hydroxyl, mercapto, poly-$C_2$-$C_6$-alkylene oxide, poly-$C_2$-$C_6$-alkylenimine, COOH, carboxylate, $SO_3H$, sulfonate, halogen, nitro, formyl, $C_1$-$C_{10}$-acyl, cyano, $NE^1E^2$, and $NE^1E^2E^{3+}X^-$, where $E^1$, $E^2$ and $E^3$ are each identical or different residues selected from hydrogen, $C_1$-$C_{10}$-alkyl, $C_3$-$C_{10}$-cycloalkyl, or $C_5$-$C_{10}$-aryl and $X^-$ is an anion equivalent;
or
$R^A$ and $R^B$ and/or $R^C$ and $R^D$ together with the phosphorus atom and, if present, the groups $X^1$, $X^2$, $X^5$ and $X^6$ to which they are bound, are a 4- to 8-membered heterocycle or heterobicycle, which is optionally additionally fused to $C_4$-$C_{10}$-cycloalkyl, $C_3$-$C_{10}$-heterocycloalkyl, $C_5$-$C_{14}$-aryl or $C_5$-$C_{30}$-hetaryl, wherein the heterocycle or heterobicycle and, if present, the fused groups, are mutually independently optionally substituted, wherein the substituents are selected from $C_1$-$C_{10}$-alkyl, $C_3$-$C_{10}$-cycloalkyl, $C_3$-$C_{10}$-heterocycloalkyl, $C_5$-$C_{14}$-aryl, $C_5$-$C_{30}$-hetaryl, hydroxyl, mercapto, poly-$C_2$-$C_6$-alkylene oxide, poly-$C_2$-$C_6$-alkylenimine, $C_1$-$C_{10}$-alkoxy, halogen, COOH, carboxylate, $SO_3H$, sulfonate, $NE^4E^5$, $NE^4E^5E^{6+}X^-$, nitro, $C_1$-$C_{10}$-alkoxycarbonyl, formyl, $C_1$-$C_{10}$-acyl and cyano, where $E^4$, $E^5$ and $E^6$ are each identical or different residues selected from hydrogen, $C_1$-$C_{10}$-alkyl, $C_3$-$C_{10}$-cycloalkyl and $C_5$-$C_{14}$-aryl and $X^-$ is an anion equivalent;
or
one of the residues $R^A$ and $R^B$ and/or one of the residues $R^C$ and $R^D$ together with the phosphorus atom and, if present, the groups $X^1$, $X^2$, $X^5$ and $X^6$ to which they are bound, and together with a bridging group atom of the bridging group Y, are a 5- to 8-membered heterocycle, which is optionally additionally fused to $C_3$-$C_{10}$-cycloalkyl, $C_3$-$C_{10}$-heterocycloalkyl, $C_5$-$C_{14}$-aryl or $C_5$-$C_{30}$-hetaryl, wherein the heterocycle and, if present, the fused groups, are in addition mutually independently optionally substituted, wherein the substituents are selected from $C_1$-$C_{10}$-alkyl, $C_3$-$C_{10}$-cycloalkyl, $C_3$-$C_{10}$-heterocycloalkyl, $C_5$-$C_{14}$-aryl, $C_5$-$C_{30}$-hetaryl, hydroxyl, mercapto, poly-$C_2$-$C_6$-alkylene oxide, poly-$C_2$-$C_6$-alkylenimine, $C_1$-$C_{10}$-alkoxy, halogen, COOH, carboxylate, $SO_3H$, sulfonate, $NE^4E^5$, $NE^4E^5E^{6+}X^-$, nitro, $C_1$-$C_{10}$-alkoxycarbonyl, formyl, $C_1$-$C_{10}$-acyl and cyano, where $E^4$, $E^5$ and $E^6$ are each identical or different residues selected from hydrogen, $C_1$-$C_{10}$-alkyl, $C_3$-$C_{10}$-cycloalkyl and $C_5$-$C_{14}$-aryl and $X^-$ is an anion equivalent.

**4.** The method according to any of the preceding claims, wherein the group Y in the bidentate phosphane ligand of the general formula III is a bridging group selected from

- straight-chain or branched alkylene,
- straight-chain or branched alkenylene,
- cycloalkylene optionally comprising at least one ring heteroatom selected from N, O, S,
- cycloalkenylene optionally comprising at least one ring heteroatom selected from N, O, S,
- bi-, tri- and tetracyclic bridging groups;
- arylene,

- heteroarylene comprising at least one ring heteroatom selected from N, O and S,
- heteroalkylene comprising at least one chain heteroatom selected from N, O and S,
- heteroalkenylene comprising at least one chain heteroatom selected from N, O and S,
- polycyclic bridging groups comprising at least two mutually bound aromatic, heteroaromatic, carbocyclic or heterocyclic, 4- to 8-membered rings; and
- metallocene bridging groups comprising at least one metallocene group of the general formula

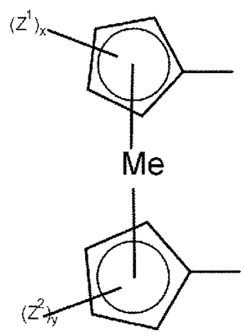

where Me is a metal atom, wherein the P atoms of the phosphane ligand are bonded directly or via one of the groups $X^3$ or $X^4$ to the same or two different Cp rings of the metallocene, x and y are mutually independently 0, 1, 2, 3 or 4 and $Z^1$ and $Z^2$ are mutually independently H, optionally mono- or polysubstituted alkyl or optionally mono- or polysubstituted aryl.

5. The method according to any of the preceding claims, wherein the group Y in the bidentate phosphane ligand of the general formula III is a polycyclic bridging group selected from groups of the formulae III.a to III.k

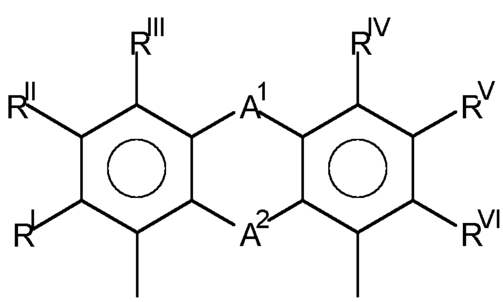

(III.a)

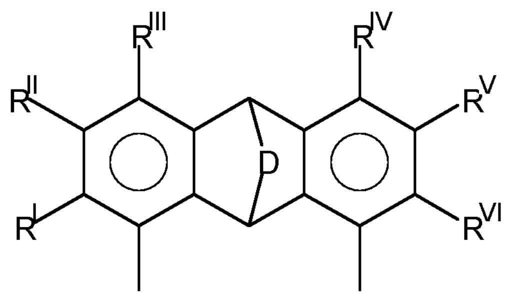

(III.b)

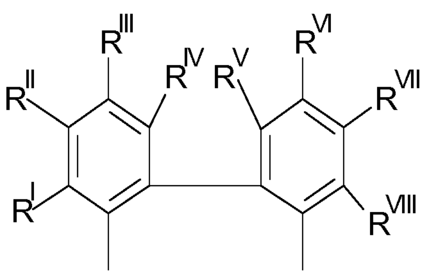

(III.c)

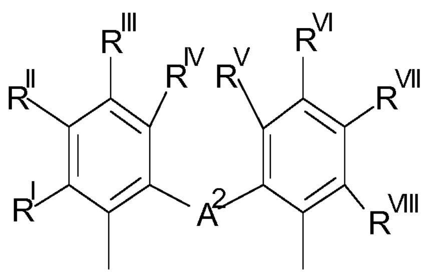

(III.d)

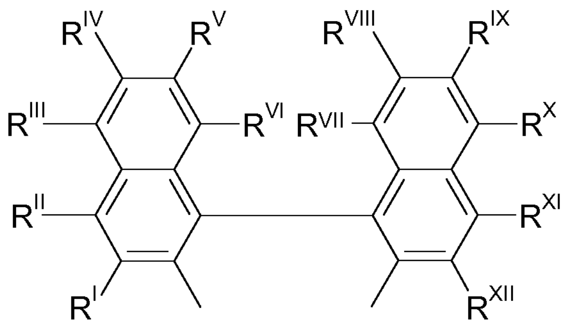

(III.e)

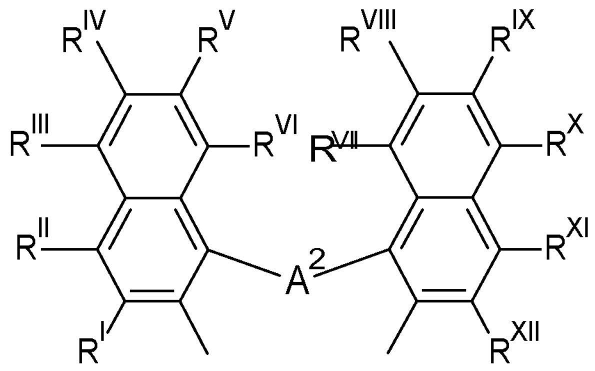

(III.f)

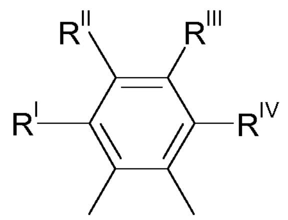

(III.g)

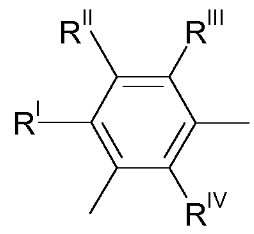

(III.h)

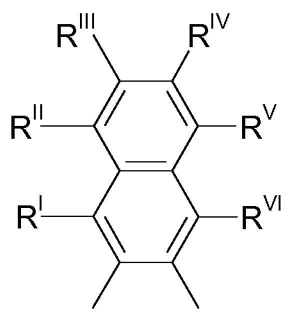

(III.i)

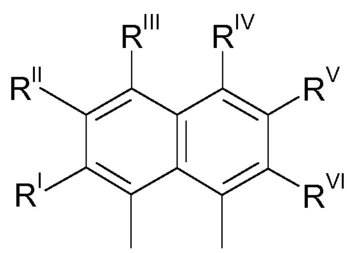

(III.k)

where

$R^I$, $R^{II}$, $R^{III}$, $R^{IV}$, $R^V$, $R^{VI}$, $R^{VII}$, $R^{VIII}$, $R^{IX}$, $R^X$, $R^{XI}$ and $R^{XII}$ are mutually independently hydrogen, alkyl, cycloalkyl, heterocycloalkyl, aryl, hetaryl, hydroxyl, mercapto, polyalkylene oxide, polyalkylenimine, alkoxy, halogen, $SO_3H$,

sulfonate, $NE^9E^{10}$, alkylene-$NE^9E^{10}$, trifluoromethyl, nitro, alkoxycarbonyl, carboxyl, formyl, acyl or cyano, where $E^9$ and $E^{10}$ are each identical or different residues selected from hydrogen, alkyl, cycloalkyl and aryl,
or
in each case two adjacent residues $R^I$, $R^{II}$, $R^{III}$, $R^{IV}$, $R^V$, $R^{VI}$, $R^{VII}$, $R^{VIII}$, $R^{IX}$, $R^X$, $R^{XI}$ and $R^{XII}$, together with the carbon atoms to which they are bound, form a 4-to 7-membered carbocyclic or heterocyclic, aromatic or non-aromatic ring, which in turn may optionally be fused to a mono- or polycyclic carbocyclic or heterocyclic, aromatic or non-aromatic ring, wherein said ring system is optionally substituted, wherein the substituents are selected from alkyl, cycloalkyl, heterocyclyl, aryl, hetaryl, hydroxyl, mercapto, polyalkylene oxide, polyalkylenimine, alkoxy, halogen, COOH, carboxylate, $SO_3H$, sulfonate, $NE^4E^5$, $NE^4E^5E^{6+}X^-$, nitro, alkoxycarbonyl, formyl, acyl and cyano, where $E^4$, $E^5$ and $E^6$ are each identical or different residues selected from hydrogen, alkyl, cycloalkyl and aryl and $X^-$ is an anion equivalent;
wherein the two adjacent residues from $R^I$, $R^{II}$, $R^{III}$, $R^{IV}$, $R^V$, $R^{VI}$, $R^{VII}$, $R^{VIII}$, $R^{IX}$, $R^X$, $R^{XI}$ or $R^{XII}$ may be bonded to carbon atoms of the identical aromatic ring or to carbon atoms of two aromatic rings which are different from one another;
$A^1$ is a bond, NH, $NR^{11}$, O, S, $CR^{11}$ or $CR^{11}R^{12}$, where $R^{11}$ and $R^{12}$ are mutually independently selected from H, alkyl and aryl;
$A^2$ is an NH, N, O or S,
D is a divalent bridging group of the general formula

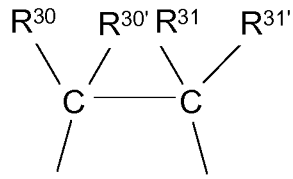

in which

$R^{30}$, $R^{30'}$, $R^{31}$ and $R^{31'}$ are mutually independently hydrogen, alkyl, cycloalkyl, aryl, halogen, trifluoromethyl, carboxyl, carboxylate or cyano,
where $R^{30'}$ together with $R^{31'}$ can also be the second bond of a double bond between the two carbon atoms to which $R^{30'}$ and $R^{31'}$ are bound, and/or $R^{30}$ and $R^{31}$ together with the carbon atoms to which they are bound can also be a 4- to 8-membered carbocycle or heterocycle, which in addition is optionally singly, doubly or triply fused with cycloalkyl, heterocycloalkyl, aryl or hetaryl, wherein the carbocycle or heterocyle and, if present, the fused groups may each mutually independently bear one, two, three or four substituents selected from alkyl, cycloalkyl, heterocycloalkyl, aryl, hetaryl, $COOR^e$, $COO^-M^+$, $SO_3R^e$, $SO_3^-M^+$, $NE^{11}E^{12}$, alkylene-$NE^{11}E^{12}$, $NE^{11}E^{12}E^{13+}X^-$, alkylene-$NE^{11}E^{12}E^{13+}X^-$, $OR^e$, $SR^e$, $(CHR^f CH_2O)_yR^e$, $(CH_2N(E^{11}))_yR^e$, $(CH_2CH_2N(E^{11}))_yR^e$, halogen, trifluoromethyl, nitro, formyl, acyl or cyano, where

$R^e$, $E^{11}$, $E^{12}$ and $E^{13}$ are each identical or different residues selected from hydrogen, alkyl, cycloalkyl or aryl,
$R^f$ is hydrogen, methyl or ethyl,
$M^+$ is a cation equivalent,
$X^-$ is an anion equivalent, and
y is an integer from 1 to 120.

**6.** The method according to any of the preceding claims, wherein the group Y in the bidentate phosphane ligand of the general formula III is a bridging group selected from polycyclic groups III.m to III.o

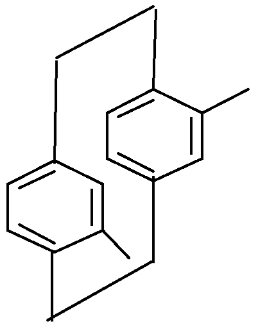

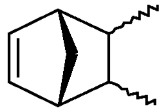

(III.n)

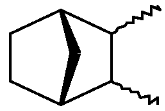

(III.o)

(III.m)

7. The method according to any of the preceding claims, wherein the group Y in the bidentate phosphane ligand of the general formula III is a bridging group selected from the heterocyclic groups V.a to V.h

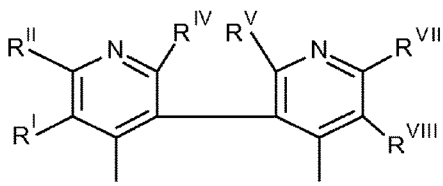

(V.a)

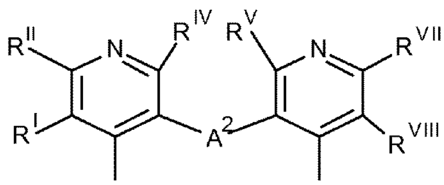

(V.b)

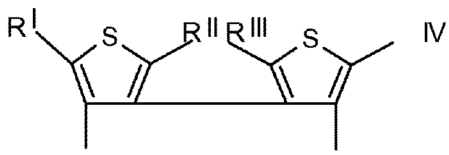

(V.c)

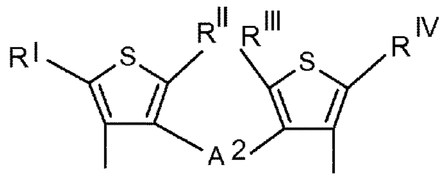

(V.d)

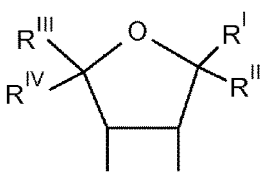

(V.e)

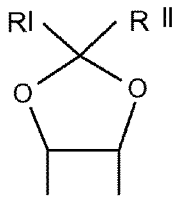

(V.f)

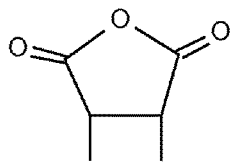

(V.g)

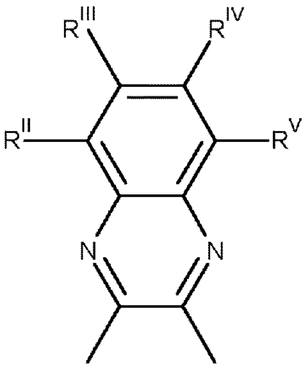

(V.h)

where

the residues $R^I$ to $R^{VIII}$ are mutually independently selected from hydrogen, alkyl, cycloalkyl, heterocycloalkyl, aryl, hetaryl, hydroxyl, mercapto, polyalkylene oxide, polyalkylenimine, alkoxy, halogen, $SO_3H$, sulfonate, $NE^9E^{10}$, alkylene-$NE^9E^{10}$, trifluoromethyl, nitro, alkoxycarbonyl, carboxyl, formyl, acyl or cyano, where $E^9$ and $E^{10}$ are each identical or different residues selected from hydrogen, alkyl, cycloalkyl and aryl; and
$A^2$ is an NH group, N, O or S.

**8.** The method according to any of the preceding claims, wherein the two phosphane end groups in the bidentate phosphane ligand of the general formula III are mutually independently selected from groups of the formulae VI.b, VI.d, VI.e, VI.f, VI.g, VI.h, VI.i and VI.k

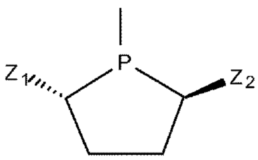

(V I.b)

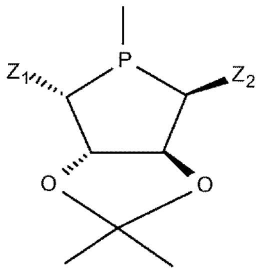

(V I.d)

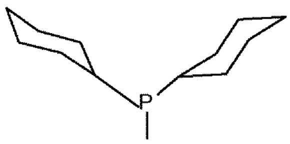

(V I.e)

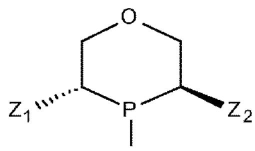

(V I.f)

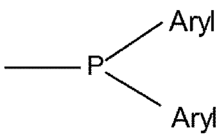

(VI.g)

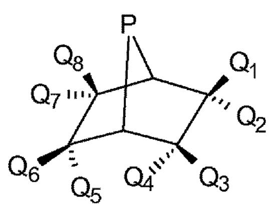

(VI.h)

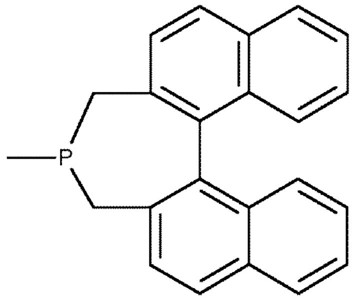

(VI.k)

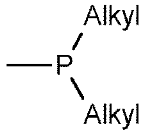

(VI.i)

where

aryl is phenyl, optionally mono- or polysubstituted, wherein the substituents are selected from alkyl or alkoxy
$Z_1$ and $Z_2$ are mutually independently selected from alkyl, alkoxy, alkoxyalkyl, aryl or hetaryl and
$Q_1$ to $Q_8$ are mutually independently H or alkyl.

**9.** The method according to any of the preceding claims, wherein the acid co-catalyst is a Lewis acid.

**10.** The method according to any of the preceding claims, wherein the acid co-catalyst is selected from: $[NH_4]TFA$, $[NH_4]Cl$, $[NH_4]_2[SO_4]$, $[NH_4]Br$, $Al(OTf)_3$, $Sc(OTf)_3$, $AlCl_3$, $AlBr_3$, $BR_3$, $BCl_3$, $BBr_3$, $BF_3$, $Al(NO_3)_3$.

**11.** The method according to any of the preceding claims, wherein the catalyst K is a ruthenium catalyst having the following general formula IV:

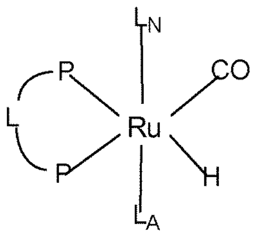

(IV)

where

P-L-P is the bidentate phosphane ligand of the formula (III) defined above;
$L_N$ is a neutral ligand selected from amines, phosphines, phosphites, alcohols, N-heterocyclic carbenes, imines, carbonyl compounds, olefines, alkynes or nitriles,
and
$L_A$ is absent or is an anionic ligand selected from chloride, fluoride, bromide, iodide, hydride, cyanide, alkoxy, amido or hydroxyl.

**12.** The method according to any of the preceding claims, wherein the amination is carried out enantioselectively by using a catalyst K bearing at least one chiral bidentate phosphane ligand according to formula III above.

**13.** The method according to any of the preceding claims, wherein the carbonyl compound to be aminated is a compound of the following general formula II,

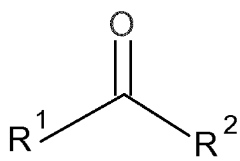

where $R^1$ and $R^2$ are the same or different and are mutually independently selected from
H,
optionally mono- or polysubstituted alkyl, wherein the carbon chain is optionally interrupted by one or more carbonyl groups,
optionally mono- or polysubstituted cycloalkyl, wherein the carbocyclic ring is optionally interrupted by one or more carbonyl groups,
optionally mono- or polysubstituted heterocyclyl, wherein the heterocyclic ring is optionally interrupted by one or more carbonyl groups,
optionally mono- or polysubstituted aryl and optionally mono- or polysubstituted hetaryl;
where the substituents optionally present are selected from halogen (in particular F, Cl, Br), -OH, -CN, $-NH_2$, $C_1$-$C_{30}$-alkyl, $C_3$-$C_{30}$-cycloalkyl, $C_3$-$C_{30}$-heterocyclyl, $C_5$-$C_{14}$-aryl, $C_5$-$C_{14}$-heteroaryl, $-OR_7$, $-NHR_7$ or $-N(R_7)_2$,$-C(O)R_7$, $-C(O)OR_7$, $-C(O)NHR_7$ or $-C(O)N(R_7)_2$,
where $R_7$ is selected from $C_1$-$C_{30}$-alkyl and $C_5$-$C_{30}$-aryl.

**14.** The method according to any of the preceding claims, wherein the catalyst K is formed from a catalyst precursor selected from the following compounds:
$[Ru(PPh_3)_3(H)(Cl)(CO)]$, $[Ru(PPh_3)_3(H)_2(CO)]$, $[Ru(PPh_3)_3(Cl)_2]$, $[Ru(PPh_3)_3(Cl)_2(CO)]$, [Ru(p-cymene)$(Cl)_2$], [Ru(benzene)$Cl_2]_n$, $[Ru(CO)_2Cp]_n$ $[Ru(CO)_3(Cl)_2]$ [Ru(COD)(allyl)], [Ru(COD)(2-methylallyl)$_2$], $[RuCl_3{*}H_2O]$, [Ru(acetylacetonate)$_3$],
$[Ru(DMSO)_4(Cl)_2]$,
$Ru_3CO_{12}$,[Ru(cyclopentadienyl)$(PPh_3)_2$(Cl)], Ru(cyclopentadienyl)$(CO)_2$(Cl)], [Ru(cyclopentadienyl)$(CO)_2$H], [Ru(cyclopentadienyl)$(CO)_2]_2$, [Ru(pentamethylcyclopentadienyl)$(CO)_2$(Cl)], [Ru(pentamethylcylcopentadienyl)$(CO)_2$H], [Ru(pentamethylcyclopentadienyl)$(CO)_2]_2$, [Ru(indenyl)$(CO)_2$(Cl)], [Ru(indenyl)$(CO)_2$(H)], [Ru(indenyl)$(CO)_2]_2$, ruthenocene, [Ru(binap)$Cl_2$], [Ru(bipyridine)$_2Cl_2{*}2H_2O$], $[Ru(COD)Cl_2]_2$, [Ru(pentamethylcyclopentadienyl)(COD)(Cl)], $[Ru_3(CO)_{12}]$, [Ru(tetraphenylhydroxycyclopentadienyl)$(CO)_2$(H)], $[Ru(PPh_3)_4(H)_2]$.

**15.** The method according to any of the preceding claims, wherein the catalyst K is used in an amount of 1 to 5000 ppm parts by weight, based on the total weight of the liquid reaction medium used and/or wherein the reaction is carried out under the following reaction conditions:

a) temperature in the range of 20 to 250°C and/or
b) total pressure in the range of 0.1 to 30 MPa and/or
c) reaction time of 0.1 to 100 h and/or
d) ammonia: 1.5 to 250-fold molar excess, based on the moles of reactant used and/or
e) 1.5 to 250-fold molar excess of hydrogen based on the moles of reactant used.

## Revendications

**1.** Procédé d'amination réductrice d'un composé de carbonyle, comprenant un ou plusieurs groupes carbonyle pouvant être soumis à une amination réductrice, **caractérisé en ce que** la réaction est réalisée en présence d'un catalyseur K dissous de manière homogène, ainsi que d'un acide en tant que co-catalyseur, le catalyseur K étant un catalyseur à base de ruthénium, qui comprend un ligand phosphane bidentate, un ligand carbonyle, un ligand hydrido et éventuellement un ligand neutre.

**2.** Procédé selon la revendication 1, selon lequel le ligand phosphane bidentate présente la formule générale III suivante :

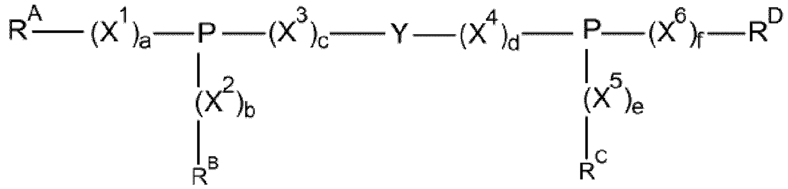

(III)

dans laquelle

a, b, c, d, e et f représentent indépendamment les uns des autres 0 ou 1,
$X^1$, $X^2$, $X^3$, $X^4$, $X^5$ et $X^6$ représentent indépendamment les uns des autres alkylène, arylène, -(C=O)-$NE^7$-métallocényle, métallocényl-$NE^7$-(C=O)- ou -(C=O)-$NE^7$-, $E^7$ représentant H ou un radical alkyle ; ce radical et le groupe métallocényle, alkylène ou arylène étant chacun indépendamment les uns des autres éventuellement substitués une ou plusieurs fois, et les substituants étant choisis indépendamment les uns des autres parmi alkyle, cycloalkyle, hétérocyclyle, aryle, hétaryle, alcoxy, cycloalcoxy, hétérocycloalcoxy, aryloxy, hétaryloxy, hydroxy, mercapto, polyoxyde d'alkylène, polyalkylène-imine, COOH, carboxylate, $SO_3H$, sulfonate, halogène, nitro, formyle, acyle, cyano, $NE^1E^2$ et $NE^1E^2E^{3+}X^-$, $E^1$, $E^2$ et $E^3$ signifiant chacun des radicaux identiques ou différents, choisis parmi hydrogène, alkyle, cycloalkyle ou aryle, et $X^-$ représentant un équivalent d'anion,
Y représente un groupe de pontage contenant des atomes de carbone, et
$R^A$, $R^B$, $R^C$ et $R^D$ représentent indépendamment les uns des autres des radicaux choisis parmi alkyle, cycloalkyle, hétérocycloalkyle, aryle ou hétaryle, ces radicaux étant indépendamment les uns des autres éventuellement substitués une ou plusieurs fois, les substituants étant choisis indépendamment les uns des autres parmi alkyle, cycloalkyle, hétérocyclyle, aryle, hétaryle, alcoxy, cycloalcoxy, hétérocycloalcoxy, aryloxy, hétaryloxy, hydroxy, mercapto, polyoxyde d'alkylène, polyalkylène-imine, COOH, carboxylate, $SO_3H$, sulfonate, halogène, nitro, formyle, acyle, cyano, $NE^1E^2$ et $NE^1E^2E^{3+}X^-$, $E^1$, $E^2$ et $E^3$ signifiant chacun des radicaux identiques ou différents, choisis parmi hydrogène, alkyle, cycloalkyle ou aryle, et $X^-$ représentant un équivalent d'anion ;
ou
$R^A$ et $R^B$ et/ou $R^C$ et $R^D$ représentent ensemble avec l'atome de phosphore et, le cas échéant, les groupes $X^1$, $X^2$, $X^5$ et $X^6$, auxquels ils sont reliés, un hétérocycle ou hétérobicycle de 4 à 8 chaînons, qui est éventuellement en outre annelé avec cycloalkyle, hétérocyclyle, aryle ou hétaryle, l'hétérocycle ou l'hétérobicycle et, le cas échéant, les groupes annelés étant indépendamment les uns des autres éventuellement substitués, les substituants étant choisis parmi alkyle, cycloalkyle, hétérocyclyle, aryle, hétaryle, hydroxy, mercapto, polyoxyde d'alkylène, polyalkylène-imine, alcoxy, halogène, COOH, carboxylate, $SO_3H$, sulfonate, $NE^4E^5$, $NE^4E^5E^{6+}X^-$, nitro, alcoxycarbonyle, formyle, acyle et cyano, $E^4$, $E^5$ et $E^6$ signifiant chacun des radicaux identiques ou différents, choisis parmi hydrogène, alkyle, cycloalkyle et aryle, et $X^-$ représentant un équivalent d'anion ;
ou
un des radicaux $R^A$ et $R^B$ et/ou un des radicaux $R^C$ et $R^D$ représentent conjointement avec l'atome de phosphore et, le cas échéant, les groupes $X^1$, $X^2$, $X^5$ et $X^6$, auxquels ils sont reliés et conjointement avec un atome de groupe de pontage du groupe de pontage Y, un hétérocycle de 5 à 8 chaînons, qui est éventuellement en outre annelé avec cycloalkyle, hétérocycloalkyle, aryle ou hétaryle, l'hétérocycle et, le cas échéant, les groupes annelés étant indépendamment les uns des autres éventuellement en outre substitués, les substituants étant choisis parmi alkyle, cycloalkyle, hétérocyclyle, aryle, hétaryle, hydroxy, mercapto, polyoxyde d'alkylène, polyalkylène-imine, alcoxy, halogène, COOH, carboxylate, $SO_3H$, sulfonate, $NE^4E^5$, $NE^4E^5E^{6+}X^-$, nitro, alcoxycarbonyle, formyle, acyle et cyano, $E^4$, $E^5$ et $E^6$ signifiant chacun des radicaux identiques ou différents, choisis parmi hydrogène, alkyle, cycloalkyle ou aryle, et $X^-$ représentant un équivalent d'anion.

3. Procédé selon la revendication 2, selon lequel le ligand phosphane bidentate est choisi parmi les composés de la formule générale III, dans laquelle
$X^1$, $X^2$, $X^3$, $X^4$, $X^5$ et $X^6$ représentent indépendamment les uns des autres alkylène en $C_1$-$C_6$ éventuellement substitué, -(C=O)-$NE^7$-ferrocényle, ferrocényl-$NE^7$-(C=O)- ou -(C=O)-$NE^7$-, $E^7$ ayant les significations indiquées précédemment ;
Y, a, b, c, d, e et f ont les significations indiquées précédemment ;
$R^A$, $R^B$, $R^C$ et $R^D$ représentent indépendamment les uns des autres des radicaux choisis parmi alkyle en $C_1$-$C_{30}$, cycloalkyle en $C_4$-$C_{10}$, hétérocyclyle en $C_3$-$C_{10}$, aryle en $C_5$-$C_{14}$ ou hétaryle en $C_5$-$C_{30}$, ces radicaux étant indépendamment les uns des autres éventuellement substitués une ou plusieurs fois, les substituants étant choisis indépendamment les uns des autres parmi alkyle en $C_1$-$C_{10}$, cycloalkyle en $C_4$-$C_{10}$, hétérocycloalkyle en $C_3$-$C_{10}$, aryle en $C_5$-$C_{14}$, hétaryle en $C_5$-$C_{30}$, alcoxy en $C_1$-$C_{10}$, cycloalcoxy en $C_3$-$C_{10}$, hétérocycloalcoxy en $C_3$-$C_{10}$, aryloxy en $C_5$-$C_{14}$, hétaryloxy en $C_5$-$C_{30}$, hydroxy, mercapto, poly-oxyde d'alkylène en $C_2$-$C_6$, poly-alkylène-imine en $C_2$-$C_6$, COOH, carboxylate, $SO_3H$, sulfonate, halogène, nitro, formyle, acyle en $C_1$-$C_{10}$, cyano, $NE^1E^2$ et $NE^1E^2E^{3+}X^-$, $E^1$, $E^2$ et $E^3$ signifiant chacun des radicaux identiques ou différents, choisis parmi hydrogène, alkyle en $C_1$-$C_{10}$, cycloalkyle en $C_3$-$C_{10}$ ou aryle en $C_5$-$C_{10}$, et $X^-$ représentant un équivalent d'anion,
ou
$R^A$ et $R^B$ et/ou $R^C$ et $R^D$ représentent ensemble avec l'atome de phosphore et, le cas échéant, les groupes $X^1$, $X^2$, $X^5$ et $X^6$, auxquels ils sont reliés, un hétérocycle ou hétérobicycle de 4 à 8 chaînons, qui est éventuellement en outre annelé avec cycloalkyle en $C_4$-$C_{10}$, hétérocycloalkyle en $C_3$-$C_{10}$, aryle en $C_5$-$C_{14}$ ou hétaryle en $C_5$-$C_{30}$, l'hétérocycle ou l'hétérobicycle et, le cas échéant, les groupes annelés étant indépendamment les uns des autres éventuellement substitués, les substituants étant choisis parmi alkyle en $C_1$-$C_{10}$, cycloalkyle en $C_3$-$C_{10}$, hétérocy-

cloalkyle en $C_3$-$C_{10}$, aryle en $C_5$-$C_{14}$, hétaryle en $C_5$-$C_{30}$, hydroxy, mercapto, poly-oxyde d'alkylène en $C_2$-$C_6$, poly-alkylène-imine en $C_2$-$C_6$, alcoxy en $C_1$-$C_{10}$, halogène, COOH, carboxylate, $SO_3H$, sulfonate, $NE^4E^5$, $NE^4E^5E^{6+}X^-$, nitro, alcoxycarbonyle en $C_1$-$C_{10}$, formyle, acyle en $C_1$-$C_{10}$ et cyano, $E^4$, $E^5$ et $E^6$ signifiant chacun des radicaux identiques ou différents, choisis parmi hydrogène, alkyle en $C_1$-$C_{10}$, cycloalkyle en $C_3$-$C_{10}$ et aryle en $C_5$-$C_{14}$, et $X^-$ représentant un équivalent d'anion ;

ou

un des radicaux $R^A$ et $R^B$ et/ou un des radicaux $R^C$ et $R^D$ représentent conjointement avec l'atome de phosphore et, le cas échéant, les groupes $X^1$, $X^2$, $X^5$ et $X^6$, auxquels ils sont reliés et conjointement avec un atome de groupe de pontage du groupe de pontage Y, un hétérocycle de 5 à 8 chaînons, qui est éventuellement en outre annelé avec cycloalkyle en $C_3$-$C_{10}$, hétérocycloalkyle en $C_3$-$C_{10}$, aryle en $C_5$-$C_{14}$ ou hétaryle en $C_5$-$C_{30}$, l'hétérocycle et, le cas échéant, les groupes annelés étant indépendamment les uns des autres éventuellement en outre substitués, les substituants étant choisis parmi alkyle en $C_1$-$C_{10}$, cycloalkyle en $C_3$-$C_{10}$, hétérocycloalkyle en $C_3$-$C_{10}$, aryle en $C_5$-$C_{14}$, hétaryle en $C_5$-$C_{30}$, hydroxy, mercapto, poly-oxyde d'alkylène en $C_2$-$C_6$, poly-alkylène-imine en $C_2$-$C_6$, alcoxy en $C_1$-$C_{10}$, halogène, COOH, carboxylate, $SO_3H$, sulfonate, $NE^4E^5$, $NE^4E^5E^{6+}X^-$, nitro, alcoxycarbonyle en $C_1$-$C_{10}$, formyle, acyle en $C_1$-$C_{10}$ et cyano, $E^4$, $E^5$ et $E^6$ signifiant chacun des radicaux identiques ou différents, choisis parmi hydrogène, alkyle en $C_1$-$C_{10}$, cycloalkyle en $C_3$-$C_{10}$ et aryle en $C_5$-$C_{14}$, et $X^-$ représentant un équivalent d'anion.

**4.** Procédé selon l'une quelconque des revendications précédentes, selon lequel, dans le ligand phosphane bidentate de la formule générale III, le groupe Y représente un groupe de pontage, choisi parmi :

- un alkylène linéaire ou ramifié,
- un alcénylène linéaire ou ramifié,
- un cycloalkylène contenant éventuellement au moins un hétéroatome de cycle choisi parmi N, O, S,
- un cycloalcénylène, contenant éventuellement au moins un hétéroatome de cycle choisi parmi N, O, S,
- des groupes de pontage bi-, tri- et tétracycliques,
- un arylène,
- un hétéroarylène contenant au moins un hétéroatome de cycle choisi parmi N, O et S,
- un hétéroalkylène contenant au moins un hétéroatome de chaîne choisi parmi N, O et S,
- un hétéroalcénylène contenant au moins un hétéroatome de chaîne choisi parmi N, O et S,
- des groupes de pontage polynucléaires, comprenant au moins deux cycles aromatiques, hétéroaromatiques, carbocycliques ou hétérocycliques, de 4 à 8 chaînons, reliés les uns avec les autres ; et
- des groupes de pontage métallocène contenant au moins un groupe métallocène de la formule générale :

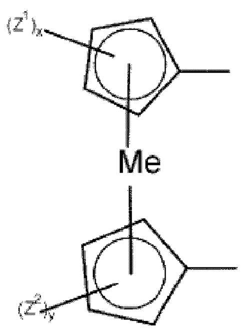

dans laquelle Me représente un atome métallique, les atomes P du ligand phosphane sont reliés directement ou par le biais d'un des groupes $X^3$ ou $X^4$ au même ou à deux cycles Cp différents du métallocène, x et y représentent indépendamment l'un de l'autre 0, 1, 2, 3 ou 4, et $Z^1$ et $Z^2$ représentent indépendamment l'un de l'autre H, alkyle éventuellement substitué une ou plusieurs fois, ou aryle éventuellement substitué une ou plusieurs fois.

**5.** Procédé selon l'une quelconque des revendications précédentes, selon lequel, dans le ligand phosphane bidentate de la formule générale III, le groupe Y représente un groupe de pontage polynucléaire, choisi parmi les groupes des formules III.a à III.k :

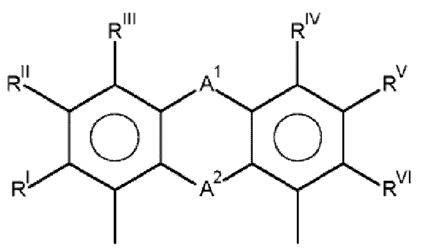

(III.a)

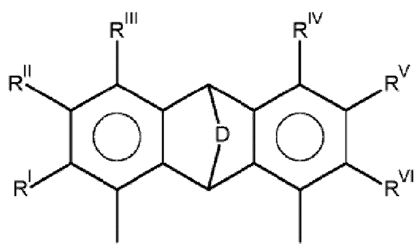

(III.b)

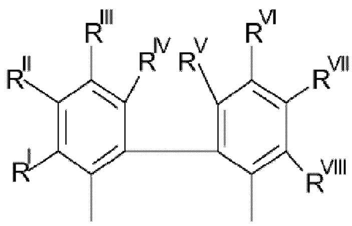

(III.c)

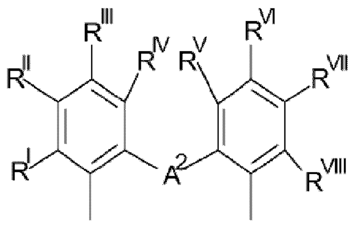

(III.d)

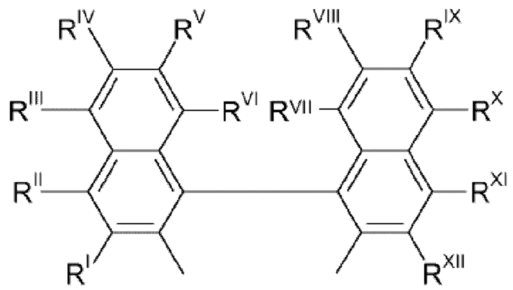

(III.e)

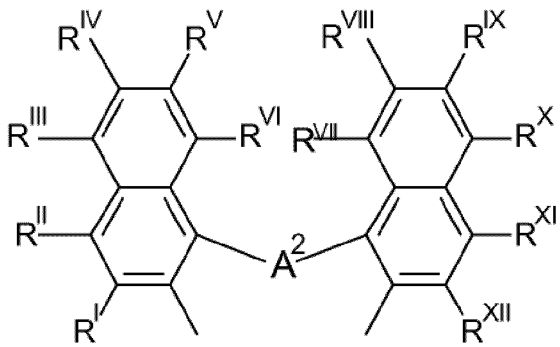

(III.f)

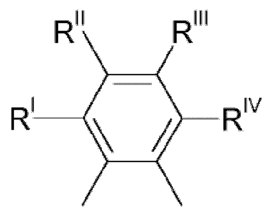

(III.g)

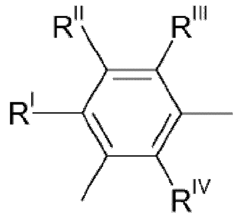

(III.h)

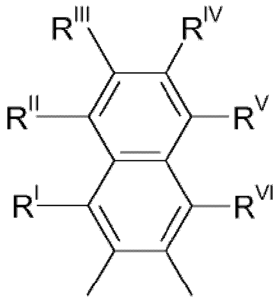

(III.i)

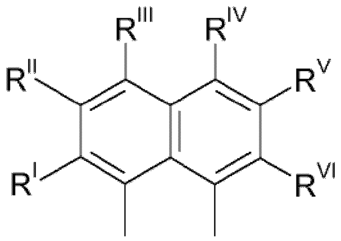

(III.k)

dans lesquelles

$R^I$, $R^{II}$, $R^{III}$, $R^{IV}$, $R^V$, $R^{VI}$, $R^{VII}$, $R^{VIII}$, $R^{IX}$, $R^X$, $R^{XI}$ et $R^{XII}$ représentent indépendamment les uns des autres hydrogène, alkyle cycloalkyle, hétérocycloalkyle, aryle, hétaryle, hydroxy, mercapto, polyoxyde d'alkylène, polyalkylène-imine, alcoxy, halogène, $SO_3H$, sulfonate, $NE^9E^{10}$, alkylène-$NE^9E^{10}$, trifluorométhyle, nitro, alcoxycarbonyle, carboxyle, formyle, acyle ou cyano, $E^9$ et $E^{10}$ signifiant chacun des radicaux identiques ou différents, choisis parmi hydrogène, alkyle, cycloalkyle et aryle,
ou
deux radicaux $R^I$, $R^{II}$, $R^{III}$, $R^{IV}$, $R^V$, $R^{VI}$, $R^{VII}$, $R^{VIII}$, $R^{IX}$, $R^X$, $R^{XI}$ et $R^{XII}$ voisins forment à chaque fois conjointement avec les atomes de carbone auxquels ils sont reliés un cycle de 4 à 7 chaînons, carbocyclique ou hétérocyclique, aromatique ou non aromatique, qui peut lui-même éventuellement être annelé avec un cycle mono- ou polynucléaire, carbocyclique ou hétérocyclique, aromatique ou non aromatique, ce système cyclique étant éventuellement substitué, les substituants étant choisis parmi alkyle, cycloalkyle, hétérocyclyle, aryle, hétaryle, hydroxy, mercapto, polyoxyde d'alkylène, polyalkylène-imine, alcoxy, halogène, COOH, carboxylate, $SO_3H$, sulfonate, $NE^4E^5$, $NE^4E^5E^{6+}X^-$, nitro, alcoxycarbonyle, formyle, acyle et cyano, $E^4$, $E^5$ et $E^6$ signifiant chacun des radicaux identiques ou différents, choisis parmi hydrogène, alkyle, cycloalkyle et aryle, et $X^-$ représentant un équivalent d'anion ;
les deux radicaux $R^I$, $R^{II}$, $R^{III}$, $R^{IV}$, $R^V$, $R^{VI}$, $R^{VII}$, $R^{VIII}$, $R^{IX}$, $R^X$, $R^{XI}$ et $R^{XII}$ voisins pouvant être reliés à des atomes de carbone du même cycle aromatique ou à des atomes de carbone de deux cycles aromatiques différents l'un de l'autre ;
$A^1$ représente une liaison, NH, $NR^{11}$, O, S, $CR^{11}$ ou $CR^{11}R^{12}$, $R^{11}$ et $R^{12}$ étant choisis indépendamment l'un de l'autre parmi H, alkyle et aryle ;
$A^2$ représente NH, N, O ou S,
D représente un groupe de pontage bivalent de la formule générale :

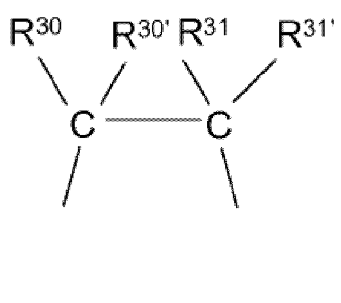

dans laquelle

$R^{30}$, $R^{30'}$, $R^{31}$ et $R^{31'}$ représentent indépendamment les uns des autres hydrogène, alkyle, cycloalkyle, aryle, halogène, trifluorométhyle, carboxyle, carboxylate ou cyano,

$R^{30'}$ pouvant également représenter conjointement avec $R^{31'}$ une fraction de liaison d'une double liaison entre les deux atomes de carbone auxquels $R^{30'}$ et $R^{31'}$ sont reliés, et/ou $R^{30}$ et $R^{31}$ pouvant représenter conjointement avec les atomes de carbone auxquels ils sont reliés un carbo- ou hétérocycle de 4 à 8 chaînons, qui est éventuellement en outre annelé une, deux ou trois fois avec cycloalkyle, hétérocycloalkyle, aryle ou hétaryle, le carbo- ou l'hétérocycle et, le cas échéant, les groupes annelés pouvant chacun porter indépendamment les uns des autres un, deux, trois ou quatre substituants, qui sont choisis parmi alkyle, cycloalkyle, hétérocycloalkyle, aryle, hétaryle, $COOR^e$, $COO^-M^+$, $SO_3R^e$, $SO_3^-M^+$, $NE^{11}E^{12}$, alkylène-$NE^{11}E^{12}$, $NE^{11}E^{12}E^{13+}X^-$, alkylène-$NE^{11}E^{12}E^{13+}X^-$, $OR^e$, $SR^e$, $(CHR^fCH_2O)_yR^e$, $(CH_2N(E^{11}))_yR^e$, $(CH_2CH_2N(E^{11}))_yR^e$, halogène, trifluorométhyle, nitro, formyle, acyle ou cyano,

$R^e$, $E^{11}$, $E^{12}$ et $E^{13}$ signifiant chacun des radicaux identiques ou différents, choisis parmi hydrogène, alkyle, cycloalkyle ou aryle,

$R^f$ représentant hydrogène, méthyle ou éthyle,

$M^+$ représentant un équivalent de cation,

$X^-$ représentant un équivalent d'anion, et

y représentant un nombre entier de 1 à 120.

**6.** Procédé selon l'une quelconque des revendications précédentes, selon lequel, dans le ligand phosphane bidentate de la formule générale III, le groupe Y représente un groupe de pontage choisi parmi les groupes polycycliques III.m à III.o :

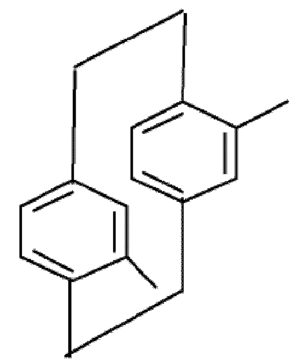

(III.m)

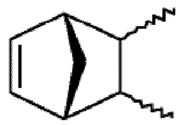

(III.n)

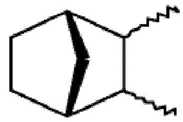

(III.o)

**7.** Procédé selon l'une quelconque des revendications précédentes, selon lequel, dans le ligand phosphane bidentate de la formule générale III, le groupe Y représente un groupe de pontage choisi parmi les groupes hétérocycliques V.a à V.h :

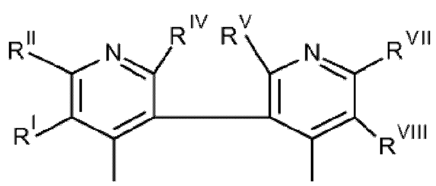

(V.a)

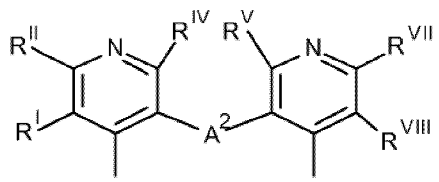

(V.b)

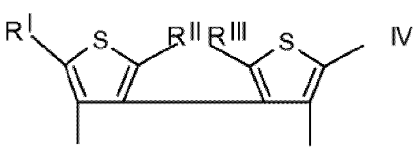

(V.c)

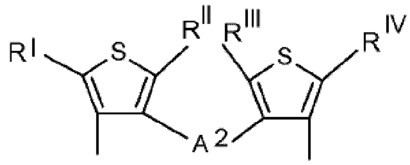

(V.d)

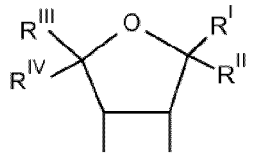

(V.e)

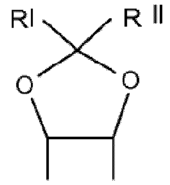

(V.f)

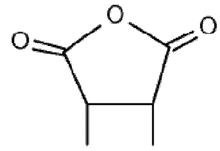

(V.g)

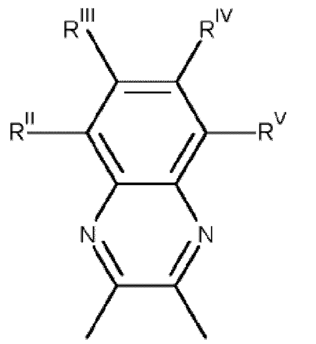

(V.h)

dans lesquelles

les radicaux $R^I$ à $R^{VIII}$ sont choisis indépendamment les uns des autres parmi hydrogène, alkyle, cycloalkyle, hétérocycloalkyle, aryle, hétaryle, hydroxy, mercapto, polyoxyde d'alkylène, polyalkylène-imine, alcoxy, halogène, $SO_3H$, sulfonate, $NE^9E^{10}$, alkylène-$NE^9E^{10}$, trifluorométhyle, nitro, alcoxycarbonyle, carboxyle, formyle, acyle ou cyano, $E^9$ et $E^{10}$ signifiant chacun des radicaux identiques ou différents, choisis parmi hydrogène, alkyle, cycloalkyle et aryle ; et

$A^2$ représente un groupe NH, N, O ou S.

**8.** Procédé selon l'une quelconque des revendications précédentes, selon lequel, dans le ligand phosphane bidentate de la formule générale III, les deux groupes terminaux phosphane sont choisis indépendamment l'un de l'autre dans les groupes des formules VI.b, VI.d, VI.e, VI.f, VI.g, VI.h, VI.i et VI.k :

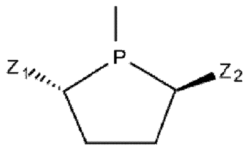

(VI.b)

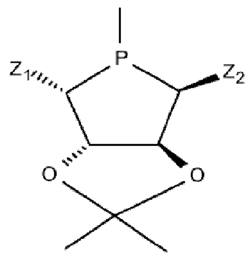

(VI.d)

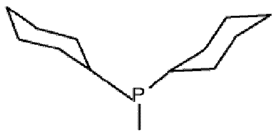

(VI.e)

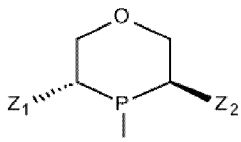

(VI.f)

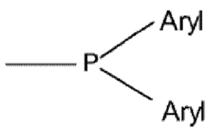

(VI.g)

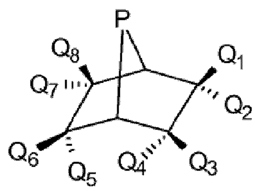

(VI.h)

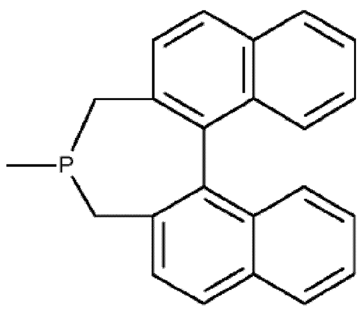

(VI.k)

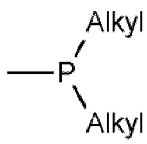

(VI.i)

dans lesquelles

aryle représente un phényle éventuellement substitué une ou plusieurs fois, les substituants étant choisis parmi alkyle ou alcoxy,

$Z_1$ et $Z_2$ sont choisis indépendamment l'un de l'autre parmi alkyle, alcoxy, alcoxyalkyle, aryle ou hétaryle, et $Q_1$ à $Q_8$ représentent indépendamment les uns des autres H ou alkyle.

**9.** Procédé selon l'une quelconque des revendications précédentes, selon lequel le co-catalyseur acide est un acide de Lewis.

**10.** Procédé selon l'une quelconque des revendications précédentes, selon lequel le co-catalyseur acide est choisi parmi : $[NH_4]TFA$, $[NH_4]Cl$, $[NH_4]_2[SO_4]$, $[NH_4]Br$, $Al(OTf)_3$, $Sc(OTf)_3$, $AlCl_3$, $AlBr_3$, $BR_3$, $BCl_3$, $BBr_3$, $BF_3$, $Al(NO_3)_3$.

**11.** Procédé selon l'une quelconque des revendications précédentes, selon lequel le catalyseur K est un catalyseur à base de ruthénium qui présente la formule générale IV suivante :

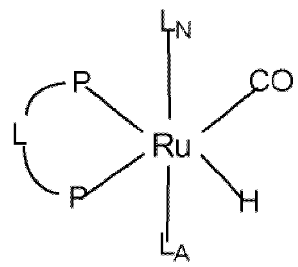

(IV)

dans laquelle

P-L-P est le ligand phosphane bidentate de la formule (III) indiquée précédemment ;
$L_N$ représente un ligand neutre, choisi parmi les amines, les phosphines, les phosphites, les alcools, les carbènes N-hétérocycliques, les imines, les composés de carbonyle, les oléfines, les alcynes ou les nitriles, et
$L_A$ est absent ou représente un ligand anionique choisi parmi chlorure, fluorure, bromure, iodure, hydrure, cyanure, alcoxy, amido ou hydroxy.

**12.** Procédé selon l'une quelconque des revendications précédentes, selon lequel l'amination est réalisée de manière énantiosélective en utilisant un catalyseur K qui porte au moins un ligand phosphane bidentate chiral selon la formule III précédente.

**13.** Procédé selon l'une quelconque des revendications précédentes, selon lequel le composé de carbonyle à aminer est un composé de la formule générale II suivante :

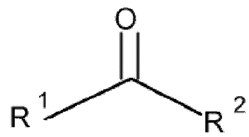

dans laquelle $R^1$ et $R^2$ sont identiques ou différents, et sont choisis indépendamment l'un de l'autre parmi :
H,
alkyle éventuellement substitué une ou plusieurs fois, la chaîne carbonée étant éventuellement interrompue par un ou plusieurs groupes carbonyle,
cycloalkyle éventuellement substitué une ou plusieurs fois, le cycle carbocyclique étant éventuellement interrompu par un ou plusieurs groupes carbonyle,
hétérocyclyle éventuellement substitué une ou plusieurs fois, le cycle hétérocyclique étant éventuellement interrompu par un ou plusieurs groupes carbonyle,
aryle éventuellement substitué une ou plusieurs fois, et
hétaryle éventuellement substitué une ou plusieurs fois ;
les substituants éventuellement présents étant choisis parmi halogène (notamment F, Cl, Br), -OH, -CN, $-NH_2$, alkyle en $C_1$-$C_{30}$, cycloalkyle en $C_3$-$C_{30}$, hétérocyclyle en $C_3$-$C_{30}$, aryle en $C_5$-$C_{14}$, hétéroaryle en $C_5$-$C_{14}$, $-OR_7$, $-NHR_7$ ou $-N(R_7)_2$, $-C(O)R_7$, $-C(O)OR_7$, $-C(O)NHR_7$ ou $-C(O)N(R_7)_2$, $R_7$ étant choisi parmi alkyle en $C_1$-$C_{30}$ et aryle en $C_5$-$C_{30}$.

**14.** Procédé selon l'une quelconque des revendications précédentes, selon lequel le catalyseur K est formé à partir d'un précurseur de catalyseur, choisi parmi les composés suivants :
$[Ru(PPh_3)_3(H)(Cl)(CO)]$, $[Ru (PPh_3)_3(H)_2(CO)]$, $[Ru(PPh_3)_3(Cl)_2]$, $[Ru(PPh_3)_3(Cl)_2(CO)]$, [Ru(p-cymène)$(Cl)_2$], [Ru (benzène)$Cl_2]_n$, $[Ru(CO)_2Cp]_n$ $[Ru(CO)_3(Cl)_2]$[Ru(COD)(allyl)], [Ru(COD)(2-méthylallyl)$_2$], $[RuCl_3{*}H_2O]$, [Ru(acétylacétonate)$_3$], $[Ru(DMSO)_4(Cl)_2]$, $Ru_3CO_{12}$, [Ru(cyclopentadiényl)$(PPh_3)_2$(Cl)], Ru(cyclopentadiényl)$(CO)_2$(Cl)], [Ru(cyclopentadiényl)$(CO)_2$H], [Ru(cyclopentadiényl)$(CO)_2]_2$, [Ru(pentaméthylcyclopentadiényl)$(CO)_2$(Cl)], [Ru(pentaméthylcyclopentadiényl)$(CO)_2$H], [Ru(pentaméthylcyclopentadiényl)$(CO)_2]_2$, [Ru(indényl)$(CO)_2$(Cl)], [Ru(indényl)$(CO)_2$(H)], [Ru(indényl)$(CO)_2]_2$, ruthénocène, [Ru(binap)$Cl_2$], [Ru(bipyridine)$_2Cl_2{*}2H_2O$],

$[Ru(COD)Cl_2]_2$, [Ru(pentaméthylcyclopentadiényl)(COD)(Cl)], $[Ru_3(CO)_{12}]$, [Ru(tétraphénylhydroxy-cyclopentadiényl)$(CO)_2(H)$], $[Ru(PPh_3)_4(H)_2]$.

**15.** Procédé selon l'une quelconque des revendications précédentes, selon lequel le catalyseur K est utilisé en une quantité de 1 à 5 000 ppm parties en poids, par rapport au poids total du milieu réactionnel liquide utilisé, et/ou selon lequel la réaction est réalisée dans les conditions de réaction suivantes :

a) une température dans la plage allant de 20 à 250 °C et/ou
b) une pression totale dans la plage allant de 0,1 à 30 MPa et/ou
c) une durée de réaction de 0,1 à 100 h, et/ou
d) un excès molaire d'ammoniac d'un facteur de 1,5 à 250, par mole de réactif utilisé, et/ou
e) un excès molaire d'hydrogène d'un facteur de 1,5 à 250, par mole de réactif utilisé.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6884887 B
- US 20040267051 A
- US 6687887 B


### In der Beschreibung aufgeführte Nicht-Patentliteratur


- **AMINES, ALIPAHTIC.** Ullmann's Encyclopedia of Industrial Chemistry. 2012, vol. 2, 647-695
- **D. GHISLIERI ; N.J. TURNER.** *Top. Catal.,* 2014, vol. 57, 284-300
- *Angew. Chem. Int. Ed.,* 2004, vol. 43, 788-824
- Chiral Amine Synthesis. Wiley-VCH, 2010, 225-245
- *Chem. Eur. J.,* 2014, vol. 20, 245-252
- Reactor Types and Their Industrial Applications. **K.D. HENKEL.** Ullmann's Encyclopedia of Industrial Chemistry. Wiley-VCH Verlag GmbH & Co. KGaA, 2005